(19) Europäisches Patentamt
European Patent Office
Office européen des brevets

(11) EP 4 434 991 A1

(12) EUROPEAN PATENT APPLICATION
published in accordance with Art. 153(4) EPC

(43) Date of publication:
**25.09.2024 Bulletin 2024/39**

(21) Application number: **22895570.4**

(22) Date of filing: **14.11.2022**

(51) International Patent Classification (IPC):
***C07D 491/048*** (2006.01) ***C09K 11/06*** (2006.01)
***C07D 519/00*** (2006.01) ***H10K 50/00*** (2023.01)

(52) Cooperative Patent Classification (CPC):
**C07D 491/048; C07D 519/00; C09K 11/06; H10K 50/00**

(86) International application number:
**PCT/JP2022/042231**

(87) International publication number:
**WO 2023/090288 (25.05.2023 Gazette 2023/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.11.2021 JP 2021188860**
**18.02.2022 JP 2022024099**

(71) Applicant: **Kyulux, Inc.**
**Fukuoka-shi, Fukuoka 819-0388 (JP)**

(72) Inventors:
- **YAMASHITA Masataka**
**Fukuoka-shi, Fukuoka 819-0388 (JP)**
- **SUZUKI Yoshitake**
**Fukuoka-shi, Fukuoka 819-0388 (JP)**
- **SHIMAMURA Naomi**
**Fukuoka-shi, Fukuoka 819-0388 (JP)**
- **OZAWA Hiroaki**
**Fukuoka-shi, Fukuoka 819-0388 (JP)**
- **HWANG Songhye**
**Fukuoka-shi, Fukuoka 819-0388 (JP)**
- **KANAHARA Kousei**
**Fukuoka-shi, Fukuoka 819-0388 (JP)**

(74) Representative: **Zimmermann & Partner Patentanwälte mbB**
**Postfach 330 920**
**80069 München (DE)**

# (54) COMPOUND, LIGHT-EMITTING MATERIAL AND LIGHT-EMITTING ELEMENT

(57) An organic light emitting device using a compound of the following general formula has excellent light emission characteristics. $R^1$ is a hydrogen atom, a deuterium atom or an alkyl group; $R^2$ to $R^4$ are donor groups, but not all are the same; $X^1$ to $X^3$ each are N or C(R); R is a hydrogen atom, a deuterium atom or a substituent; $Ar^1$ and $Ar^2$ each are an aryl group; $L^1$ is a single bond or a linking group.

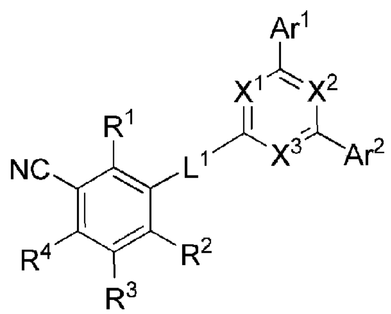

EP 4 434 991 A1

## Description

### Technical Field

The present invention relates to a compound useful as a light emitting material, and a light emitting device using the compound.

### Background Art

Studies for enhancing the light emission efficiency of light emitting devices such as organic electroluminescent devices (organic EL devices) are being made actively. In particular, various kinds of efforts have been made for increasing light emission efficiency by newly developing and combining an electron transporting material, a hole transporting material, and a light-emitting material to constitute an organic electroluminescent device. Among them, there are seen some reports relating to an organic electroluminescent device that utilizes a delayed fluorescent material.

A delayed fluorescent material is a material which, in an excited state, after having undergone reverse intersystem crossing from an excited triplet state to an excited singlet state, emits fluorescence when returning back from the excited singlet state to a ground state thereof. Fluorescence through the route is observed later than fluorescence from the excited singlet state directly occurring from the ground state (ordinary fluorescence), and is therefore referred to as delayed fluorescence. Here, for example, in the case where a light emitting compound is excited through carrier injection thereinto, the occurring probability of the excited singlet state to the excited triplet state is statistically 25%/75%, and therefore improvement of light emission efficiency by the fluorescence alone from the directly occurring excited singlet state is limited. On the other hand, in a delayed fluorescent material, not only the excited singlet state thereof but also the excited triplet state can be utilized for fluorescent emission through the route via the above-mentioned reverse intersystem crossing, and therefore as compared with an ordinary fluorescent material, a delayed fluorescent material can realize a higher emission efficiency.

Since such a principle has been clarified, various studies have led to the discovery of various delayed fluorescent materials. Among these, many compounds in which cyanobenzene is substituted with a donor group and an acceptor group are included. For example, a compound of cyanobenzene substituted with a benzofurocarbazolyl group as a donor group and with a diphenyltriazinyl group as an acceptor group has been proposed, and one example thereof is a compound having the following structure (see PTL 1).

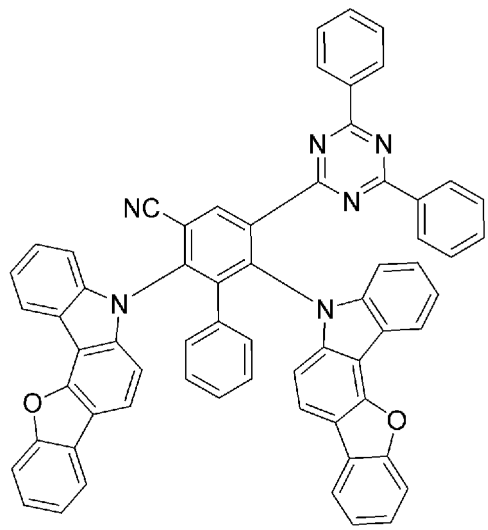

### Citation List

### Patent Literature

PTL 1: WO2021/045623A1

Summary of Invention

Technical Problem

Even if a material emits delayed fluorescence, one having extremely good characteristics and having no problem in practical use has not been provided. Therefore, for example, it is more useful if it is possible to provide a delayed fluorescent material having much better light emission characteristics than the delayed fluorescent material proposed in PTL 1. However, the improvement of delayed fluorescent materials is in the stage of trial and error, and it is not easy to generalize the chemical structure of useful light emitting materials.

Under such circumstances, the present inventors have conducted research for the purpose of providing a compound more useful as a light emitting material for a light emitting device. Then, the present inventors have conducted intensive studies for the purpose of deriving and generalizing a general formula of a compound more useful as a light emitting material.

Solution to Problem

As a result of intensive studies for achieving the above object, the present inventors have found that a cyanobenzene compound having a structure satisfying a specific condition is useful as a light emitting material. The present invention has been proposed based on these findings, and specifically has the following configuration.

[1] A compound represented by the following general formula (1):

General Formula (1)

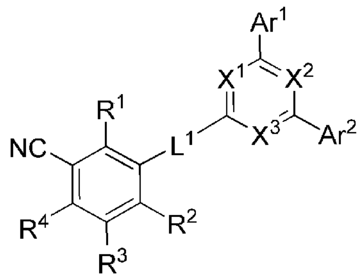

in which $R^1$ represents a hydrogen atom, a deuterium atom, or a substituted or unsubstituted alkyl group; $R^2$ to $R^4$ each independently represent a donor group that is not a substituted or unsubstituted aryl group, and $R^2$ to $R^4$ are not all the same; $X^1$ to $X^3$ each independently represent N or C(R), and at least one of $X^1$ to $X^3$ is N; R represents a hydrogen atom, a deuterium atom or a substituent; $Ar^1$ and $Ar^2$ each independently represent a substituted or unsubstituted aryl group, or a substituted or unsubstituted heteroaryl group; and $L^1$ represents a single bond or a divalent linking group.

[2] The compound according to [1], in which $R^2$ to $R^4$ each independently represent a substituted or unsubstituted diarylamino group, provided that the two aryl groups constituting the diarylamino group can bond to each other.

[3] The compound according to [2], in which $R^2$ to $R^4$ each independently represent a substituted or unsubstituted carbazol-9-yl group.

[4] The compound according to any one of [1] to [3], in which at least one of $R^2$ to $R^4$ is a substituted or unsubstituted ring-fused carbazol-9-yl group.

[5] The compound according to [4], in which at least one of $R^2$ to $R^4$ is a ring-fused carbazol-9-yl group substituted with one atom or group selected from the group consisting of a deuterium atom, an alkyl group and an aryl group or with a group formed by combining two or more thereof.

[6] The compound according to [5], in which the ring-fused carbazol-9-yl group is substituted with an aryl group optionally substituted with one atom or group selected from the group consisting of a deuterium atom, an alkyl group and an aryl group or with a group formed by combining two or more thereof.

[7] The compound according to any one of [4] to [6], in which the ring-fused carbazol-9-yl group is a benzofuro-ring-fused carbazol-9-yl group or a benzothieno-ring-fused carbazol-9-yl group.

[8] The compound according to any one of [1] to [7], in which at least one of $R^2$ to $R^4$ is a carbazol-9-yl group

optionally substituted with a deuterium atom.

[9] The compound according to any one of [1] to [8], in which $R^2$ and $R^3$ are the same.

[10] The compound according to any one of [1] to [9], in which $R^2$ and $R^4$ are the same.

[11] The compound according to any one of [1] to [10], in which $X^1$ to $X^3$ are N.

[12] The compound according to any one of [1] to [11], in which $Ar^1$ and $Ar^2$ each are an aryl group optionally substituted with a deuterium atom.

[13] The compound according to any one of [1] to [12], in which $L^1$ is a single bond.

[14] The compound according to any one of [1] to [13], in which $R^1$ is a hydrogen atom.

[15] The compound according to any one of [1] to [14], in which the compound has at least one deuterium atom.

[16] A light emitting material including the compound according to any one of [1] to [15].

[17] A delayed fluorescent material including the compound according to any one of [1] to [15].

[18] A film including the compound according to any one of [1] to [15].

[19] An organic semiconductor device including the compound according to any one of [1] to [15].

[20] An organic light emitting device including the compound according to any one of [1] to [15].

[21] The organic light emitting device according to [20], in which the device has a layer containing the compound, and the layer also contains a host material.

[22] The organic light emitting device according to [21], in which the layer containing the compound further contains a delayed fluorescent material in addition to the compound and the host material, and the delayed fluorescent material has a lowest excited singlet energy lower than that of the host material and higher than that of the compound.

[23] The organic light emitting device according to [21], in which the device has a layer containing the compound, and the layer also contains a light emitting material having a structure different from that of the compound.

[24] The organic light emitting device according to any one of [21] to [23], in which the amount of light emitted from the compound is the largest among materials contained in the device.

[25] The organic light emitting device according to [23], in which the amount of light emitted from the light emitting material is larger than the amount of light emitted from the compound.

[26] The organic light emitting device according to any one of [20] to [25], which is an organic electroluminescent device.

[27] The organic light emitting device according to any one of [20] to [26], which emits delayed fluorescence.

Advantageous Effects of Invention

The compound of the present invention is useful as a light emitting material. The compound of the present invention includes compounds having good light emission characteristics. Further, the organic light emitting device using the compound of the present invention includes excellent devices having a high light emission efficiency, devices having a low drive voltage and a good energy efficiency, and useful devices having a long device lifetime.

Description of Embodiments

The contents of the invention will be described in detail below. The constitutional elements may be described below with reference to representative embodiments and specific examples of the invention, but the invention is not limited to the embodiments and the specific examples. In the description herein, a numerical range expressed as "to" means a range that includes the numerical values described before and after "to" as the lower limit and the upper limit. A part or all of hydrogen atoms existing in the molecule of the compound for use in the present invention can be substituted with deuterium atoms ($^2H$, deuterium D). In the chemical structural formulae in the description herein, the hydrogen atom is expressed as H, or the expression thereof is omitted. For example, when expression of the atoms bonding to the ring skeleton-constituting carbon atoms of a benzene ring is omitted, H is considered to bond to the ring skeleton-constituting carbon atom at the site having the omitted expression. In the chemical structural formulae in the present description, a deuterium atom is expressed as D. The term "light emission characteristics" used in the present application refers to properties relating to light emission, such as light emission efficiency, drive voltage, and light emission lifetime. The compound of the present invention is excellent in at least one light emission characteristic.

[Compound Represented by General Formula (1)]

General Formula (1)

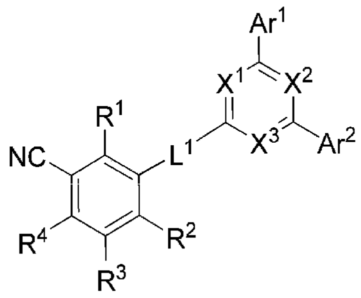

In the general formula (1), $R^1$ represents a hydrogen atom, a deuterium atom, or a substituted or unsubstituted alkyl group. In one aspect of the present invention, $R^1$ is a hydrogen atom. In one aspect of the present invention, $R^1$ is a deuterium atom . In one aspect of the present invention, $R^1$ is a substituted or unsubstituted alkyl group. Regarding the description and the preferred range of the alkyl group which can be employed by $R^1$, reference can be made to the section of the alkyl group in the description of the donor group to be given hereinunder. Preferably, $R^1$ is a hydrogen atom or a deuterium atom.

$R^2$ to $R^4$ each independently represent a donor group, and the donor group as referred to herein does not include a substituted or unsubstituted aryl group.

The "donor group" can be selected from groups having a negative Hammett's $\sigma p$ value. The Hammett's $\sigma p$ value is proposed by L. P. Hammett and quantifies the influence of a substituent on the reaction rate or equilibrium of a para-substituted benzene derivative. Specifically, the value is a constant ($\sigma p$) peculiar to the substituent in the following equation that is established between a substituent and a reaction rate constant or an equilibrium constant in a para-substituted benzene derivative:

$$\log(k/k_0) = \rho\sigma p$$

or

$$\log(K/K_0) = \rho\sigma p$$

In the above equations, $k_0$ represents a rate constant of a benzene derivative not having a substituent; k represents a rate constant of a benzene derivative substituted with a substituent; $K_0$ represents an equilibrium constant of a benzene derivative not having a substituent; K represents an equilibrium constant of a benzene derivative substituted with a substituent; and $\rho$ represents a reaction constant to be determined by the kind and the condition of reaction. Regarding the description relating to the "Hammett's $\sigma p$ value" and the numerical value of each substituent in the present invention, reference can be made to the description relating to $\sigma p$ value in Hansch, C. et. al., Chem. Rev., 91, 165-195 (1991).

The donor group which can be employed by $R^2$ to $R^4$ preferably has $\sigma p$ of -0.3 or less, more preferably -0.5 or less, and even more preferably -0.7 or less. For example, the value can be selected from a range of -0.9 or less, or from a range of -1.1 or less.

The donor group in the present invention is preferably a group containing a substituted amino group. The donor group can be a substituted amino group, or can be a substituted amino group-bonded aryl group, especially a substituted amino group-bonded phenyl group. In one preferred aspect of the present invention, the donor group is a substituted amino group.

The substituent bonding to the nitrogen atom of a substituted amino group is preferably a substituted or unsubstituted alkyl group, a substituted or unsubstituted alkenyl group, a substituted or unsubstituted aryl group, or a substituted or unsubstituted heteroaryl group, more preferably a substituted or unsubstituted aryl group or a substituted or unsubstituted heteroaryl group. Especially, the substituted amino group is preferably a substituted or unsubstituted diarylamino group, or a substituted or unsubstituted diheteroarylamino group. As referred to herein, the two aryl groups constituting the diarylamino group can bond to each other, and the two heteroaryl groups constituting the diheteroarylamino group can bond to each other.

The "aryl group" can be a monocyclic ring or a fused ring in which two or more rings are fused. In the case of a fused ring, the number of fused rings is preferably 2 to 6, and can be selected from, for example, 2 to 4. Specific

examples of the ring include a benzene ring, a naphthalene ring, an anthracene ring, a phenanthrene ring, and a triphenylene ring. In one aspect of the present invention, the aryl group is a substituted or unsubstituted phenyl group, a substituted or unsubstituted naphthalen-1-yl group, or a substituted or unsubstituted naphthalen-2-yl group, and is preferably a substituted or unsubstituted phenyl group. For example, the substituent for the aryl group can be selected from Substituent Group A, can be selected from Substituent Group B, can be selected from Substituent Group C, can be selected from Substituent Group D, or can be selected from Substituent Group E. In one aspect of the present invention, the substituent for the aryl group is at least one selected from the group consisting of an alkyl group, an aryl group and a deuterium atom. In one preferred aspect of the present invention, the aryl group is unsubstituted.

The "heteroaryl group" can be a monocyclic ring or a fused ring in which two or more rings are fused. In the case of a fused ring, the number of fused rings is preferably 2 to 6, and can be selected from, for example, 2 to 4. Specific examples of the ring include a pyridine ring and a pyrimidine ring, and these rings can be fused with any other ring. Specific examples of the heteroaryl group include a 2-pyridyl group, a 3-pyridyl group and a 4-pyridyl group. The number of the ring skeleton-constituting atoms of the heteroaryl group is preferably 4 to 40, more preferably 5 to 20, and can be selected from a range of 5 to 14, or can be selected from a range of 5 to 10.

The "alkyl group" can be any of linear, branched or cyclic ones. Two or more of a linear moiety, a cyclic moiety and a branched moiety can be in the group as mixed. The carbon number of the alkyl group can be, for example, 1 or more, 2 or more, or 4 or more. The carbon number can also be 30 or less, 20 or less, 10 or less, 6 or less, or 4 or less. Specific examples of the alkyl group include a methyl group, an ethyl group, an n-propyl group, an isopropyl group, an n-butyl group, an isobutyl group, a tert-butyl group, an n-pentyl group, an isopentyl group, an n-hexyl group, an isohexyl group, a 2-ethylhexyl group, an n-heptyl group, an isoheptyl group, an n-octyl group, an isooctyl group, an n-nonyl group, an isononyl group, an n-decanyl group, an isodecanyl group, a cyclopentyl group, a cyclohexyl group, and a cycloheptyl group. The alkyl group which is the substituent can be further substituted with, for example, a deuterium atom, an aryl group, an alkoxy group, an aryloxy group, and a halogen atom. In one aspect of the present invention, the substituent for the alkyl group is at least one selected from the group consisting of an aryl group and a deuterium atom. In one preferred aspect of the present invention, the alkyl group is unsubstituted.

The "alkenyl group" can be linear, branched or cyclic. Two or more of a linear moiety, a cyclic moiety and a branched moiety can be in the group as mixed. The carbon number of the alkenyl group can be, for example, 2 or more, or 4 or more. The carbon number can also be 30 or less, 20 or less, 10 or less, 6 or less, or 4 or less. Specific examples of the alkenyl group include an ethenyl group, an n-propenyl group, an isopropenyl group, an n-butenyl group, an isobutenyl group, an n-pentenyl group, an isopentenyl group, an n-hexenyl group, an isohexenyl group, and a 2-ethylhexenyl group. The alkenyl group which is the substituent can be further substituted with a substituent.

The donor group which can be employed by $R^2$ to $R^4$ is preferably a group represented by the following general formula (a).

General Formula (a)

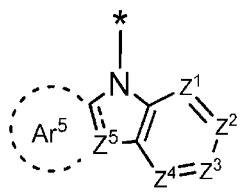

In the general formula (a), $Z^1$ represents C-$R^{14}$ or N, $Z^2$ represents C-$R^{15}$ or N, $Z^3$ represents C-$R^{16}$ or N, and $Z^4$ represents C-$R^{17}$ or N. $Z^5$ represents C or N, $Ar^5$ represents a substituted or unsubstituted aromatic ring or a substituted or unsubstituted heteroaromatic ring. $R^{14}$ and $R^{15}$, $R^{15}$ and $R^{16}$, and $R^{16}$ and $R^{17}$ each can bond to each other to form a cyclic structure.

Among $Z^1$ to $Z^4$, the number of groups represented by N is preferably 0 to 3, and preferably 0 to 2. In one aspect of the present invention, among $Z^1$ to $Z^4$, the number of groups represented by N is 1. In one aspect of the present invention, among $Z^1$ to $Z^4$, the number of groups represented by N is 0.

$R^{14}$ to $R^{17}$ each independently represent a hydrogen atom, a deuterium atom, or a substituent.

For example, the substituent can be selected from Substituent Group A, can be selected from Substituent Group B, can be selected from Substituent Group C, can be selected from Substituent Group D, or can be selected from Substituent Group E. When two or more of $R^{14}$ to $R^{17}$ represent substituents, the two or more substituents can be the same or different. Zero to two of $R^{14}$ to $R^{17}$ are preferably a substituent, and for example, one can be a substituent, or zero can be a substituent ($R^{14}$ to $R^{17}$ are a hydrogen atom or a deuterium atom).

$R^{14}$ and $R^{15}$, $R^{15}$ and $R^{16}$, and $R^{16}$ and $R^{17}$ each can bond to each other to form a cyclic structure. The cyclic structure can be any of an aromatic ring, an heteroaromatic ring, an aliphatic hydrocarbon ring, and an aliphatic heterocyclic ring, and can be a ring obtained by fusing these rings. The structure is preferably an aromatic ring or a heteroaromatic ring. Examples of the aromatic ring include a substituted or unsubstituted benzene ring. Another benzene ring can be further fused to the benzene ring, and a heterocyclic ring such as a pyridine ring can be fused to the benzene ring. The heteroaromatic ring means a ring exhibiting aromaticity including a heteroatom as a ring skeleton-constituting atom, and is preferably a 5- to 7-membered ring, and for example, a 5-membered ring or a 6-membered ring can be employed. In one aspect of the present invention, a furan ring, a thiophene ring, or a pyrrole ring can be employed as the heteroaromatic ring. In one preferred aspect of the present invention, the cyclic structure is a furan ring of a substituted or unsubstituted benzofuran, a thiophene ring of a substituted or unsubstituted benzothiophene, or a pyrrole ring of a substituted or unsubstituted indole. The benzofuran, benzothiophene, and indole referred to herein can be unsubstituted, can be substituted with a substituent selected from Substituent Group A, can be substituted with a substituent selected from Substituent Group B, can be substituted with a substituent selected from Substituent Group C, can be substituted with a substituent selected from Substituent Group D, and can be substituted with a substituent selected from Substituent Group E. It is preferable that a substituted or unsubstituted aryl group bonds to the nitrogen atom constituting the pyrrole ring of indole, and examples of the substituent include a substituent selected from any of Substituent Group A to Substituent Group E. The cyclic structure can be a substituted or unsubstituted cyclopentadiene ring. In one aspect of the present invention, a pair of $R^{14}$ and $R^{15}$, $R^{15}$ and $R^{16}$, and $R^{16}$ and $R^{17}$ bonds to each other to form a cyclic structure. In one aspect of the present invention, none of $R^{14}$ and $R^{15}$, $R^{15}$ and $R^{16}$, and $R^{16}$ and $R^{17}$ bonds to each other to form a cyclic structure.

In the general formula (a), $Z^5$ represents C or N, $Ar^5$ represents a substituted or unsubstituted aromatic ring or a substituted or unsubstituted heteroaromatic ring. In one aspect of the present invention, $Z^5$ is C, and $Ar^5$ is a substituted or unsubstituted aromatic ring or a substituted or unsubstituted heteroaromatic ring. In one aspect of the present invention, $Z^5$ is N, and $Ar^5$ is a substituted or unsubstituted heteroaromatic ring.

Examples of the aromatic ring which is employable by $Ar^5$ include a benzene ring. Another benzene ring can be further fused to the benzene ring, and a heterocyclic ring such as a pyridine ring can be fused to the benzene ring. The heteroaromatic ring which is employable by $Ar^5$ is preferably a 5- to 7-membered ring., and for example, a 5-membered ring or a 6-membered ring can be employed. In one aspect of the present invention, as the heteroaromatic ring, a furan ring, a thiophene ring, a pyrrole ring, an imidazole ring, a pyridine ring, a pyridazine ring, a pyrimidine ring, or a pyrazine ring can be employed. In one aspect of the present invention, $Z^5$ is C, and the heteroaromatic ring is a furan ring of a substituted or unsubstituted benzofuran, a thiophene ring of a substituted or unsubstituted benzothiophene, a pyridine ring of a substituted or unsubstituted quinoline, or a pyridine ring of a substituted or unsubstituted isoquinoline. In one aspect of the present invention, $Z^5$ is N, and the heteroaromatic ring is a pyrrole ring of a substituted or unsubstituted indole, or an imidazole ring of a substituted or unsubstituted benzimidazole. The benzofuran, benzothiophene, quinoline, isoquinoline, indole and benzimidazole referred to herein can be unsubstituted, or can be substituted with a substituent selected from Substituent Group A, can be substituted with a substituent selected from Substituent Group B, can be substituted with a substituent selected from Substituent Group C, can be substituted with a substituent selected from Substituent Group D, and can be substituted with a substituent selected from Substituent Group E.

When $Z^5$ in the general formula (a) is C, a group represented by the following general formula (b) is preferred.

General Formula (b)

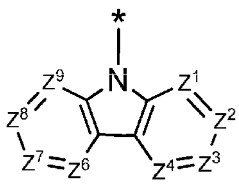

In the general formula (b), $Z^1$ represents C-$R^{14}$ or N, $Z^2$ represents C-$R^{15}$ or N, $Z^3$ represents C-$R^{16}$ or N, $Z^4$ represents C-$R^{17}$ or N, $Z^6$ represents C-$R^{18}$ or N, $Z^7$ represents C-$R^{19}$ or N, $Z^8$ represents C-$R^{20}$ or N, and $Z^9$ represents C-$R^{21}$ or N. $R^{14}$ and $R^{15}$, $R^{15}$ and $R^{16}$, $R^{16}$ and $R^{17}$, $R^{18}$ and $R^{19}$, $R^{19}$ and $R^{20}$ , and $R^{20}$ and $R^{21}$ each can bond to each other to form a cyclic structure.

For $Z^1$ to $Z^4$ and $R^{14}$ to $R^{17}$ in the general formula (b), the corresponding description of the general formula (a) can be referred to. $Z^6$ to $Z^9$ and $R^{18}$ to $R^{21}$ in the general formula (b) correspond to $Z^1$ to $Z^4$ and $R^{14}$ to $R^{17}$ in the general formula (a), respectively, and for the contents thereof, reference can be made to the descriptions of $Z^1$ to $Z^4$ and $R^{14}$ to $R^{17}$ in the general formula (a).

In one aspect of the present invention, among $Z^1$ to $Z^4$ and $Z^6$ to $Z^9$, the number of groups represented by N is preferably 0 to 2, and more preferably 0 or 1. In one aspect of the present invention, among $Z^1$ to $Z^4$ and $Z^6$ to $Z^9$, the number of groups represented by N is 1. In one preferred aspect of the present invention, among $Z^1$ to $Z^4$ and $Z^6$ to $Z^9$, the number of groups represented by N is 0. When the number is 0, the formula represents a substituted or unsubstituted carbazol-9-yl group.

Preferably, $R^2$ to $R^4$ each independently represent a substituted or unsubstituted carbazol-9-yl group. The carbazol-9-yl group referred to herein can be unsubstituted, or can be substituted with a substituent selected from Substituent Group A, can be substituted with a substituent selected from Substituent Group B, can be substituted with a substituent selected from Substituent Group C, can be substituted with a substituent selected from Substituent Group D, and can be substituted with a substituent selected from Substituent Group E. Further one or more rings can be fused to the two benzene rings constituting the carbazol-9-yl group. In one preferred aspect of the present invention, $R^2$ to $R^4$ are each independently a carbazol-9-yl group optionally substituted with a group selected from Substituent Group E, and optionally fused with one or more rings. In the case where the carbazol-9-yl group not fused with a ring is substituted, the substitution site is not specifically limited, but is preferably at least one of 2 to 7-positions, more preferably at least one of 3 to 6-positions, even more preferably a 3-position and a 6-position.

In one preferred aspect of the present invention, at least one of $R^2$ to $R^4$ is a carbazol-9-yl group fused with one or more rings, and hereinunder this is referred to as "ring-fused carbazol-9-yl group". The ring-fused carbazol-9-yl group which $R^2$ to $R^4$ can employ can be unsubstituted, or can be substituted with a substituent selected from Substituent Group A, can be substituted with a substituent selected from Substituent Group B, can be substituted with a substituent selected from Substituent Group C, can be substituted with a substituent selected from Substituent Group D, and can be substituted with a substituent selected from Substituent Group E. Preferably, the group is unsubstituted, or substituted with a substituent selected from Substituent Group E. In one aspect of the present invention, the ring-fused carbazol-9-yl group is unsubstituted. In one preferred aspect of the present invention, the ring-fused carbazol-9-yl group is substituted with an aryl group optionally substituted with one atom or group selected from the group consisting of a deuterium atom, an alkyl group and an aryl group or with a group formed by combining two or more thereof.

The number of rings constituting the fused ring in the ring-fused carbazol-9-yl group is 4 or more, preferably 5 or more, more preferably 5 to 9, even more preferably 5 to 7. In one preferred aspect of the present invention, the number of rings constituting the fused ring is 5. Here, the number of rings includes the number of rings of carbazole to be fused (i.e. 3).

The ring-fused carbazol-9-yl group is a group that bonds via the nitrogen atom constituting the ring skeleton of carbazole, and has a structure in which a ring is fused to at least one of the two benzene rings constituting carbazole. The fused ring can be any of an aromatic hydrocarbon ring, an aromatic heterocyclic ring, an aliphatic hydrocarbon ring, and an aliphatic heterocyclic ring, and can be a ring obtained by further fusing these rings. An aromatic hydrocarbon ring and an aromatic heterocyclic ring are preferable. Examples of the aromatic hydrocarbon ring include a substituted or unsubstituted benzene ring. Another benzene ring can be further fused to the benzene ring, and a heterocyclic ring such as a pyridine ring can be fused to the benzene ring. The aromatic heterocyclic ring means a ring exhibiting aromaticity including a heteroatom as a ring skeleton-constituting atom, and is preferably a 5- to 7-membered ring, and for example, a 5-membered ring or a 6-membered ring can be employed. In one aspect of the present invention, a furan ring, a thiophene ring, or a pyrrole ring can be employed as the aromatic heterocyclic ring. In one aspect of the present invention, the fused ring is a furan ring of a substituted or unsubstituted benzofuran, a thiophene ring of a substituted or unsubstituted benzothiophene, or a pyrrole ring of a substituted or unsubstituted indole. It is preferable that a substituent selected from Substituent Group E (but except for a deuterium atom alone) bonds to the nitrogen atom of the pyrrole ring, and it is more preferable that an aryl group which can be substituted with an alkyl group or an aryl group is bonded. In the present invention, it is preferable to employ a carbazol-9-yl group in which a ring having one or more atoms selected from the group consisting of an oxygen atom, a sulfur atom, and a nitrogen atom as a ring skeleton-constituting atom is fused. Above all, preferably employed are a benzofuro structure-fused carbazol-9-yl group, a benzothieno structure-fused carbazol-9-yl group, and an indolo structure-fused carbazol-9-yl group. In one aspect of the present invention, the compound has at least one benzofuro structure-fused carbazol-9-yl group, and for example, has two or more such groups. In one aspect of the present invention, the compound has at least one benzothieno structure-fused carbazol-9-yl group, and for example, has two or more such groups.

In the present invention, a benzofuro[2,3-a]carbazol-9-yl group can be employed as the ring-fused carbazol-9-yl group. Also a benzofuro[3,2-a]carbazol-9-yl group can be employed. Also a benzofuro[2,3-b]carbazol-9-yl group can be employed. Also a benzofuro[3,2-b]carbazol-9-yl group can be employed. Also a benzofuro[2,3-c]carbazol-9-yl group can be employed. Also a benzofuro[3,2-c]carbazol-9-yl group can be employed.

A preferred benzofuro-fused carbazol-9-yl group is a carbazol-9-yl group in which only one benzofuran ring is fused at the 2,3-positions and no ring is fused to the other. Specifically, it is a group having any one of the following structures, and at least one hydrogen atom in the following structure may or may not be substituted.

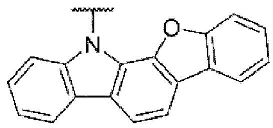 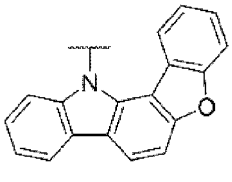 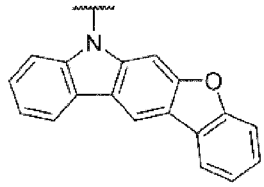

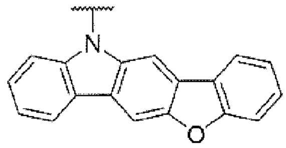 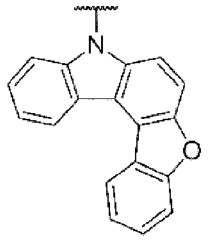 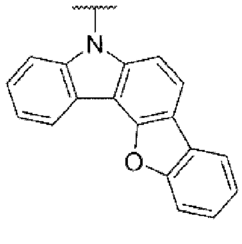

In the present invention, a benzothieno[2,3-a]carbazol-9-yl group can be employed as the ring-fused carbazol-9-yl group. Also a benzothieno[3,2-a]carbazol-9-yl group can be employed. Also a benzothieno[2,3-b]carbazol-9-yl group can be employed. Also a benzothieno[3,2-b]carbazol-9-yl group can be employed. Also a substituted or unsubstituted benzothieno[2,3-c]carbazol-9-yl group can be employed. Also a benzothieno[3,2-c]carbazol-9-yl group can be employed.

A preferred benzothieno-fused carbazol-9-yl group is a carbazol-9-yl group in which only one benzothiophene ring is fused at the 2,3-positions and no ring is fused to the other. Specifically, it is a group having any one of the following structures, and at least one hydrogen atom in the following structure may or may not be substituted.

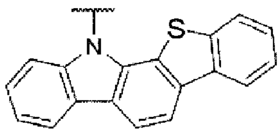 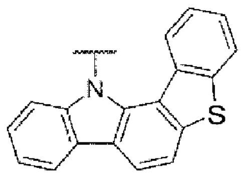 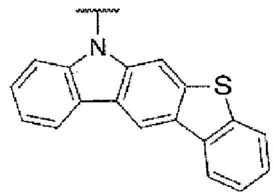

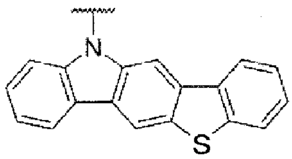 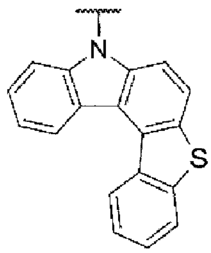 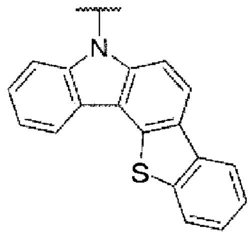

In the present invention, an indolo[2,3-a]carbazol-9-yl group can be employed, as the ring-fused carbazol-9-yl group. Also an indolo[3,2-a]carbazol-9-yl group can be employed. Also an indolo[2,3-b]carbazol-9-yl group can be employed. Also an indolo[3,2-b]carbazol-9-yl group can be employed. Also an indolo[2,3-c]carbazol-9-yl group can be employed. Also an indolo[3,2-c]carbazol-9-yl group can be employed.

A preferred indole-fused carbazol-9-yl group is a carbazol-9-yl group in which only one indole ring is fused at the 2,3-positions and no ring is fused to the other. Specifically, it is a group having any one of the following structures, and at least one hydrogen atom in the following structure may or may not be substituted. R' in the following structures represents a hydrogen atom, or a substituent. In one aspect of the present invention, R' is a substituted or unsubstituted aryl group or a substituted or unsubstituted alkyl group, and is preferably a substituted or unsubstituted aryl group. The substituent for the aryl group and the alkyl group can be selected from Substituent Group A, can be selected from Substituent Group B, can be selected from Substituent Group C, can be selected from Substituent Group D, or can be selected from Substituent Group E (but except for a deuterium atom alone). In one preferred aspect of the present invention, the aryl group and the alkyl group are unsubstituted.

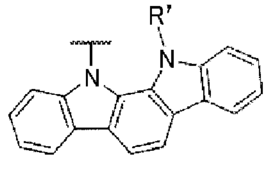 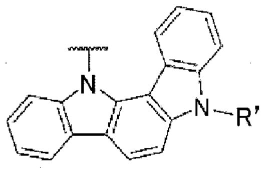 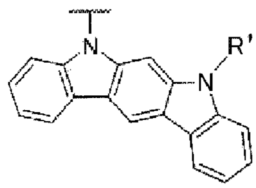

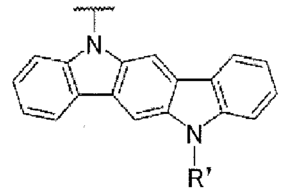 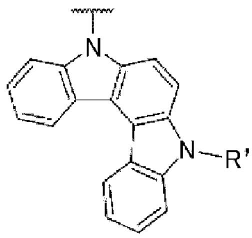 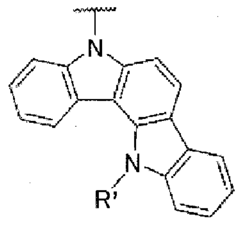

A substituent can bond to at least one ring skeleton-constituting carbon atom that constitutes the ring-fused carbazol-9-yl group. For example, the substituent for the ring-fused carbazol-9-yl group can be selected from Substituent Group A, can be selected from Substituent Group B, can be selected from Substituent Group C, can be selected from Substituent Group D, or can be selected from Substituent Group E. In one preferred aspect of the present invention, the substituent for the ring-fused carbazol-9-yl group can be selected from a substituted or unsubstituted aryl group and a substituted or unsubstituted alkyl group, and a part or all of the hydrogen atoms of these substituents can be substituted with deuterium atoms. Regarding the description and the preferred range of the alkyl group and the aryl group as referred to herein, reference can be made to the description of the alkyl group and the aryl group of the donor group described hereinabove. In one preferred aspect of the present invention, the ring-fused carbazol-9-yl group does not have any other substituent than those described herein.

The number of the substituents substituted on the ring-fused carbazol-9-yl group is preferably 1 to 10, more preferably 1 to 6, even more preferably 1 to 4, and can be, for example 1, or can be, for example 2. In one preferred aspect of the present invention, any of the 3-position or the 6-position of the ring-fused carbazol-9-yl group is substituted. In one preferred aspect of the present invention, the compound has at least one substituent on the para-position of the benzene ring viewed from the heteroatom present in the ring-fused carbazol-9-yl group. In one preferred aspect of the present invention, the compound has at least one substituent only on the para-position of the benzene ring viewed from the heteroatom present in the ring-fused carbazol-9-yl group. In one preferred aspect of the present invention, the compound has substituents on all the substitutable para-positions of the benzene ring viewed from the heteroatom present in the ring-fused carbazol-9-yl group.

Specific examples of the optionally-substituted ring-fused carbazol-9-yl group which can be employed as $R^2$ to $R^4$ in the general formula (1) are shown below. However, the optionally-substituted ring-fused carbazol-9-yl group which can be employed in the present invention shall not be construed as being limited by the following specific examples. In the following specific examples, * indicates a bonding site, and Ph represents a phenyl group. A methyl group is not shown. Accordingly, D218 to D241 have a methyl group.

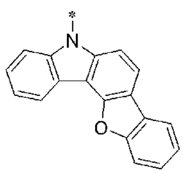
D1

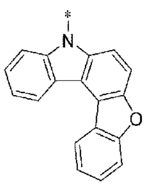
D2

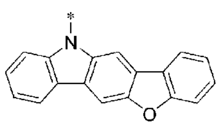
D3

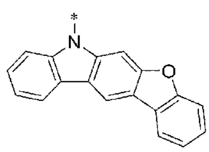
D4

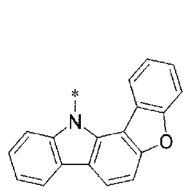
D5

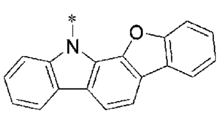
D6

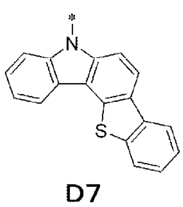
D7

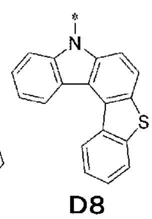
D8

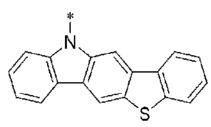
D9

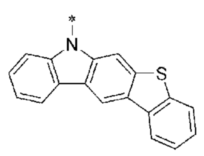
D10

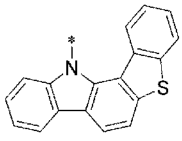
D11
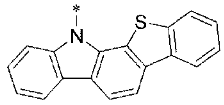
D12
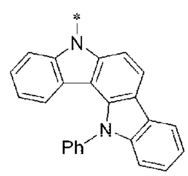
D13
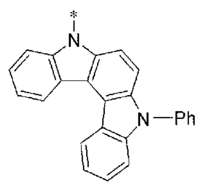
D14

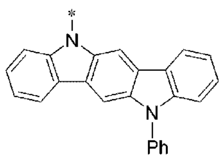
D15
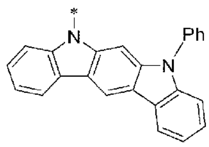
D16
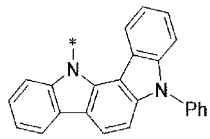
D17
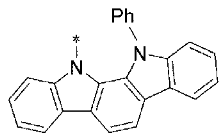
D18

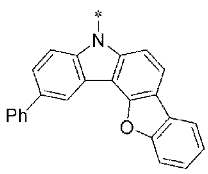
D19
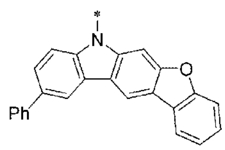
D20
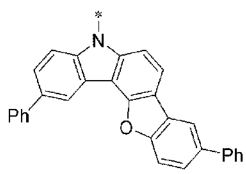
D21
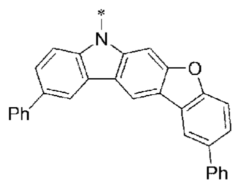
D22

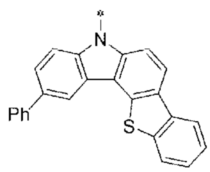
D23
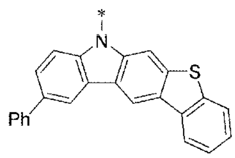
D24
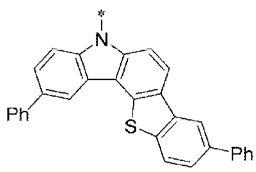
D25
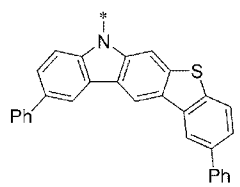
D26

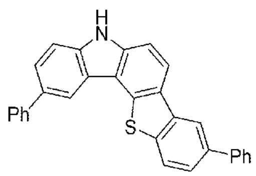
D27
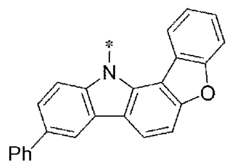
D28
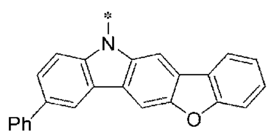
D29
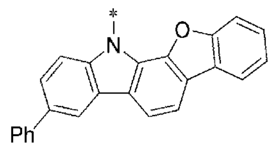
D30

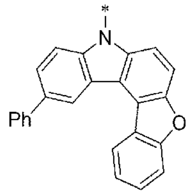
D31
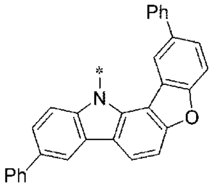
D32
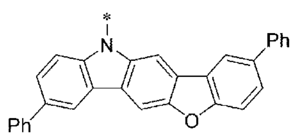
D33
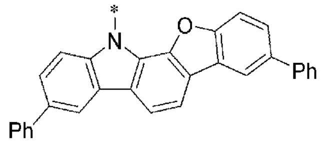
D34

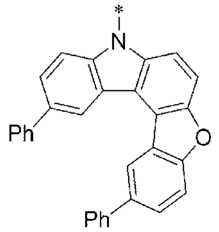
D35
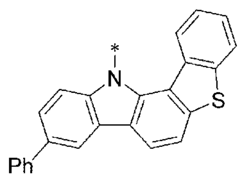
D36
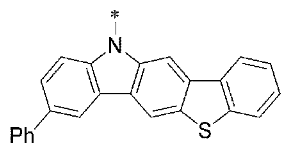
D37
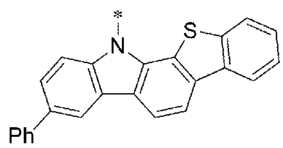
D38

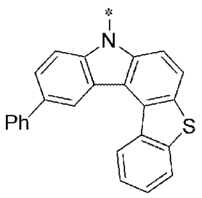

D39

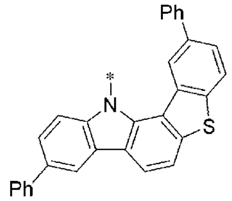

D40

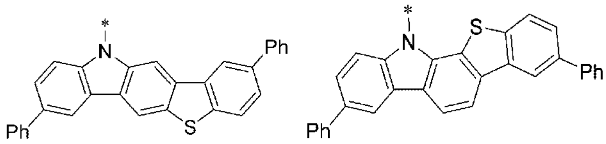

D41

D42

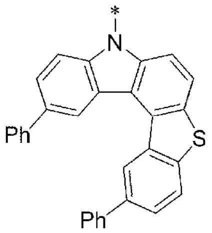

D43

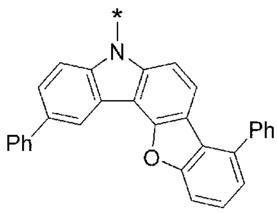

D44

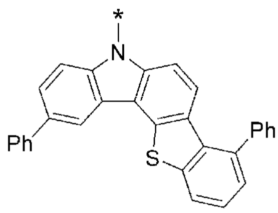

D45

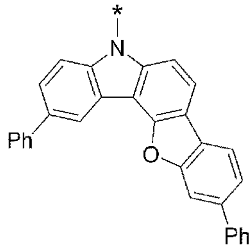

D46

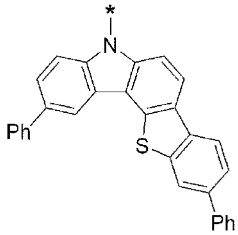

D47

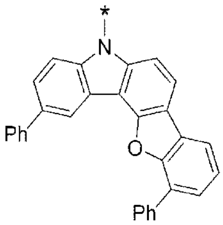

D48

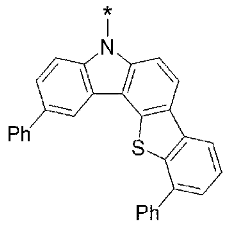

D49

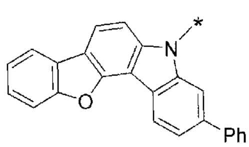

D50

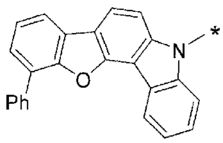

D51

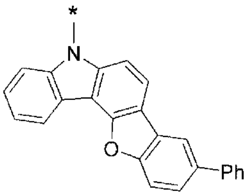

D52

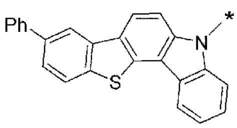

D53

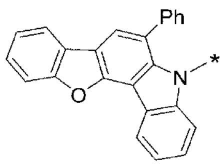

D54

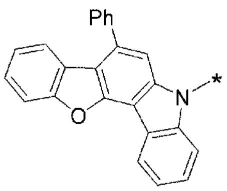

D55

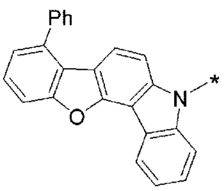

D56

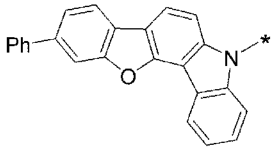

D57

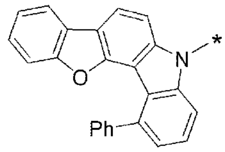

D58

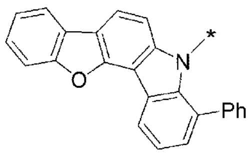

D59

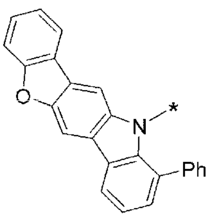

D60

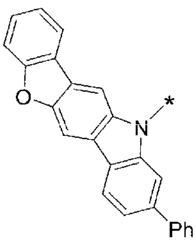

D61

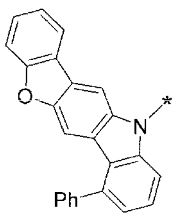

D62

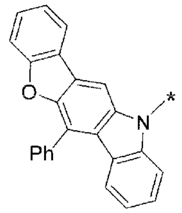
D63

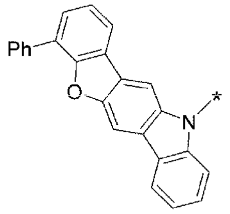
D64

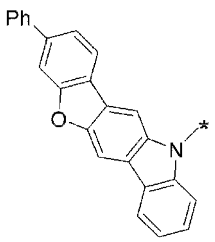
D65

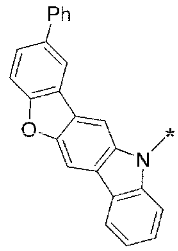
D66

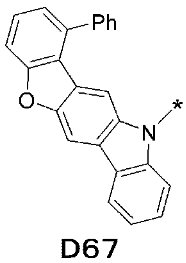
D67

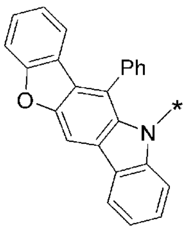
D68

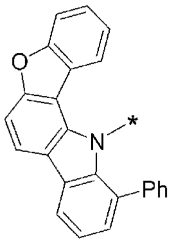
D69

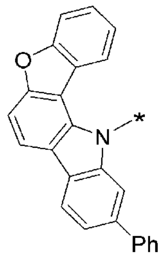
D70

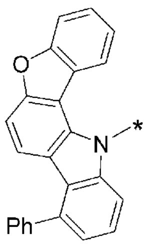
D71

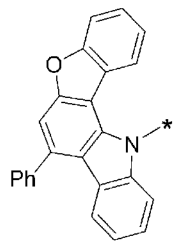
D72

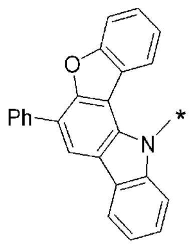
D73

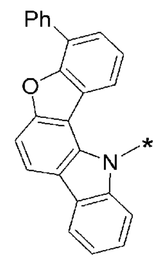
D74

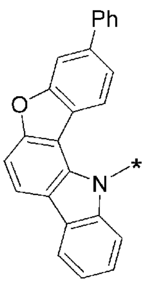
D75

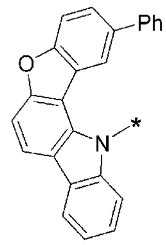
D76

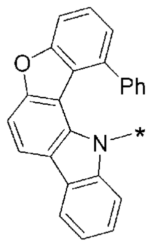
D77

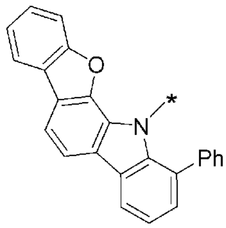
D78

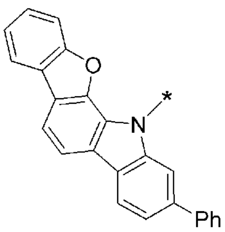
D79

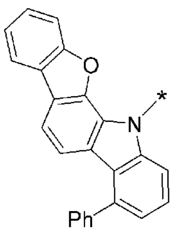
D80

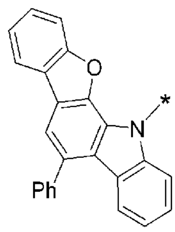
D81

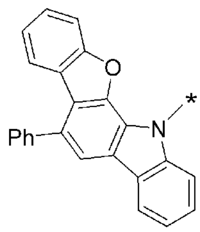
D82

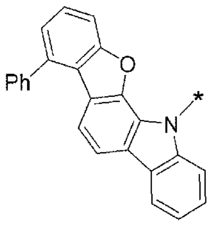
D83
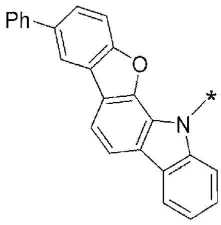
D84
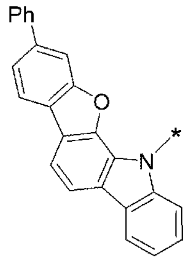
D85
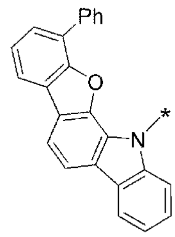
D86

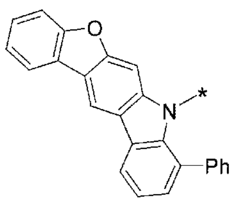
D87
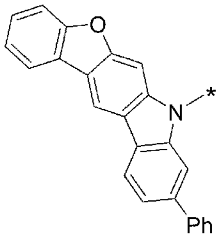
D88
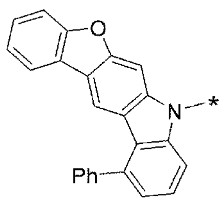
D89
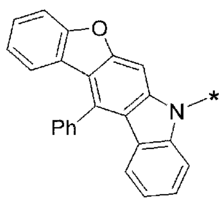
D90

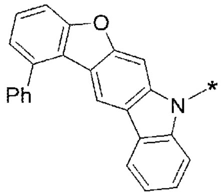
D91
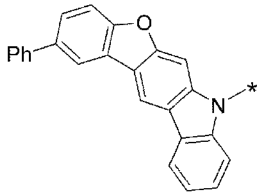
D92
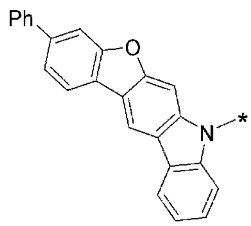
D93
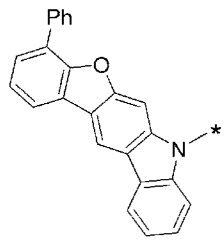
D94

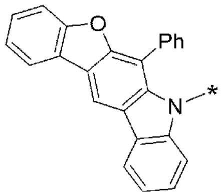
D95
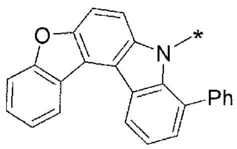
D96
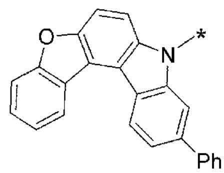
D97
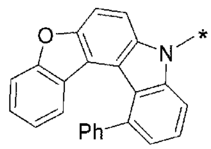
D98

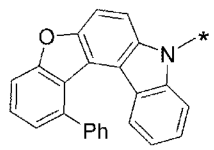
D99
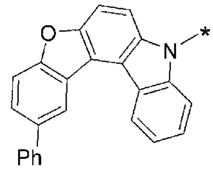
D100
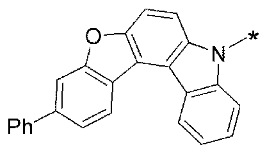
D101
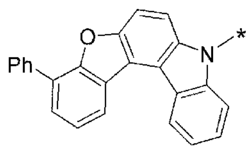
D102

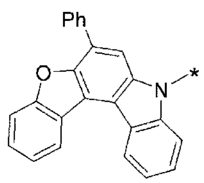
D103
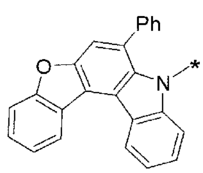
D104
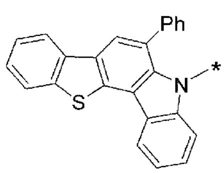
D105
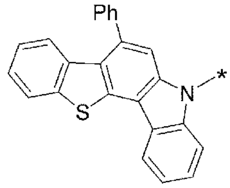
D106

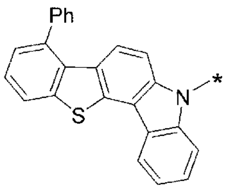
D107

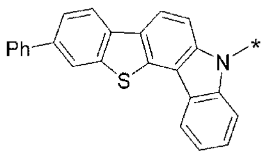
D108

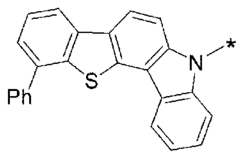
D109

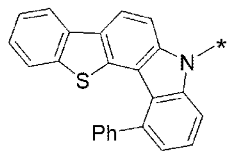
D110

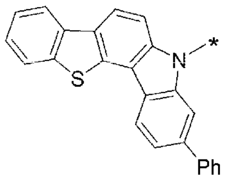
D111

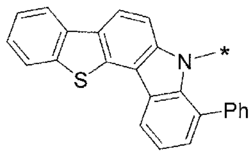
D112

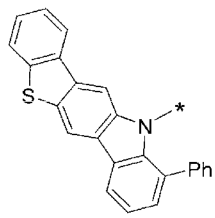
D113

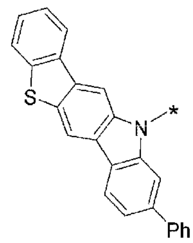
D114

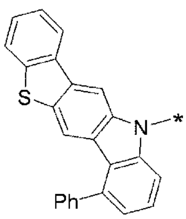
D115

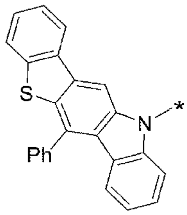
D116

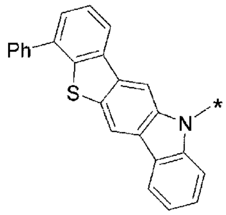
D117

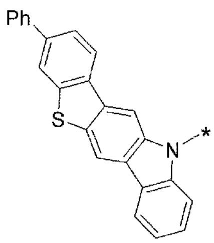
D118

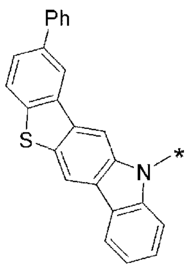
D119

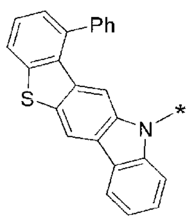
D120

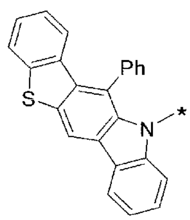
D121

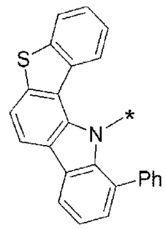
D122

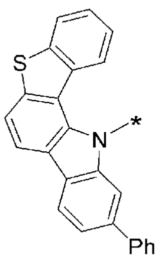
D123

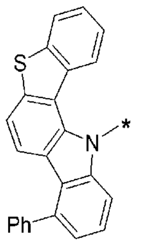
D124

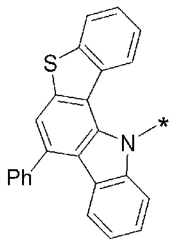
D125

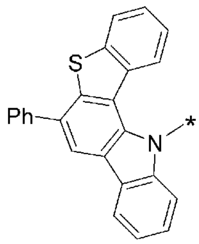
D126

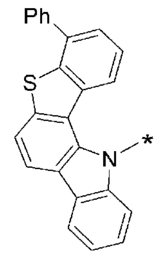

D127

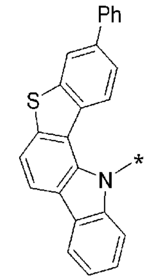

D128

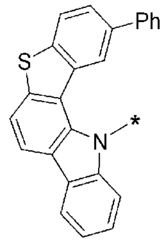

D129

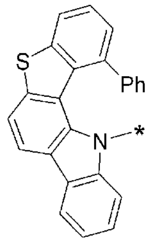

D130

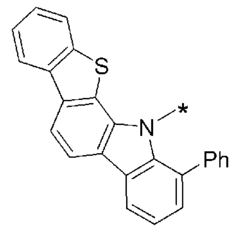

D131

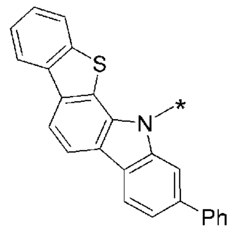

D132

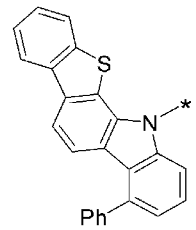

D133

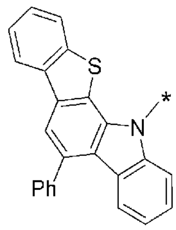

D134

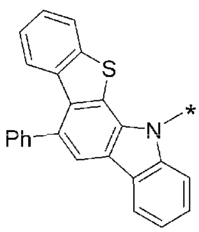

D135

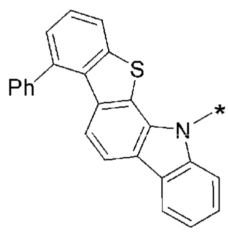

D136

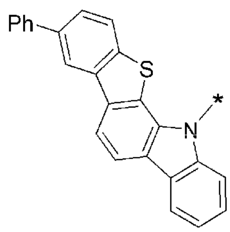

D137

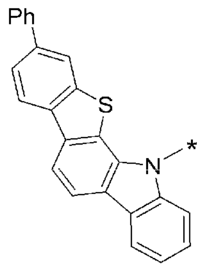

D138

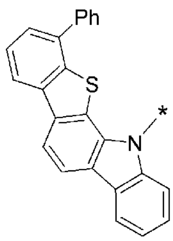

D139

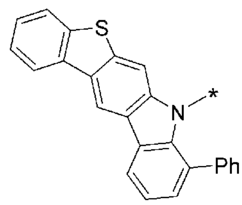

D140

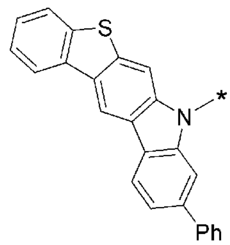

D141

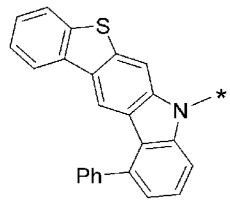

D142

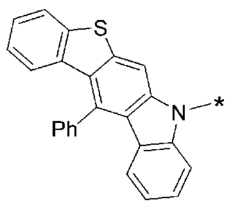

D143

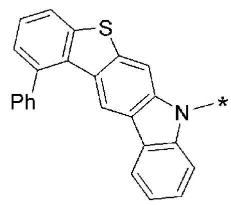

D144

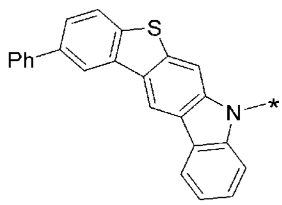

D145

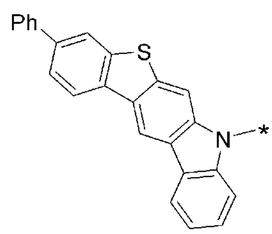

D146

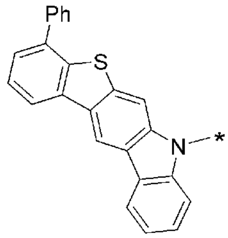 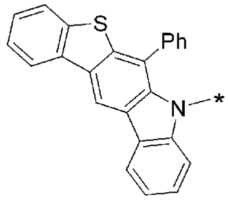 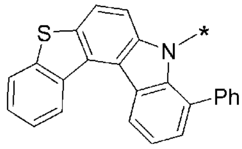 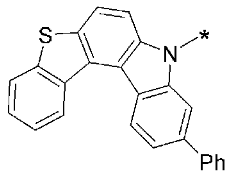

D147 D148 D149 D150

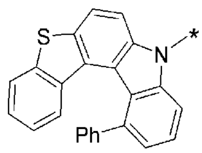 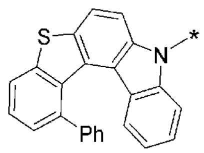 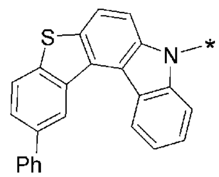 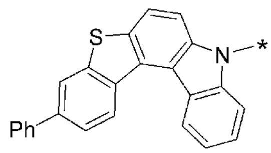

D151 D152 D153 D154

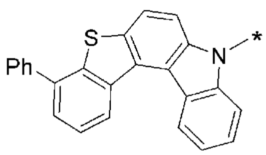 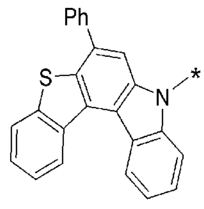 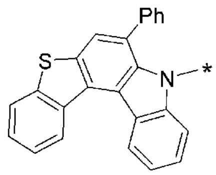 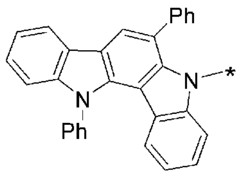

D155 D156 D157 D158

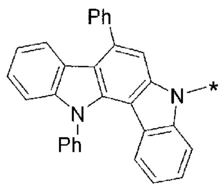 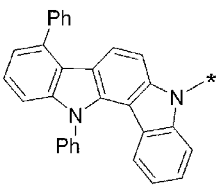 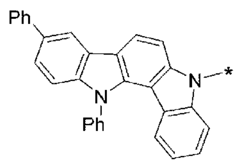 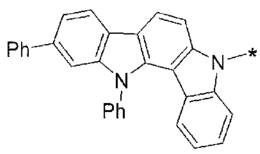

D159 D160 D161 D162

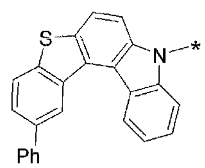 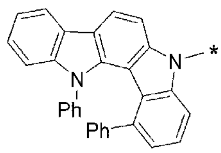 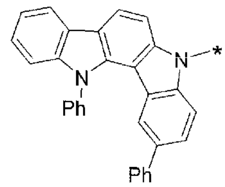 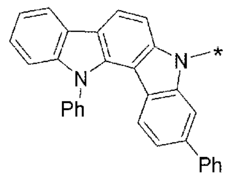

D163 D164 D165 D166

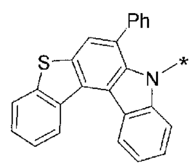 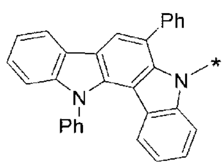 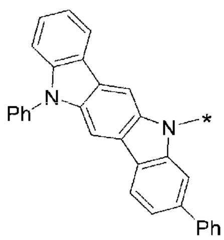 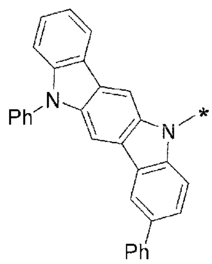

D167 D168 D169 D170

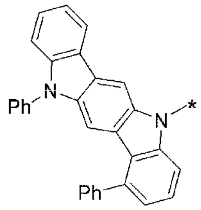
D171

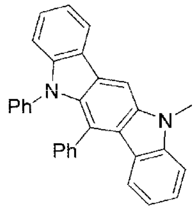
D172

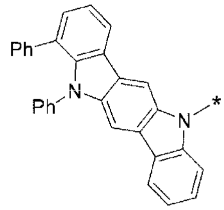
D173

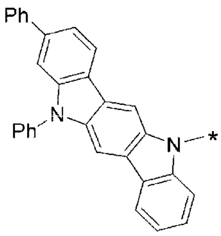
D174

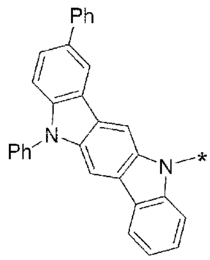
D175

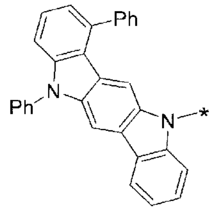
D176

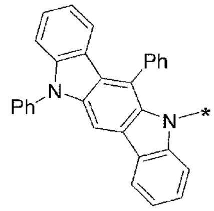
D177

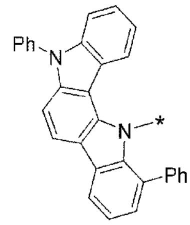
D178

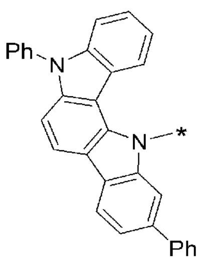
D179

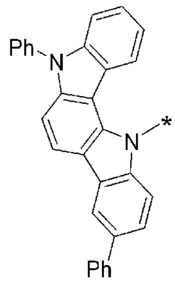
D180

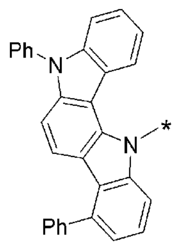
D181

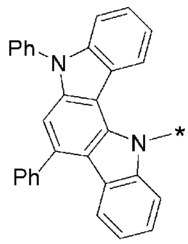
D182

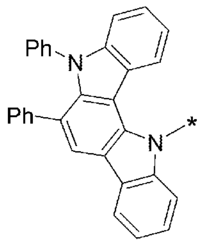
D183

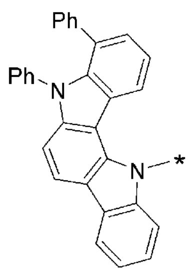
D184

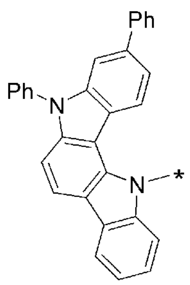
D185

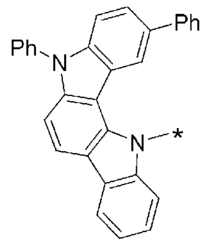
D186

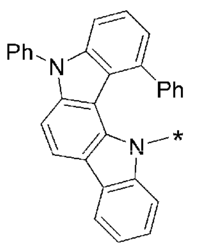
D187

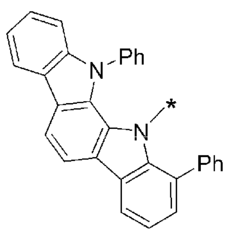
D188

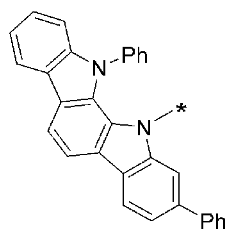
D189

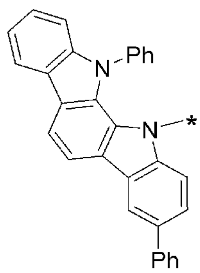
D190

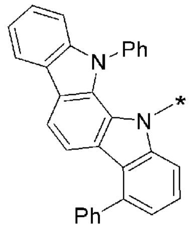
D191

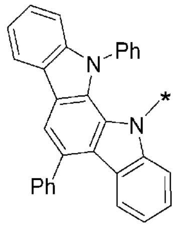
D192

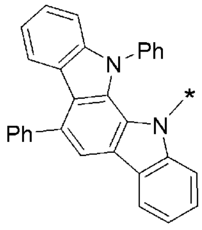
D193

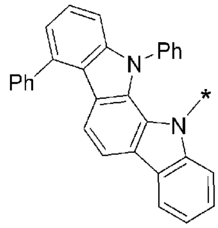
D194

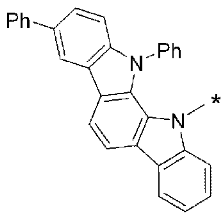
D195

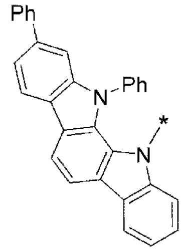
D196

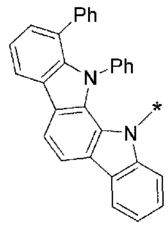
D197

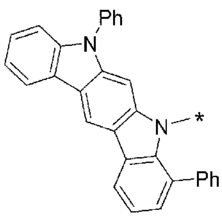
D198

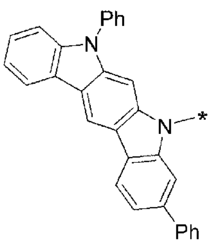
D199

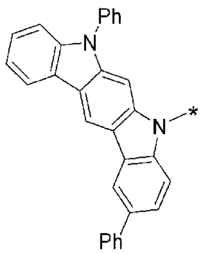
D200

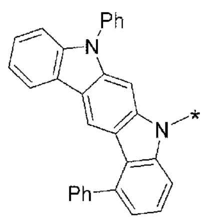
D201

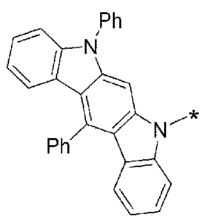
D202

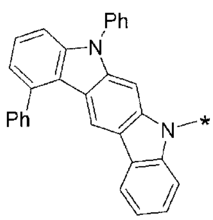
D203

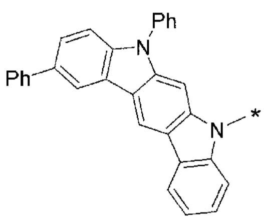
D204

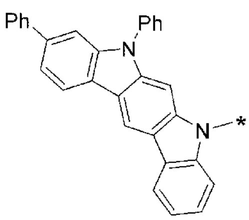
D205

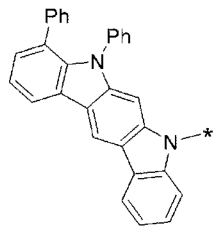
D206

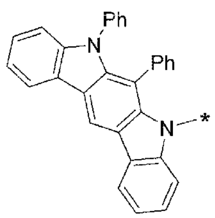
D207

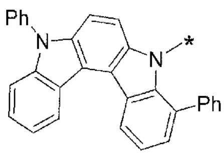
D208

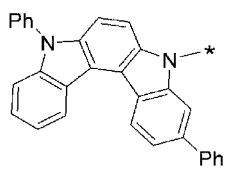
D209

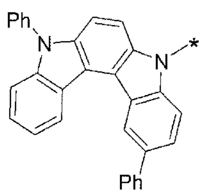
D210

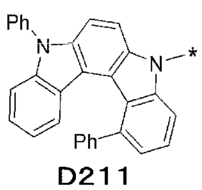
D211

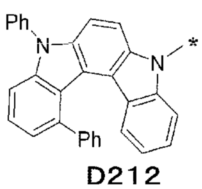
D212

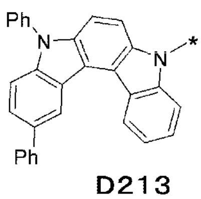
D213

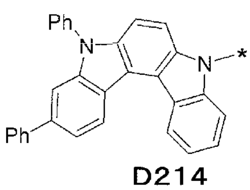
D214

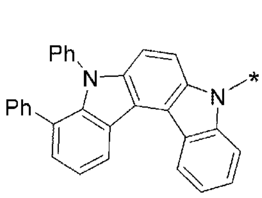
D215

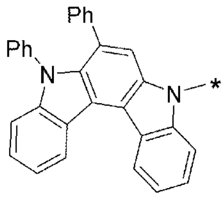
D216

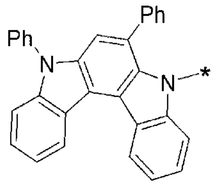
D217

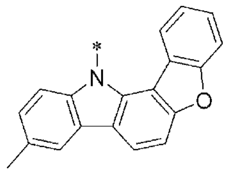
D218

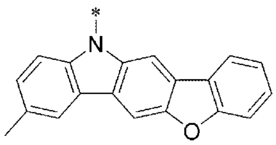
D219

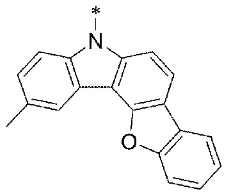
D220

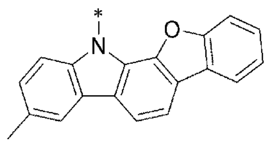
D221

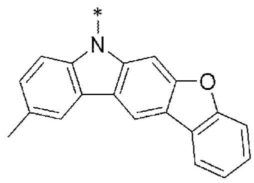
D222

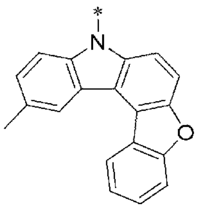
D223

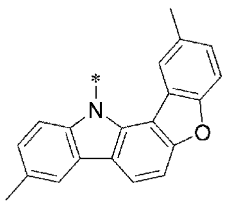
D224

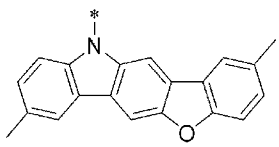
D225

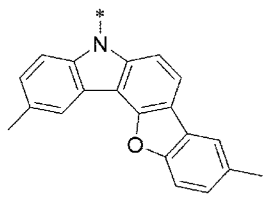
D226

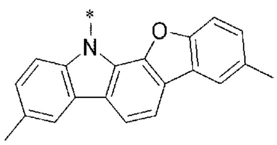
D227

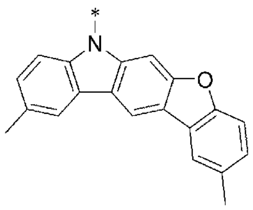
D228

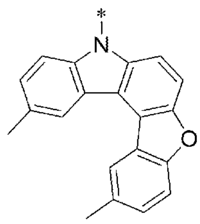
D229

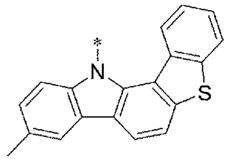
D230

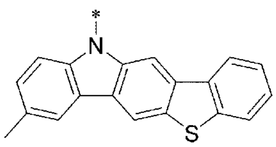
D231

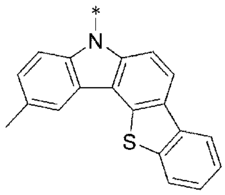
D232

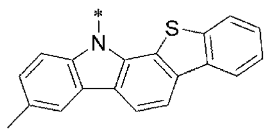
D233

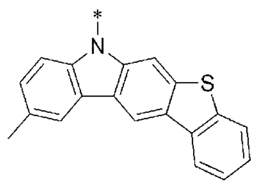
D234

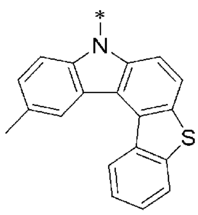
D235

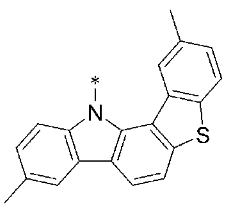
D236

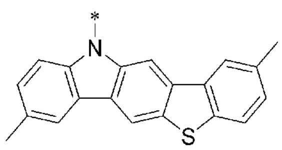
D237

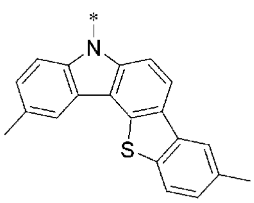
D238

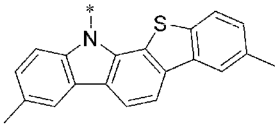 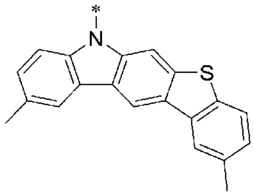 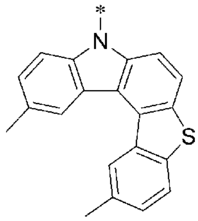 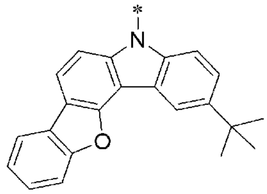

D239 D240 D241 D242

Groups obtained by substituting all hydrogen atoms present in the above D1 to D242 with deuterium atoms are disclosed as D243 to D484. Groups obtained by substituting all hydrogen atoms present in the phenyl group or the alkyl group which is the substituent in the above D13 to D242 with deuterium atoms are disclosed as D484 to D713.

In one aspect of the present invention, the compounds represented by the general formula (1) have a group selected from the group consisting of D1 to D713. In one aspect of the present invention, the compounds represented by the general formula (1) have a group selected from the group consisting of D1 to D242. In one aspect of the present invention, the compounds represented by the general formula (1) have a group selected from the group consisting of D243 to D484. In one aspect of the present invention, the compounds represented by the general formula (1) have a group selected from the group consisting of D484 to D713.

As $R^2$ to $R^4$ in the general formula (1), a substituted or unsubstituted carbazol-9-yl group not fused with a ring can be employed. Specific examples of such a substituted or unsubstituted carbazol-9-yl group not fused with a ring are shown below. However, the substituted or unsubstituted carbazol-9-yl group not fused with a ring which can be employed in the present invention shall not be construed as being limited by the following specific examples.

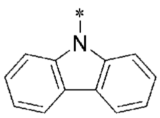 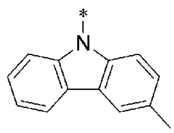 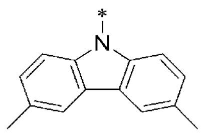 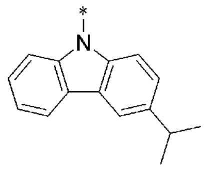

D714 D715 D716 D717

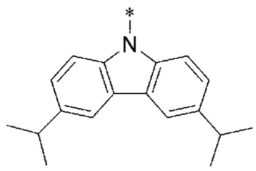 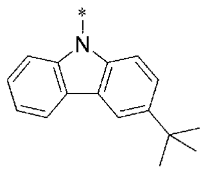 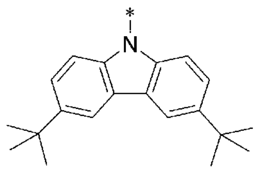 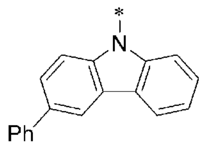

D718 D719 D720 D721

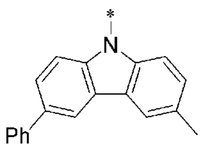 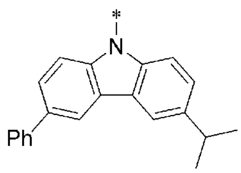 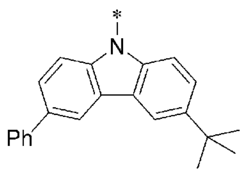 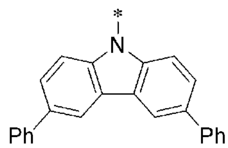

D722 D723 D724 D725

All hydrogen atoms present in the above D714 to D725 are substituted with deuterium atoms, and the resultant compounds are disclosed as D726 to D737, respectively. Groups obtained by substituting all hydrogen atoms present in the phenyl group and the alkyl group which is the substituent for the above D715 to D725 with deuterium atoms are disclosed as D738 to D748.

In the general formula (1), $R^2$ to $R^4$ are not all the same. Namely, at least one of $R^2$ to $R^4$ differs from the others. In one aspect of the present invention, $R^2$ to $R^4$ all differ from each other. In one aspect of the present invention, $R^2$ and $R^3$ are the same, and $R^4$ differs. In one aspect of the present invention, $R^2$ and $R^4$ are the same, and $R^3$ differs.

In one aspect of the present invention, one or two of $R^2$ to $R^4$ are optionally ring-fused unsubstituted carbazol-9-yl groups, and the remaining $R^2$ to $R^4$ are optionally ring-fused substituted carbazol-9-yl groups. In one aspect of the present invention, $R^2$ and $R^4$ are the same, and are optionally ring-fused unsubstituted carbazol-9-yl groups, and $R^3$ is

an optionally ring-fused substituted carbazol-9-yl group. In one aspect of the present invention, $R^2$ and $R^4$ are the same and are optionally ring-fused substituted carbazol-9-yl groups, and $R^3$ is an optionally ring-fused, unsubstituted or substituted carbazol-9-yl group. The substituent for the substituted carbazol-9-yl group referred to in this paragraph is preferably an aryl group optionally substituted with one atom or group selected from the group consisting of an alkyl group and an aryl group or with a group formed by combining two or more thereof.

In one aspect of the present invention, one or two of $R^2$ to $R^4$ are substituted or unsubstituted benzofuro-fused carbazol-9-yl groups, or substituted or unsubstituted benzothieno-fused carbazol-9-yl groups, and the remaining $R^2$ to $R^4$ are substituted or unsubstituted carbazol-9-yl groups not fused with a ring. In one aspect of the present invention, $R^2$ and $R^4$ are the same, and are substituted or unsubstituted benzofuro-fused carbazol-9-yl groups, or substituted or unsubstituted benzothieno-fused carbazol-9-yl groups, and $R^3$ is a substituted or unsubstituted carbazol-9-yl groups not fused with a ring. In one aspect of the present invention, $R^2$ and $R^4$ are the same and are substituted or unsubstituted carbazol-9-yl groups not fused with a ring, and $R^3$ is a substituted or unsubstituted benzofuro-fused carbazol-9-yl group, or a substituted or unsubstituted benzothieno-fused carbazol-9-yl group. In one aspect of the present invention, $R^2$ and $R^4$ are the same and are substituted benzofuro-fused carbazol-9-yl groups, or substituted benzothieno-fused carbazol-9-yl groups, and $R^3$ is a substituted or unsubstituted carbazol-9-yl group not fused with a ring. In one aspect of the present invention, $R^2$ and $R^4$ are the same and are substituted or unsubstituted carbazol-9-yl groups not fused with a ring, and $R^3$ is a substituted benzofuro-fused carbazol-9-yl group, or a substituted benzothieno-fused carbazol-9-yl group. The substituent for the benzofuro-fused carbazol-9-yl group and the benzothieno-fused carbazol-9-yl group as referred to herein is preferably an aryl group optionally substituted with one atom or group selected from the group consisting of an alkyl group and an aryl group or with a group formed by combining two or more thereof.

In the general formula (1) of the present invention, none of $R^2$ and $R^3$, or $R^3$ and $R^4$ bonds to each other to form a cyclic structure.

In the general formula (1), $X^1$ to $X^3$ each independently represent N or C(R). However, at least one of $X^1$ to $X^3$ is N. R represents a hydrogen atom, a deuterium atom or a substituent. As referred to herein, the substituent can be selected from Substituent Group A, can be selected from Substituent Group B, can be selected from Substituent Group C, can be selected from Substituent Group D, or can be selected from Substituent Group E. In one preferred aspect of the present invention, $X^1$ to $X^3$ are N. In one aspect of the present invention, $X^1$ and $X^3$ are N, and $X^2$ is C(R). In one aspect of the present invention, $X^1$ and $X^2$ are N, and $X^3$ is C(R). In one aspect of the present invention, $X^1$ is N, and $X^2$ and $X^3$ are C(R). In one aspect of the present invention, $X^2$ is N, and $X^1$ and $X^3$ are C(R). In one aspect of the present invention, R is a hydrogen atom or a deuterium atom. In one aspect of the present invention, R is an alkyl group optionally substituted with a deuterium atom. In one aspect of the present invention, R is an aryl group optionally substituted with a deuterium atom, an alkyl group or an aryl group.

In the general formula (1), $Ar^1$ and $Ar^2$ each independently represent a substituted or unsubstituted aryl group, or a substituted or unsubstituted heteroaryl group. Regarding the description and the preferred range of the substituted or unsubstituted aryl group, reference can be made to the description and the preferred range of the aryl group and the heteroaryl group of the donor group described hereinabove. In one aspect of the present invention, $Ar^1$ and $Ar^2$ are substituted or unsubstituted aryl groups, preferably substituted or unsubstituted phenyl groups, more preferably unsubstituted phenyl groups. In one aspect of the present invention, $Ar^1$ and $Ar^2$ are substituted or unsubstituted heteroaryl groups. In a preferred aspect of the present invention, $Ar^1$ and $Ar^2$ are the same.

Specific examples of the substituted or unsubstituted aryl group employable by $Ar^1$ and $Ar^2$ are shown below. However, the substituted or unsubstituted aryl group which can be employed in the present invention shall not be construed as being limited by the following specific examples. In the following specific examples, expression of a methyl group is omitted. Accordingly, Ar4 is substituted by a methyl group, and Ar5 is substituted by an isopropyl group. * indicates a bonding site.

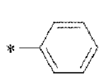
Ar1
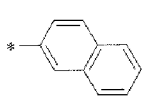
Ar2
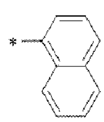
Ar3
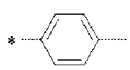
Ar4
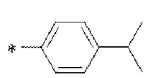
Ar5

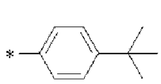
Ar6
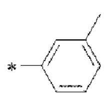
Ar7
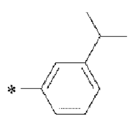
Ar8
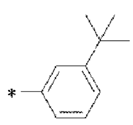
Ar9
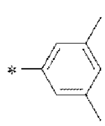
Ar10

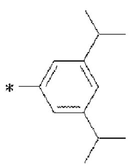
Ar11
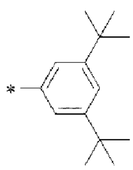
Ar12
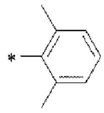
Ar13
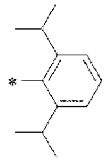
Ar14
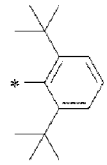
Ar15

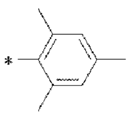
Ar16
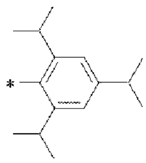
Ar17
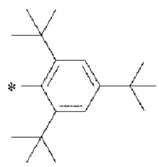
Ar18
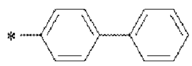
Ar19
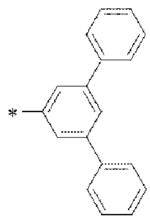
Ar20

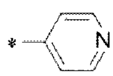
Ar21
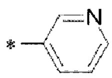
Ar22
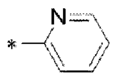
Ar23
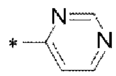
Ar24
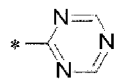
Ar25

Groups obtained by substituting all hydrogen atoms present in the above Ar1 to Ar25 with deuterium atoms are disclosed as Ar26 to Ar50, respectively. Groups obtained by substituting all hydrogen atoms present in the phenyl group or the alkyl group which is the substituent in the above Ar4 to Ar20 with deuterium atoms are disclosed as Ar51 to Ar67, respectively.

In the general formula (1), $L^1$ represents a single bond or a divalent linking group. The divalent linking group includes a substituted or unsubstituted arylene group, and a substituted or unsubstituted heteroarylene group. In one preferred aspect of the present invention, $L^1$ is a single bond. In one aspect of the present invention, $L^1$ is a substituted or unsubstituted arylene group. In one aspect of the present invention, $L^1$ is a substituted or unsubstituted heteroarylene group. Regarding the aryl moiety constituting the arylene group, reference can be made to the description and the preferred range of the aryl group in the description section of the donor group mentioned above. The heteroarylene group includes a linking group formed by substituting at least one ring skeleton carbon atom constituting the arylene group with a nitrogen atom.

Specific examples of $L^1$ are shown below. However, $L^1$ which can be employed in the present invention shall not be construed as being limited by the following specific examples. In the following specific examples, expression of a methyl group is omitted. Consequently, for example, L3 to L5 are substituted with a methyl group. * indicates a bonding site. L1 is a single bond.

none (direct bond)
L1
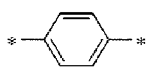
L2
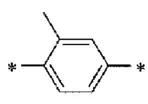
L3
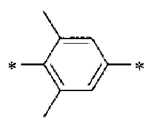
L4
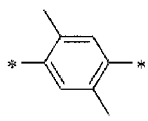
L5

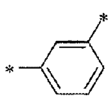
L6
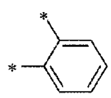
L7
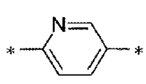
L8
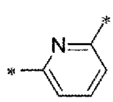
L9
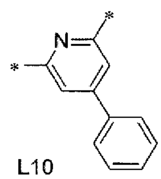
L10

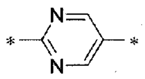
L11

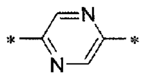
L12

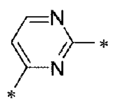
L13

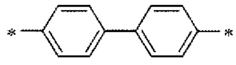
L14

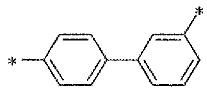
L15

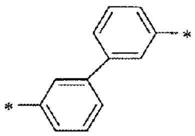
L16

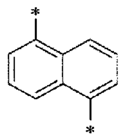
L17

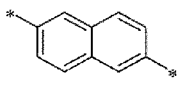
L18

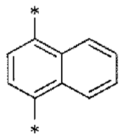
L19

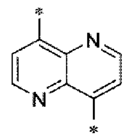
L20

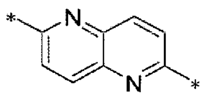
L21

In one preferred aspect of the present invention, $X^1$ to $X^3$ are N, $Ar^1$ and $Ar^2$ are substituted or unsubstituted aryl groups (preferably substituted or unsubstituted phenyl groups, more preferably phenyl groups), and $L^1$ is a single bond.

In one aspect of the present invention, $X^1$ to $X^3$ are N, $Ar^1$ and $Ar^2$ are substituted or unsubstituted aryl groups (preferably substituted or unsubstituted phenyl groups, more preferably phenyl groups), and $L^1$ is L2.

In one aspect of the present invention, $X^1$ to $X^3$ are N, $Ar^1$ and $Ar^2$ are substituted or unsubstituted aryl groups (preferably substituted or unsubstituted phenyl groups, more preferably phenyl groups), and $L^1$ is L6.

The compound represented by the general formula (1) preferably does not contain a metal atom, and can be a compound composed only of atoms selected from the group consisting of a carbon atom, a hydrogen atom, a deuterium atom, a nitrogen atom, an oxygen atom, and a sulfur atom. In one preferred aspect of the present invention, the compound represented by the general formula (1) is composed only of atoms selected from the group consisting of a carbon atom, a hydrogen atom, a deuterium atom, a nitrogen atom, and an oxygen atom. In addition, the compound represented by the general formula (1) can be a compound composed only of atoms selected from the group consisting of a carbon atom, a hydrogen atom, a deuterium atom, a nitrogen atom, and a sulfur atom. The compound represented by the general formula (1) can be a compound composed only of atoms selected from the group consisting of a carbon atom, a hydrogen atom, a deuterium atom, and a nitrogen atom. The compound represented by the general formula (1) can be a compound composed only of atoms selected from the group consisting of a carbon atom, a hydrogen atom, and a nitrogen atom. Further, the compound represented by the general formula (1) can be a compound which does not contain a hydrogen atom but contains a deuterium atom.

In the description herein, the term "Substituent Group A" means one atom or group or a combination of two or more thereof selected from the group consisting of a deuterium atom, a hydroxyl group, a halogen atom (for example, a fluorine atom, a chlorine atom, a bromine atom, and an iodine atom), an alkyl group (for example, having 1 to 40 carbon atoms), an alkoxy group (for example, having 1 to 40 carbon atoms), an alkylthio group (for example, having 1 to 40 carbon atoms), an aryl group (for example, having 6 to 30 carbon atoms), an aryloxy group (for example, having 6 to 30 carbon atoms), an arylthio group (for example, having 6 to 30 carbon atoms), a heteroaryl group (for example, having 5 to 30 ring skeleton-constituting atoms), a heteroaryloxy group (for example, having 5 to 30 ring skeleton-constituting atoms), a heteroarylthio group (for example, having 5 to 30 ring skeleton-constituting atoms), an acyl group (for example, having 1 to 40 carbon atoms), an alkenyl group (for example, having 1 to 40 carbon atoms), an alkynyl group (for example, having 1 to 40 carbon atoms), an alkoxycarbonyl group (for example, having 1 to 40 carbon atoms), an aryloxycarbonyl group (for example, having 1 to 40 carbon atoms), a heteroaryloxycarbonyl group (for example, having 1 to 40 carbon atoms), a silyl group (for example, a trialkylsilyl group having 1 to 40 carbon atoms), and a nitro group.

In the description herein, the term "Substituent Group B" means one atom or group or a combination of two or more thereof selected from the group consisting of a deuterium atom, an alkyl group (for example, having 1 to 40 carbon atoms), an alkoxy group (for example, having 1 to 40 carbon atoms), an aryl group (for example, having 6 to 30 carbon atoms), an aryloxy group (for example, having 6 to 30 carbon atoms), a heteroaryl group (for example, having 5 to 30 ring skeleton-constituting atoms), a heteroaryloxy group (for example, having 5 to 30 ring skeleton-constituting atoms),

and a diarylaminoamino group (for example, having 0 to 20 carbon atoms).

In the description herein, the term "Substituent Group C" means one atom or group or a combination of two or more thereof selected from the group consisting of a deuterium atom, an alkyl group (for example, having 1 to 20 carbon atoms), an aryl group (for example, having 6 to 22 carbon atoms), a heteroaryl group (for example, having 5 to 20 ring skeleton-constituting atoms), and a diarylamino group (for example, having 12 to 20 carbon atoms).

In the description herein, the term "Substituent Group D" means one atom or group or a combination of two or more thereof selected from the group consisting of a deuterium atom, an alkyl group (for example, having 1 to 20 carbon atoms), an aryl group (for example, having 6 to 22 carbon atoms), and a heteroaryl group (for example, having 5 to 20 ring skeleton-constituting atoms).

In the description herein, the term "Substituent Group E" means one atom or group or a combination of two or more groups selected from the group consisting of a deuterium atom, an alkyl group (for example, having 1 to 20 carbon atoms), and an aryl group (for example, having 6 to 22 carbon atoms).

In the description herein, the substituent meant by an expression of "substituted or unsubstituted" or "optionally substituted" can be selected, for example, from Substituent Group A, can be selected from Substituent Group B, can be selected from Substituent Group C, can be selected from Substituent Group D, or can be selected from Substituent Group E.

In Table 1 and Table 2 below, specific examples of the compound represented by the general formula (1) are shown. However, the compound represented by the general formula (1) that can be used in the present invention should not be construed as being limited by these specific examples.

In Table 1 and Table 2, structures of Compounds 1 to 99820 are individually shown by specifying $R^2$ to $R^4$ of the following general formula for each compound. Specifically, structures where $Ar^1$ and $Ar^2$ are phenyl groups (Ar1), $X^1$ to $X^3$ are nitrogen atoms (N), $L^1$ is a single bond, $R^1$ is a hydrogen atom, and $R^2$ to $R^4$ are groups specified in Table 1 and Table 2 are individually shown as structures of Compounds 1 to 99820.

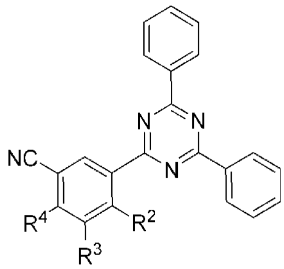

In Table 2, structures of Compounds 1 to 99820 are shown by collectively displaying $R^2$ to $R^4$ of a plurality of compounds in each row. For example, in the row of Compounds 1 to 713 in Table 2, compounds in which $R^3$ is fixed to D714, and $R^2$ and $R^4$ are both D1 to D713 are referred to as Compounds 1 to 713 in that order. $R^2$ and $R^4$ are the same. That is, the row of Compounds 1 to 713 in Table 2 collectively represents Compounds 1 to 713 specified in Table 1. Similarly, in the row of Compounds 714 to 1426 in Table 2, those in which $R^3$ is fixed to D715, and $R^2$ and $R^4$ are both D1 to D713 are referred to as Compounds 714 to 1426 in that order. In the same manner, Compounds 1427 to 24955 in Table 2 are also specified. For example, in the row of Compounds 24956 to 25668 in Table 2, compounds in which $R^2$ and $R^4$ are both fixed to D714, and $R^3$ is D1 to D713 are referred to as Compounds 24956 to 25668 in that order. In the same manner, Compounds 25669 to 49910 in Table 2 are also specified. In each row of Compounds 49911 to 99820 in Table 2, those where $R^2$ and $R^3$ are the same and $R^4$ differs are specified in the same manner.

Table 1

| No. | $R^2$ | $R^3$ | $R^4$ |
|---|---|---|---|
| 1 | D1 | D714 | D1 |
| 2 | D2 | D714 | D2 |
| 3 | D3 | D714 | D3 |
| 4 | D4 | D714 | D4 |
| 5 | D5 | D714 | D5 |
| 6 | D6 | D714 | D6 |
| 7 | D7 | D714 | D7 |

(continued)

| No. | $R^2$ | $R^3$ | $R^4$ |
|---|---|---|---|
| 8 | D8 | D714 | D8 |
| 9 | D9 | D714 | D9 |
| 10 | D10 | D714 | D10 |
| 11 | D11 | D714 | D11 |
| 12 | D12 | D714 | D12 |
| 13 | D13 | D714 | D13 |
| 14 | D14 | D714 | D14 |
| 15 | D15 | D714 | D15 |
| 16 | D16 | D714 | D16 |
| 17 | D17 | D714 | D17 |
| 18 | D18 | D714 | D18 |
| 19 | D19 | D714 | D19 |
| 20 | D20 | D714 | D20 |
| 21 | D21 | D714 | D21 |
| 22 | D22 | D714 | D22 |
| 23 | D23 | D714 | D23 |
| 24 | D24 | D714 | D24 |
| 25 | D25 | D714 | D25 |
| 26 | D26 | D714 | D26 |
| 27 | D27 | D714 | D27 |
| 28 | D28 | D714 | D28 |
| 29 | D29 | D714 | D29 |
| 30 | D30 | D714 | D30 |
| 31 | D31 | D714 | D31 |
| 32 | D32 | D714 | D32 |
| 33 | D33 | D714 | D33 |
| 34 | D34 | D714 | D34 |
| 35 | D35 | D714 | D35 |
| 36 | D36 | D714 | D36 |
| 37 | D37 | D714 | D37 |
| 38 | D38 | D714 | D38 |
| 39 | D39 | D714 | D39 |
| 40 | D40 | D714 | D40 |
| 41 | D41 | D714 | D41 |
| 42 | D42 | D714 | D42 |
| 43 | D43 | D714 | D43 |
| 44 | D44 | D714 | D44 |
| 45 | D45 | D714 | D45 |

(continued)

| No. | $R^2$ | $R^3$ | $R^4$ |
|---|---|---|---|
| 46 | D46 | D714 | D46 |
| 47 | D47 | D714 | D47 |
| 48 | D48 | D714 | D48 |
| 49 | D49 | D714 | D49 |
| 50 | D50 | D714 | D50 |
| 51 | D51 | D714 | D51 |
| 52 | D52 | D714 | D52 |
| 53 | D53 | D714 | D53 |
| 54 | D54 | D714 | D54 |
| 55 | D55 | D714 | D55 |
| 56 | D56 | D714 | D56 |
| 57 | D57 | D714 | D57 |
| 58 | D58 | D714 | D58 |
| 59 | D59 | D714 | D59 |
| 60 | D60 | D714 | D60 |
| 61 | D61 | D714 | D61 |
| 62 | D62 | D714 | D62 |
| 63 | D63 | D714 | D63 |
| 64 | D64 | D714 | D64 |
| 65 | D65 | D714 | D65 |
| 66 | D66 | D714 | D66 |
| 67 | D67 | D714 | D67 |
| 68 | D68 | D714 | D68 |
| 69 | D69 | D714 | D69 |
| 70 | D70 | D714 | D70 |
| 71 | D71 | D714 | D71 |
| 72 | D72 | D714 | D72 |
| 73 | D73 | D714 | D73 |
| 74 | D74 | D714 | D74 |
| 75 | D75 | D714 | D75 |
| 76 | D76 | D714 | D76 |
| 77 | D77 | D714 | D77 |
| 78 | D78 | D714 | D78 |
| 79 | D79 | D714 | D79 |
| 80 | D80 | D714 | D80 |
| 81 | D81 | D714 | D81 |
| 82 | D82 | D714 | D82 |
| 83 | D83 | D714 | D83 |

(continued)

| No. | $R^2$ | $R^3$ | $R^4$ |
|---|---|---|---|
| 84 | D84 | D714 | D84 |
| 85 | D85 | D714 | D85 |
| 86 | D86 | D714 | D86 |
| 87 | D87 | D714 | D87 |
| 88 | D88 | D714 | D88 |
| 89 | D89 | D714 | D89 |
| 90 | D90 | D714 | D90 |
| 91 | D91 | D714 | D91 |
| 92 | D92 | D714 | D92 |
| 93 | D93 | D714 | D93 |
| 94 | D94 | D714 | D94 |
| 95 | D95 | D714 | D95 |
| 96 | D96 | D714 | D96 |
| 97 | D97 | D714 | D97 |
| 98 | D98 | D714 | D98 |
| 99 | D99 | D714 | D99 |
| 100 | D100 | D714 | D100 |
| 101 | D101 | D714 | D101 |
| 102 | D102 | D714 | D102 |
| 103 | D103 | D714 | D103 |
| 104 | D104 | D714 | D104 |
| 105 | D105 | D714 | D105 |
| 106 | D106 | D714 | D106 |
| 107 | D107 | D714 | D107 |
| 108 | D108 | D714 | D108 |
| 109 | D109 | D714 | D109 |
| 110 | D110 | D714 | D110 |
| 111 | D111 | D714 | D111 |
| 112 | D112 | D714 | D112 |
| 113 | D113 | D714 | D113 |
| 114 | D114 | D714 | D114 |
| 115 | D115 | D714 | D115 |
| 116 | D116 | D714 | D116 |
| 117 | D117 | D714 | D117 |
| 118 | D118 | D714 | D118 |
| 119 | D119 | D714 | D119 |
| 120 | D120 | D714 | D120 |
| 121 | D121 | D714 | D121 |

(continued)

| No. | $R^2$ | $R^3$ | $R^4$ |
|---|---|---|---|
| 122 | D122 | D714 | D122 |
| 123 | D123 | D714 | D123 |
| 124 | D124 | D714 | D124 |
| 125 | D125 | D714 | D125 |
| 126 | D126 | D714 | D126 |
| 127 | D127 | D714 | D127 |
| 128 | D128 | D714 | D128 |
| 129 | D129 | D714 | D129 |
| 130 | D130 | D714 | D130 |
| 131 | D131 | D714 | D131 |
| 132 | D132 | D714 | D132 |
| 133 | D133 | D714 | D133 |
| 134 | D134 | D714 | D134 |
| 135 | D135 | D714 | D135 |
| 136 | D136 | D714 | D136 |
| 137 | D137 | D714 | D137 |
| 138 | D138 | D714 | D138 |
| 139 | D139 | D714 | D139 |
| 140 | D140 | D714 | D140 |
| 141 | D141 | D714 | D141 |
| 142 | D142 | D714 | D142 |
| 143 | D143 | D714 | D143 |
| 144 | D144 | D714 | D144 |
| 145 | D145 | D714 | D145 |
| 146 | D146 | D714 | D146 |
| 147 | D147 | D714 | D147 |
| 148 | D148 | D714 | D148 |
| 149 | D149 | D714 | D149 |
| 150 | D150 | D714 | D150 |
| 151 | D151 | D714 | D151 |
| 152 | D152 | D714 | D152 |
| 153 | D153 | D714 | D153 |
| 154 | D154 | D714 | D154 |
| 155 | D155 | D714 | D155 |
| 156 | D156 | D714 | D156 |
| 157 | D157 | D714 | D157 |
| 158 | D158 | D714 | D158 |
| 159 | D159 | D714 | D159 |

(continued)

| No. | $R^2$ | $R^3$ | $R^4$ |
|---|---|---|---|
| 160 | D160 | D714 | D160 |
| 161 | D161 | D714 | D161 |
| 162 | D162 | D714 | D162 |
| 163 | D163 | D714 | D163 |
| 164 | D164 | D714 | D164 |
| 165 | D165 | D714 | D165 |
| 166 | D166 | D714 | D166 |
| 161 | D167 | D714 | D167 |
| 168 | D168 | D714 | D168 |
| 169 | D169 | D714 | D169 |
| 170 | D170 | D714 | D170 |
| 171 | D171 | D714 | D171 |
| 172 | D172 | D714 | D172 |
| 173 | D173 | D714 | D173 |
| 174 | D174 | D714 | D174 |
| 175 | D175 | D714 | D175 |
| 176 | D176 | D714 | D176 |
| 177 | D177 | D714 | D177 |
| 178 | D178 | D714 | D178 |
| 179 | D179 | D714 | D179 |
| 180 | D180 | D714 | D180 |
| 181 | D181 | D714 | D181 |
| 182 | D182 | D714 | D182 |
| 183 | D183 | D714 | D183 |
| 184 | D184 | D714 | D184 |
| 185 | D185 | D714 | D185 |
| 186 | D186 | D714 | D186 |
| 187 | D187 | D714 | D187 |
| 188 | D188 | D714 | D188 |
| 189 | D189 | D714 | D189 |
| 190 | D190 | D714 | D190 |
| 191 | D191 | D714 | D191 |
| 192 | D192 | D714 | D192 |
| 193 | D193 | D714 | D193 |
| 194 | D194 | D714 | D194 |
| 195 | D 195 | D714 | D 195 |
| 196 | D196 | D714 | D196 |
| 197 | D197 | D714 | D197 |

(continued)

| No. | $R^2$ | $R^3$ | $R^4$ |
|---|---|---|---|
| 198 | D198 | D714 | D198 |
| 199 | D199 | D714 | D199 |
| 200 | D200 | D714 | D200 |
| 201 | D201 | D714 | D201 |
| 202 | D202 | D714 | D202 |
| 203 | D203 | D714 | D203 |
| 204 | D204 | D714 | D204 |
| 205 | D205 | D714 | D205 |
| 206 | D206 | D714 | D206 |
| 207 | D207 | D714 | D207 |
| 208 | D208 | D714 | D208 |
| 209 | D209 | D714 | D209 |
| 210 | D210 | D714 | D210 |
| 211 | D211 | D714 | D211 |
| 212 | D212 | D714 | D212 |
| 213 | D213 | D714 | D213 |
| 214 | D214 | D714 | D214 |
| 215 | D215 | D714 | D215 |
| 216 | D216 | D714 | D216 |
| 217 | D217 | D714 | D217 |
| 218 | D218 | D714 | D218 |
| 219 | D219 | D714 | D219 |
| 220 | D220 | D714 | D220 |
| 221 | D221 | D714 | D221 |
| 222 | D222 | D714 | D222 |
| 223 | D223 | D714 | D223 |
| 224 | D224 | D714 | D224 |
| 225 | D225 | D714 | D225 |
| 226 | D226 | D714 | D226 |
| 227 | D227 | D714 | D227 |
| 228 | D228 | D714 | D228 |
| 229 | D229 | D714 | D229 |
| 230 | D230 | D714 | D230 |
| 231 | D231 | D714 | D231 |
| 232 | D232 | D714 | D232 |
| 233 | D233 | D714 | D233 |
| 234 | D234 | D714 | D234 |
| 235 | D235 | D714 | D235 |

(continued)

| No. | $R^2$ | $R^3$ | $R^4$ |
|---|---|---|---|
| 236 | D236 | D714 | D236 |
| 237 | D237 | D714 | D237 |
| 238 | D238 | D714 | D238 |
| 239 | D239 | D714 | D239 |
| 240 | D240 | D714 | D240 |
| 241 | D241 | D714 | D241 |
| 242 | D242 | D714 | D242 |
| 243 | D243 | D714 | D243 |
| 244 | D244 | D714 | D244 |
| 245 | D245 | D714 | D245 |
| 246 | D246 | D714 | D246 |
| 247 | D247 | D714 | D247 |
| 248 | D248 | D714 | D248 |
| 249 | D249 | D714 | D249 |
| 250 | D250 | D714 | D250 |
| 251 | D251 | D714 | D251 |
| 252 | D252 | D714 | D252 |
| 253 | D253 | D714 | D253 |
| 254 | D254 | D714 | D254 |
| 255 | D255 | D714 | D255 |
| 256 | D256 | D714 | D256 |
| 257 | D257 | D714 | D257 |
| 258 | D258 | D714 | D258 |
| 259 | D259 | D714 | D259 |
| 260 | D260 | D714 | D260 |
| 261 | D261 | D714 | D261 |
| 262 | D262 | D714 | D262 |
| 263 | D263 | D714 | D263 |
| 264 | D264 | D714 | D264 |
| 265 | D265 | D714 | D265 |
| 266 | D266 | D714 | D266 |
| 267 | D267 | D714 | D267 |
| 268 | D268 | D714 | D268 |
| 269 | D269 | D714 | D269 |
| 270 | D270 | D714 | D270 |
| 271 | D271 | D714 | D271 |
| 272 | D272 | D714 | D272 |
| 273 | D273 | D714 | D273 |

(continued)

| No. | $R^2$ | $R^3$ | $R^4$ |
|---|---|---|---|
| 274 | D274 | D714 | D274 |
| 275 | D275 | D714 | D275 |
| 276 | D276 | D714 | D276 |
| 277 | D277 | D714 | D277 |
| 278 | D278 | D714 | D278 |
| 279 | D279 | D714 | D279 |
| 280 | D280 | D714 | D280 |
| 281 | D281 | D714 | D281 |
| 282 | D282 | D714 | D282 |
| 283 | D283 | D714 | D283 |
| 284 | D284 | D714 | D284 |
| 285 | D285 | D714 | D285 |
| 286 | D286 | D714 | D286 |
| 287 | D287 | D714 | D287 |
| 288 | D288 | D714 | D288 |
| 289 | D289 | D714 | D289 |
| 290 | D290 | D714 | D290 |
| 291 | D291 | D714 | D291 |
| 292 | D292 | D714 | D292 |
| 293 | D293 | D714 | D293 |
| 294 | D294 | D714 | D294 |
| 295 | D295 | D714 | D295 |
| 296 | D296 | D714 | D296 |
| 297 | D297 | D714 | D297 |
| 298 | D298 | D714 | D298 |
| 299 | D299 | D714 | D299 |
| 300 | D300 | D714 | D300 |
| 301 | D301 | D714 | D301 |
| 302 | D302 | D714 | D302 |
| 303 | D303 | D714 | D303 |
| 304 | D304 | D714 | D304 |
| 305 | D305 | D714 | D305 |
| 306 | D306 | D714 | D306 |
| 307 | D307 | D714 | D307 |
| 308 | D308 | D714 | D308 |
| 309 | D309 | D714 | D309 |
| 310 | D310 | D714 | D310 |
| 311 | D311 | D714 | D311 |

(continued)

| No. | $R^2$ | $R^3$ | $R^4$ |
|---|---|---|---|
| 312 | D312 | D714 | D312 |
| 313 | D313 | D714 | D313 |
| 314 | D314 | D714 | D314 |
| 315 | D315 | D714 | D315 |
| 316 | D316 | D714 | D316 |
| 317 | D317 | D714 | D317 |
| 318 | D318 | D714 | D318 |
| 319 | D319 | D714 | D319 |
| 320 | D320 | D714 | D320 |
| 321 | D321 | D714 | D321 |
| 322 | D322 | D714 | D322 |
| 323 | D323 | D714 | D323 |
| 324 | D324 | D714 | D324 |
| 325 | D325 | D714 | D325 |
| 326 | D326 | D714 | D326 |
| 327 | D327 | D714 | D327 |
| 328 | D328 | D714 | D328 |
| 329 | D329 | D714 | D329 |
| 330 | D330 | D714 | D330 |
| 331 | D331 | D714 | D331 |
| 332 | D332 | D714 | D332 |
| 333 | D333 | D714 | D333 |
| 334 | D334 | D714 | D334 |
| 335 | D335 | D714 | D335 |
| 336 | D336 | D714 | D336 |
| 337 | D337 | D714 | D337 |
| 338 | D338 | D714 | D338 |
| 339 | D339 | D714 | D339 |
| 340 | D340 | D714 | D340 |
| 341 | D341 | D714 | D341 |
| 342 | D342 | D714 | D342 |
| 343 | D343 | D714 | D343 |
| 344 | D344 | D714 | D344 |
| 345 | D345 | D714 | D345 |
| 346 | D346 | D714 | D346 |
| 347 | D347 | D714 | D347 |
| 348 | D348 | D714 | D348 |
| 349 | D349 | D714 | D349 |

(continued)

| No. | $R^2$ | $R^3$ | $R^4$ |
|---|---|---|---|
| 350 | D350 | D714 | D350 |
| 351 | D351 | D714 | D351 |
| 352 | D352 | D714 | D352 |
| 353 | D353 | D714 | D353 |
| 354 | D354 | D714 | D354 |
| 355 | D355 | D714 | D355 |
| 356 | D356 | D714 | D356 |
| 357 | D357 | D714 | D357 |
| 358 | D358 | D714 | D358 |
| 359 | D359 | D714 | D359 |
| 360 | D360 | D714 | D360 |
| 361 | D361 | D714 | D361 |
| 362 | D362 | D714 | D362 |
| 363 | D363 | D714 | D363 |
| 364 | D364 | D714 | D364 |
| 365 | D365 | D714 | D365 |
| 366 | D366 | D714 | D366 |
| 367 | D367 | D714 | D367 |
| 368 | D368 | D714 | D368 |
| 369 | D369 | D714 | D369 |
| 370 | D370 | D714 | D370 |
| 371 | D371 | D714 | D371 |
| 372 | D372 | D714 | D372 |
| 373 | D373 | D714 | D373 |
| 374 | D374 | D714 | D374 |
| 375 | D375 | D714 | D375 |
| 376 | D376 | D714 | D376 |
| 377 | D377 | D714 | D377 |
| 378 | D378 | D714 | D378 |
| 379 | D379 | D714 | D379 |
| 380 | D380 | D714 | D380 |
| 381 | D381 | D714 | D381 |
| 382 | D382 | D714 | D382 |
| 383 | D383 | D714 | D383 |
| 384 | D384 | D714 | D384 |
| 385 | D385 | D714 | D385 |
| 386 | D386 | D714 | D386 |
| 387 | D387 | D714 | D387 |

(continued)

| No. | $R^2$ | $R^3$ | $R^4$ |
|---|---|---|---|
| 388 | D388 | D714 | D388 |
| 389 | D389 | D714 | D389 |
| 390 | D390 | D714 | D390 |
| 391 | D391 | D714 | D391 |
| 392 | D392 | D714 | D392 |
| 393 | D393 | D714 | D393 |
| 394 | D394 | D714 | D394 |
| 395 | D395 | D714 | D395 |
| 396 | D396 | D714 | D396 |
| 397 | D397 | D714 | D397 |
| 398 | D398 | D714 | D398 |
| 399 | D399 | D714 | D399 |
| 400 | D400 | D714 | D400 |
| 401 | D401 | D714 | D401 |
| 402 | D402 | D714 | D402 |
| 403 | D403 | D714 | D403 |
| 404 | D404 | D714 | D404 |
| 405 | D405 | D714 | D405 |
| 406 | D406 | D714 | D406 |
| 407 | D407 | D714 | D407 |
| 408 | D408 | D714 | D408 |
| 409 | D409 | D714 | D409 |
| 410 | D410 | D714 | D410 |
| 411 | D411 | D714 | D411 |
| 412 | D412 | D714 | D412 |
| 413 | D413 | D714 | D413 |
| 414 | D414 | D714 | D414 |
| 415 | D415 | D714 | D415 |
| 416 | D416 | D714 | D416 |
| 417 | D417 | D714 | D417 |
| 418 | D418 | D714 | D418 |
| 419 | D419 | D714 | D419 |
| 420 | D420 | D714 | D420 |
| 421 | D421 | D714 | D421 |
| 422 | D422 | D714 | D422 |
| 423 | D423 | D714 | D423 |
| 424 | D424 | D714 | D424 |
| 425 | D425 | D714 | D425 |

(continued)

| No. | $R^2$ | $R^3$ | $R^4$ |
|---|---|---|---|
| 426 | D426 | D714 | D426 |
| 427 | D427 | D714 | D427 |
| 428 | D428 | D714 | D428 |
| 429 | D429 | D714 | D429 |
| 430 | D430 | D714 | D430 |
| 431 | D431 | D714 | D431 |
| 432 | D432 | D714 | D432 |
| 433 | D433 | D714 | D433 |
| 434 | D434 | D714 | D434 |
| 435 | D435 | D714 | D435 |
| 436 | D436 | D714 | D436 |
| 437 | D437 | D714 | D437 |
| 438 | D438 | D714 | D438 |
| 439 | D439 | D714 | D439 |
| 440 | D440 | D714 | D440 |
| 441 | D441 | D714 | D441 |
| 442 | D442 | D714 | D442 |
| 443 | D443 | D714 | D443 |
| 444 | D444 | D714 | D444 |
| 445 | D445 | D714 | D445 |
| 446 | D446 | D714 | D446 |
| 447 | D447 | D714 | D447 |
| 448 | D448 | D714 | D448 |
| 449 | D449 | D714 | D449 |
| 450 | D450 | D714 | D450 |
| 451 | D451 | D714 | D451 |
| 452 | D452 | D714 | D452 |
| 453 | D453 | D714 | D453 |
| 454 | D454 | D714 | D454 |
| 455 | D455 | D714 | D455 |
| 456 | D456 | D714 | D456 |
| 457 | D457 | D714 | D457 |
| 458 | D458 | D714 | D458 |
| 459 | D459 | D714 | D459 |
| 460 | D460 | D714 | D460 |
| 461 | D461 | D714 | D461 |
| 462 | D462 | D714 | D462 |
| 463 | D463 | D714 | D463 |

(continued)

| No. | $R^2$ | $R^3$ | $R^4$ |
|---|---|---|---|
| 464 | D464 | D714 | D464 |
| 465 | D465 | D714 | D465 |
| 466 | D466 | D714 | D466 |
| 467 | D467 | D714 | D467 |
| 468 | D468 | D714 | D468 |
| 469 | D469 | D714 | D469 |
| 470 | D470 | D714 | D470 |
| 471 | D471 | D714 | D471 |
| 472 | D472 | D714 | D472 |
| 473 | D473 | D714 | D473 |
| 474 | D474 | D714 | D474 |
| 475 | D475 | D714 | D475 |
| 476 | D476 | D714 | D476 |
| 477 | D477 | D714 | D477 |
| 478 | D478 | D714 | D478 |
| 479 | D479 | D714 | D479 |
| 480 | D480 | D714 | D480 |
| 481 | D481 | D714 | D481 |
| 482 | D482 | D714 | D482 |
| 483 | D483 | D714 | D483 |
| 484 | D484 | D714 | D484 |
| 485 | D485 | D714 | D485 |
| 486 | D486 | D714 | D486 |
| 487 | D487 | D714 | D487 |
| 488 | D488 | D714 | D488 |
| 489 | D489 | D714 | D489 |
| 490 | D490 | D714 | D490 |
| 491 | D491 | D714 | D491 |
| 492 | D492 | D714 | D492 |
| 493 | D493 | D714 | D493 |
| 494 | D494 | D714 | D494 |
| 495 | D495 | D714 | D495 |
| 496 | D496 | D714 | D496 |
| 497 | D497 | D714 | D497 |
| 498 | D498 | D714 | D498 |
| 499 | D499 | D714 | D499 |
| 500 | D500 | D714 | D500 |
| 501 | D501 | D714 | D501 |

(continued)

| No. | $R^2$ | $R^3$ | $R^4$ |
|---|---|---|---|
| 502 | D502 | D714 | D502 |
| 503 | D503 | D714 | D503 |
| 504 | D504 | D714 | D504 |
| 505 | D505 | D714 | D505 |
| 506 | D506 | D714 | D506 |
| 507 | D507 | D714 | D507 |
| 508 | D508 | D714 | D508 |
| 509 | D509 | D714 | D509 |
| 510 | D510 | D714 | D510 |
| 511 | D511 | D714 | D511 |
| 512 | D512 | D714 | D512 |
| 513 | D513 | D714 | D513 |
| 514 | D514 | D714 | D514 |
| 515 | D515 | D714 | D515 |
| 516 | D516 | D714 | D516 |
| 517 | D517 | D714 | D517 |
| 518 | D518 | D714 | D518 |
| 519 | D519 | D714 | D519 |
| 520 | D520 | D714 | D520 |
| 521 | D521 | D714 | D521 |
| 522 | D522 | D714 | D522 |
| 523 | D523 | D714 | D523 |
| 524 | D524 | D714 | D524 |
| 525 | D525 | D714 | D525 |
| 526 | D526 | D714 | D526 |
| 527 | D527 | D714 | D527 |
| 528 | D528 | D714 | D528 |
| 529 | D529 | D714 | D529 |
| 530 | D530 | D714 | D530 |
| 531 | D531 | D714 | D531 |
| 532 | D532 | D714 | D532 |
| 533 | D533 | D714 | D533 |
| 534 | D534 | D714 | D534 |
| 535 | D535 | D714 | D535 |
| 536 | D536 | D714 | D536 |
| 537 | D537 | D714 | D537 |
| 538 | D538 | D714 | D538 |
| 539 | D539 | D714 | D539 |

(continued)

| No. | $R^2$ | $R^3$ | $R^4$ |
|---|---|---|---|
| 540 | D540 | D714 | D540 |
| 541 | D541 | D714 | D541 |
| 542 | D542 | D714 | D542 |
| 543 | D543 | D714 | D543 |
| 544 | D544 | D714 | D544 |
| 545 | D545 | D714 | D545 |
| 546 | D546 | D714 | D546 |
| 547 | D547 | D714 | D547 |
| 548 | D548 | D714 | D548 |
| 549 | D549 | D714 | D549 |
| 550 | D550 | D714 | D550 |
| 551 | D551 | D714 | D551 |
| 552 | D552 | D714 | D552 |
| 553 | D553 | D714 | D553 |
| 554 | D554 | D714 | D554 |
| 555 | D555 | D714 | D555 |
| 556 | D556 | D714 | D556 |
| 557 | D557 | D714 | D557 |
| 558 | D558 | D714 | D558 |
| 559 | D559 | D714 | D559 |
| 560 | D560 | D714 | D560 |
| 561 | D561 | D714 | D561 |
| 562 | D562 | D714 | D562 |
| 563 | D563 | D714 | D563 |
| 564 | D564 | D714 | D564 |
| 565 | D565 | D714 | D565 |
| 566 | D566 | D714 | D566 |
| 567 | D567 | D714 | D567 |
| 568 | D568 | D714 | D568 |
| 569 | D569 | D714 | D569 |
| 570 | D570 | D714 | D570 |
| 571 | D571 | D714 | D571 |
| 572 | D572 | D714 | D572 |
| 573 | D573 | D714 | D573 |
| 574 | D574 | D714 | D574 |
| 575 | D575 | D714 | D575 |
| 576 | D576 | D714 | D576 |
| 577 | D577 | D714 | D577 |

(continued)

| No. | $R^2$ | $R^3$ | $R^4$ |
|---|---|---|---|
| 578 | D578 | D714 | D578 |
| 579 | D579 | D714 | D579 |
| 580 | D580 | D714 | D580 |
| 581 | D581 | D714 | D581 |
| 582 | D582 | D714 | D582 |
| 583 | D583 | D714 | D583 |
| 584 | D584 | D714 | D584 |
| 585 | D585 | D714 | D585 |
| 586 | D586 | D714 | D586 |
| 587 | D587 | D714 | D587 |
| 588 | D588 | D714 | D588 |
| 589 | D589 | D714 | D589 |
| 590 | D590 | D714 | D590 |
| 591 | D591 | D714 | D591 |
| 592 | D592 | D714 | D592 |
| 593 | D593 | D714 | D593 |
| 594 | D594 | D714 | D594 |
| 595 | D595 | D714 | D595 |
| 596 | D596 | D714 | D596 |
| 597 | D597 | D714 | D597 |
| 598 | D598 | D714 | D598 |
| 599 | D599 | D714 | D599 |
| 600 | D600 | D714 | D600 |
| 601 | D601 | D714 | D601 |
| 602 | D602 | D714 | D602 |
| 603 | D603 | D714 | D603 |
| 604 | D604 | D714 | D604 |
| 605 | D605 | D714 | D605 |
| 606 | D606 | D714 | D606 |
| 607 | D607 | D714 | D607 |
| 608 | D608 | D714 | D608 |
| 609 | D609 | D714 | D609 |
| 610 | D610 | D714 | D610 |
| 611 | D611 | D714 | D611 |
| 612 | D612 | D714 | D612 |
| 613 | D613 | D714 | D613 |
| 614 | D614 | D714 | D614 |
| 615 | D615 | D714 | D615 |

(continued)

| No. | $R^2$ | $R^3$ | $R^4$ |
|---|---|---|---|
| 616 | D616 | D714 | D616 |
| 617 | D617 | D714 | D617 |
| 618 | D618 | D714 | D618 |
| 619 | D619 | D714 | D619 |
| 620 | D620 | D714 | D620 |
| 621 | D621 | D714 | D621 |
| 622 | D622 | D714 | D622 |
| 623 | D623 | D714 | D623 |
| 624 | D624 | D714 | D624 |
| 625 | D625 | D714 | D625 |
| 626 | D626 | D714 | D626 |
| 627 | D627 | D714 | D627 |
| 628 | D628 | D714 | D628 |
| 629 | D629 | D714 | D629 |
| 630 | D630 | D714 | D630 |
| 631 | D631 | D714 | D631 |
| 632 | D632 | D714 | D632 |
| 633 | D633 | D714 | D633 |
| 634 | D634 | D714 | D634 |
| 635 | D635 | D714 | D635 |
| 636 | D636 | D714 | D636 |
| 637 | D637 | D714 | D637 |
| 638 | D638 | D714 | D638 |
| 639 | D639 | D714 | D639 |
| 640 | D640 | D714 | D640 |
| 641 | D641 | D714 | D641 |
| 642 | D642 | D714 | D642 |
| 643 | D643 | D714 | D643 |
| 644 | D644 | D714 | D644 |
| 645 | D645 | D714 | D645 |
| 646 | D646 | D714 | D646 |
| 647 | D647 | D714 | D647 |
| 648 | D648 | D714 | D648 |
| 649 | D649 | D714 | D649 |
| 650 | D650 | D714 | D650 |
| 651 | D651 | D714 | D651 |
| 652 | D652 | D714 | D652 |
| 653 | D653 | D714 | D653 |

(continued)

| No. | $R^2$ | $R^3$ | $R^4$ |
|---|---|---|---|
| 654 | D654 | D714 | D654 |
| 655 | D655 | D714 | D655 |
| 656 | D656 | D714 | D656 |
| 657 | D657 | D714 | D657 |
| 658 | D658 | D714 | D658 |
| 659 | D659 | D714 | D659 |
| 660 | D660 | D714 | D660 |
| 661 | D661 | D714 | D661 |
| 662 | D662 | D714 | D662 |
| 663 | D663 | D714 | D663 |
| 664 | D664 | D714 | D664 |
| 665 | D665 | D714 | D665 |
| 666 | D666 | D714 | D666 |
| 667 | D667 | D714 | D667 |
| 668 | D668 | D714 | D668 |
| 669 | D669 | D714 | D669 |
| 670 | D670 | D714 | D670 |
| 671 | D671 | D714 | D671 |
| 672 | D672 | D714 | D672 |
| 673 | D673 | D714 | D673 |
| 674 | D674 | D714 | D674 |
| 675 | D675 | D714 | D675 |
| 676 | D676 | D714 | D676 |
| 677 | D677 | D714 | D677 |
| 678 | D678 | D714 | D678 |
| 679 | D679 | D714 | D679 |
| 680 | D680 | D714 | D680 |
| 681 | D681 | D714 | D681 |
| 682 | D682 | D714 | D682 |
| 683 | D683 | D714 | D683 |
| 684 | D684 | D714 | D684 |
| 685 | D685 | D714 | D685 |
| 686 | D686 | D714 | D686 |
| 687 | D687 | D714 | D687 |
| 688 | D688 | D714 | D688 |
| 689 | D689 | D714 | D689 |
| 690 | D690 | D714 | D690 |
| 691 | D691 | D714 | D691 |

(continued)

| No. | $R^2$ | $R^3$ | $R^4$ |
|---|---|---|---|
| 692 | D692 | D714 | D692 |
| 693 | D693 | D714 | D693 |
| 694 | D694 | D714 | D694 |
| 695 | D695 | D714 | D695 |
| 696 | D696 | D714 | D696 |
| 697 | D697 | D714 | D697 |
| 698 | D698 | D714 | D698 |
| 699 | D699 | D714 | D699 |
| 700 | D700 | D714 | D700 |
| 701 | D701 | D714 | D701 |
| 702 | D702 | D714 | D702 |
| 703 | D703 | D714 | D703 |
| 704 | D704 | D714 | D704 |
| 705 | D705 | D714 | D705 |
| 706 | D706 | D714 | D706 |
| 707 | D707 | D714 | D707 |
| 708 | D708 | D714 | D708 |
| 709 | D709 | D714 | D709 |
| 710 | D710 | D714 | D710 |
| 711 | D711 | D714 | D711 |
| 712 | D712 | D714 | D712 |
| 713 | D713 | D714 | D713 |

Table 2

| No. | $R^2$ | $R^3$ | $R^4$ | Note |
|---|---|---|---|---|
| 1 ~ 713 | D1~D713 | D714 | D1~D713 | $R^2 = R^4$ |
| 714 ~ 1426 | D1~D713 | D715 | D1~D713 | |
| 1427 ~ 2139 | D1~D713 | D716 | D1~D713 | |
| 2140 ~ 2852 | D1~D713 | D717 | D1~D713 | |
| 2853 ~ 3565 | D1~D713 | D718 | D1~D713 | |
| 3566 ~ 4278 | D1~D713 | D719 | D1~D713 | |
| 4279 ~ 4991 | D1~D713 | D720 | D1~D713 | |
| 4992 ~ 5704 | D1~D713 | D721 | D1~D713 | |
| 5705 ~ 6417 | D1~D713 | D722 | D1~D713 | |
| 6418 ~ 7130 | D1~D713 | D723 | D1~D713 | |
| 7131 ~ 7843 | D1~D713 | D724 | D1~D713 | |
| 7844 ~ 8556 | D1~D713 | D725 | D1~D713 | |
| 8557 ~ 9269 | D1~D713 | D726 | D1~D713 | |
| 9270 ~ 9982 | D1~D713 | D727 | D1~D713 | |
| 9983 ~ 10695 | D1~D713 | D728 | D1~D713 | |
| 10696 ~ 11408 | D1~D713 | D729 | D1~D713 | |
| 11409 ~ 12121 | D1~D713 | D730 | D1~D713 | |
| 12122 ~ 12834 | D1~D713 | D731 | D1~D713 | |
| 12835 ~ 13547 | D1~D713 | D732 | D1~D713 | |
| 13548 ~ 14260 | D1~D713 | D733 | D1~D713 | |
| 14261 ~ 14973 | D1~D713 | D734 | D1~D713 | |
| 14974 ~ 15686 | D1~D713 | D735 | D1~D713 | |
| 15687 ~ 16399 | D1~D713 | D736 | D1~D713 | |
| 16400 ~ 17112 | D1~D713 | D737 | D1~D713 | |
| 17113 ~ 17825 | D1~D713 | D738 | D1~D713 | |
| 17826 ~ 18538 | D1~D713 | D739 | D1~D713 | |
| 18539 ~ 19251 | D1~D713 | D740 | D1~D713 | |
| 19252 ~ 19964 | D1~D713 | D741 | D1~D713 | |
| 19965 ~ 20677 | D1~D713 | D742 | D1~D713 | |
| 20678 ~ 21390 | D1~D713 | D743 | D1~D713 | |
| 21391 ~ 22103 | D1~D713 | D744 | D1~D713 | |
| 22104 ~ 22816 | D1~D713 | D745 | D1~D713 | |
| 22817 ~ 23529 | D1~D713 | D746 | D1~D713 | |
| 23530 ~ 24242 | D1~D713 | D747 | D1~D713 | |
| 24243 ~ 24955 | D1~D713 | D748 | D1~D713 | |

(continued)

| No. | $R^2$ | $R^3$ | $R^4$ | Note |
|---|---|---|---|---|
| 24956 ~ 25668 | D714 | D1~D713 | D714 | $R^2 = R^4$ |
| 25669 ~ 26381 | D715 | D1~D713 | D715 | |
| 26382 ~ 27094 | D716 | D1~D713 | D716 | |
| 27095 ~ 27807 | D717 | D1~D713 | D717 | |
| 27808 ~ 28520 | D718 | D1~D713 | D718 | |
| 28521 ~ 29233 | D719 | D1~D713 | D719 | |
| 29234 ~ 29946 | D720 | D1~D713 | D720 | |
| 29947 ~ 30659 | D721 | D1~D713 | D721 | |
| 30660 ~ 31372 | D722 | D1~D713 | D722 | |
| 31373 ~ 32085 | D723 | D1~D713 | D723 | |
| 32086 ~ 32798 | D724 | D1~D713 | D724 | $R^2 = R^4$ |
| 32799 ~ 33511 | D725 | D1~D713 | D725 | |
| 33512 ~ 34224 | D726 | D1~D713 | D726 | |
| 34225 ~ 34937 | D727 | D1~D713 | D727 | |
| 34938 ~ 35650 | D728 | D1~D713 | D728 | |
| 35651 ~ 36363 | D729 | D1~D713 | D729 | |
| 36364 ~ 37076 | D730 | D1~D713 | D730 | |
| 37077 ~ 37789 | D731 | D1~D713 | D731 | |
| 37790 ~38502 | D732 | D1~D713 | D732 | |
| 38503 ~ 39215 | D733 | D1~D713 | D733 | |
| 39216 ~ 39928 | D734 | D1~D713 | D734 | |
| 39929 ~ 40641 | D735 | D1~D713 | D735 | |
| 40642 ~ 41354 | D736 | D1~D713 | D736 | |
| 41355 ~ 42067 | D737 | D1~D713 | D737 | |
| 42068 ~ 42780 | D738 | D1~D713 | D738 | |
| 42781 ~ 43493 | D739 | D1~D713 | D739 | |
| 43494 ~ 44206 | D740 | D1~D713 | D740 | |
| 44207 ~ 44919 | D741 | D1~D713 | D741 | |
| 44920 ~ 45632 | D742 | D1~D713 | D742 | |
| 45633 ~ 46345 | D743 | D1~D713 | D743 | |
| 46346 ~ 47058 | D744 | D1~D713 | D744 | |
| 47059 ~ 47771 | D745 | D1~D713 | D745 | |
| 47772 ~ 48484 | D746 | D1~D713 | D746 | |
| 48485 ~ 49197 | D747 | D1~D713 | D747 | |
| 49198 ~ 49910 | D748 | D1~D713 | D748 | |

(continued)

| No. | $R^2$ | $R^3$ | $R^4$ | Note |
|---|---|---|---|---|
| 49911～50623 | D714 | D714 | D1~D713 | $R^2 = R^3$ |
| 50624～51336 | D715 | D715 | D1~D713 | |
| 51337～52049 | D716 | D716 | D1~D713 | |
| 52050～52762 | D717 | D717 | D1~D713 | |
| 52763～53475 | D718 | D718 | D1~D713 | |
| 53476～54188 | D719 | D719 | D1~D713 | |
| 54189～54901 | D720 | D720 | D1~D713 | |
| 54902～55614 | D721 | D721 | D1~D713 | |
| 55615～56327 | D722 | D722 | D1~D713 | |
| 56328～57040 | D723 | D723 | D1~D713 | |
| 57041～57753 | D724 | D724 | D1~D713 | |
| 57754～58466 | D725 | D725 | D1~D713 | |
| 58467～59179 | D726 | D726 | D1~D713 | |
| 59180～59892 | D727 | D727 | D1~D713 | |
| 59893～60605 | D728 | D728 | D1~D713 | |
| 60606～61318 | D729 | D729 | D1~D713 | |
| 61319～62031 | D730 | D730 | D1~D713 | |
| 62032～62744 | D731 | D731 | D1~D713 | |
| 62745～63457 | D732 | D732 | D1~D713 | |
| 63458～64170 | D733 | D733 | D1~D713 | |
| 64171～64883 | D734 | D734 | D1～D713 | $R^2 = R^3$ |
| 64884～65596 | D735 | D735 | D1~D713 | |
| 65597～66309 | D736 | D736 | D1~D713 | |
| 66310～67022 | D737 | D737 | D1~D713 | |
| 67023～67735 | D738 | D738 | D1~D713 | |
| 67736～68448 | D739 | D739 | D1~D713 | |
| 68449～69161 | D740 | D740 | D1~D713 | |
| 69162～69874 | D741 | D741 | D1~D713 | |
| 69875～70587 | D742 | D742 | D1~D713 | |
| 70588～71300 | D743 | D743 | D1~D713 | |
| 71301～72013 | D744 | D744 | D1~D713 | |
| 72014～72726 | D745 | D745 | D1~D713 | |
| 72727～73439 | D746 | D746 | D1~D713 | |
| 73440 ~74152 | D747 | D747 | D1～D713 | |
| 74153～74865 | D748 | D748 | D1～D713 | |

(continued)

| No. | $R^2$ | $R^3$ | $R^4$ | Note |
|---|---|---|---|---|
| 74866~75578 | D1~D713 | D1~D713 | D714 | $R^2$ = $R^3$ |
| 75579~76291 | D1~D713 | D1~D713 | D715 | |
| 76292~77004 | D1~D713 | D1~D713 | D716 | |
| 77005~77717 | D1~D713 | D1~D713 | D717 | |
| 77718~78430 | D1~D713 | D1~D713 | D718 | |
| 78431~79143 | D1~D713 | D1~D713 | D719 | |
| 79144~79856 | D1~D713 | D1~D713 | D720 | |
| 79857~80569 | D1~D713 | D1~D713 | D721 | |
| 80570~81282 | D1~D713 | D1~D713 | D722 | |
| 81283~81995 | D1~D713 | D1~D713 | D723 | |
| 81996~82708 | D1~0713 | D1~D713 | D724 | |
| 82709~83421 | D1~D713 | D1~D713 | D725 | |
| 83422~84134 | D1~D713 | D1~D713 | D726 | |
| 84135~84847 | D1~D713 | D1~D713 | D727 | |
| 84848~85560 | D1~D713 | D1~D713 | D728 | |
| 85561~86273 | D1~D713 | D1~D713 | D729 | |
| 86274~86986 | D1~D713 | D1~D713 | D730 | |
| 86987~87699 | D1~D713 | D1~D713 | D731 | |
| 87700~88412 | D1~D713 | D1~D713 | D732 | |
| 88413~89125 | D1~D713 | D1~D713 | D733 | |
| 89126~89838 | D1~D713 | D1~D713 | D734 | |
| 89839~90551 | D1~D713 | D1~D713 | D735 | |
| 90552~91264 | D1~D713 | D1~D713 | D736 | |
| 91265~91977 | D1~D713 | D1~D713 | D737 | |
| 91978~92690 | D1~D713 | D1~D713 | D738 | |
| 92691~93403 | D1~D713 | D1~D713 | D739 | |
| 93404~94116 | D1~D713 | D1~D713 | D740 | |
| 94117~94829 | D1~D713 | D1~D713 | D741 | |
| 94830~95542 | D1~D713 | D1~D713 | D742 | |
| 95543~96255 | D1-D713 | D1~D713 | D743 | |
| 96256~96968 | D1~D713 | D1~D713 | D744 | $R^2$ = $R^3$ |
| 96969~97681 | D1~D713 | D1~D713 | D745 | |
| 97682~98394 | D1~D713 | D1~D713 | D746 | |
| 98395~99107 | D1~D713 | D1~D713 | D747 | |
| 99108~99820 | D1~D713 | D1~D713 | D748 | |

The compounds specified by the above numbers are all individually disclosed. In addition, among the specific examples of the compounds, in the case where a rotamer is present, a mixture of rotamers and each separated rotamer are also disclosed in the description herein.

In one aspect of the present invention, compounds are selected from Compounds 1 to 99820.

In one aspect of the present invention, compounds are selected from Compounds 1 to 24955. In one aspect of the present invention, compounds are selected from Compounds 24956 to 49910. In one aspect of the present invention, compounds are selected from Compounds 49911 to 74865. In one aspect of the present invention, compounds are selected from Compounds 74866 to 99820.

In one aspect of the present invention, compounds are selected from Compounds 1 to 713, 8557 to 9269, 24956 to 25668, 33512 to 34224, 49911 to 50623, 58467 to 59179, 74866 to 75578, and 83422 to 84134. In one aspect of the present invention, compounds are selected from Compounds 4992 to 8556, 13548 to 17112, 21391 to 24955, 29947 to 33511, 38503 to 42067, 46346 to 49910, 54902 to 58466, 63458 to 67022, 71301 to 74865, 79857 to 83421, 88413 to 91977, and 96256 to 99820.

In the above, structures where, in the general formula (1), $Ar^1$ and $Ar^2$ are phenyl groups (Ar1), $X^1$ to $X^3$ are nitrogen atoms (N), $L^1$ is a single bond, and $R^1$ to $R^4$ are groups specified as in Table 1 and Table 2 are specified as structures of Compounds 1 to 99820. $Ar^1$ and $Ar^2$ in Compounds 1 to 99820 were changed as in Table 3, in which the resultant compounds were sequentially displayed in a table format. In Table 3, Compounds 1 to 99820 are also shown for clarifying the correspondence relationship. For example, Compound 1(1) indicates a compound having a structure in which $Ar^2$ of Compound 1 is changed to Ar2. Compound 2(1) indicates a compound having a structure in which $Ar^2$ of Compound 2 is changed to Ar2. Compound 99820(1) indicates a compound having a structure in which $Ar^2$ of Compound 99820 is changed to Ar2. Compounds 1(2) to 99820(2) and the subsequent compounds are specified in the same manner. $X^1$ to $X^3$ of the compounds specified in Table 3 are all nitrogen atoms (N) and $L^1$ is a single bond.

Table 3

| No. | $Ar^1$ | $Ar^2$ |
|---|---|---|
| 1 ~ 99820 | Ar1 | Ar1 |
| 1(1) ~ 99820(1) | Ar1 | Ar2 |
| 1(2) ~ 99820(2) | Ar1 | Ar3 |
| 1(3) ~ 99820(3) | Ar1 | Ar4 |
| 1(4) ~ 99820(4) | Ar1 | Ar5 |
| 1(5) ~ 99820(5) | Ar1 | Ar6 |
| 1(6) ~ 99820(6) | Ar1 | Ar7 |
| 1(7) ~ 99820(7) | Ar1 | Ar8 |
| 1(8) ~ 99820(8) | Ar1 | Ar9 |
| 1(9) ~ 99820(9) | Ar1 | Ar10 |
| 1(10) ~ 99820(10) | Ar1 | Ar11 |
| 1(11) ~ 99820(11) | Ar1 | Ar12 |
| 1(12) ~ 99820(12) | Ar1 | Ar13 |
| 1(13) ~ 99820(13) | Ar1 | Ar14 |
| 1(14) ~ 99820(14) | Ar1 | Ar15 |
| 1(15) ~ 99820(15) | Ar1 | Ar16 |
| 1(16) ~ 99820(16) | Ar1 | Ar17 |
| 1(17) ~ 99820(17) | Ar1 | Ar18 |
| 1(18) ~ 99820(18) | Ar1 | Ar19 |
| 1(19) ~ 99820(19) | Ar1 | Ar20 |
| 1(20) ~ 99820(20) | Ar1 | Ar21 |
| 1(21) ~ 99820(21) | Ar1 | Ar22 |
| 1(22) ~ 99820(22) | Ar1 | Ar23 |
| 1(23) ~ 99820(23) | Ar1 | Ar24 |

(continued)

| No. | $Ar^1$ | $Ar^2$ |
|---|---|---|
| 1(24) ~ 99820(24) | Ar1 | Ar25 |
| 1(25) ~ 99820(25) | Ar1 | Ar26 |
| 1(26) ~ 99820(26) | Ar1 | Ar27 |
| 1(27) ~ 99820(27) | Ar1 | Ar28 |
| 1(28) ~ 99820(28) | Ar1 | Ar29 |
| 1(29) ~ 99820(29) | Ar1 | Ar30 |
| 1(30) ~ 99820(30) | Ar1 | Ar31 |
| 1(31) ~ 99820(31) | Ar1 | Ar32 |
| 1(32) ~ 99820(32) | Ar1 | Ar33 |
| 1(33) ~ 99820(33) | Ar1 | Ar34 |
| 1(34) ~ 99820(34) | Ar1 | Ar35 |
| 1(35) ~ 99820(35) | Ar1 | Ar36 |
| 1(36) ~ 99820(36) | Ar1 | Ar37 |
| 1(37) ~ 99820(37) | Ar1 | Ar38 |
| 1(38) ~ 99820(38) | Ar1 | Ar39 |
| 1(39) ~ 99820(39) | Ar1 | Ar40 |
| 1(40) ~ 99820(40) | Ar1 | Ar41 |
| 1(41) ~ 99820(41) | Ar1 | Ar42 |
| 1(42) ~ 99820(42) | Ar1 | Ar43 |
| 1(43) ~ 99820(43) | Ar1 | Ar44 |
| 1(44) ~ 99820(44) | Ar1 | Ar45 |
| 1(45) ~ 99820(45) | Ar1 | Ar46 |
| 1(46) ~ 99820(46) | Ar1 | Ar47 |
| 1(47) ~ 99820(47) | Ar1 | Ar48 |
| 1(48) ~ 99820(48) | Ar1 | Ar49 |
| 1(49) ~ 99820(49) | Ar1 | Ar50 |
| 1(50) ~ 99820(50) | Ar1 | Ar51 |
| 1(51) ~ 99820(51) | Ar1 | Ar52 |
| 1(52) ~ 99820(52) | Ar1 | Ar53 |
| 1(53) ~ 99820(53) | Ar1 | Ar54 |
| 1(54) ~ 99820(54) | Ar1 | Ar55 |
| 1(55) ~ 99820(55) | Ar1 | Ar56 |
| 1(56) ~ 99820(56) | Ar1 | Ar57 |
| 1(57) ~ 99820(57) | Ar1 | Ar58 |
| 1(58) ~ 99820(58) | Ar1 | Ar59 |
| 1(59) ~ 99820(59) | Art | Ar60 |
| 1(60) ~ 99820(60) | Ar1 | Ar61 |
| 1(61) ~ 99820(61) | Ar1 | Ar62 |

(continued)

| No. | $Ar^1$ | $Ar^2$ |
|---|---|---|
| 1(62) ~ 99820(62) | Ar1 | Ar63 |
| 1(63) ~ 99820(63) | Ar1 | Ar64 |
| 1(64) ~ 99820(64) | Ar1 | Ar65 |
| 1(65) ~ 99820(65) | Ar1 | Ar66 |
| 1(66) ~ 99820(66) | Ar1 | Ar67 |
| 1(67) ~ 99820(67) | Ar2 | Ar2 |
| 1(68) ~ 99820(68) | Ar3 | Ar3 |
| 1(69) ~ 99820(69) | Ar4 | Ar4 |
| 1(70) ~ 99820(70) | Ar5 | Ar5 |
| 1(71) ~ 99820(71) | Ar6 | Ar6 |
| 1(72) ~ 99820(72) | Ar7 | Ar7 |
| 1(73) ~ 99820(73) | Ar8 | Ar8 |
| 1(74) ~ 99820(74) | Ar9 | Ar9 |
| 1(75) ~ 99820(75) | Ar10 | Ar10 |
| 1(76) ~ 99820(76) | Ar11 | Ar11 |
| 1(77) ~ 99820(77) | Ar12 | Ar12 |
| 1(78) ~ 99820(78) | Ar13 | Ar13 |
| 1(79) ~ 99820(79) | Ar14 | Ar14 |
| 1(80) ~ 99820(80) | Ar15 | Ar15 |
| 1(81) ~ 99820(81) | Ar16 | Ar16 |
| 1(82) ~ 99820(82) | Ar17 | Ar17 |
| 1(83) ~ 99820(83) | Ar18 | Ar18 |
| 1(84) ~ 99820(84) | Ar19 | Ar19 |
| 1(85) ~ 99820(85) | Ar20 | Ar20 |
| 1(86) ~ 99820(86) | Ar21 | Ar21 |
| 1(87) ~ 99820(87) | Ar22 | Ar22 |
| 1(88) ~ 99820(88) | Ar23 | Ar23 |
| 1(89) ~ 99820(89) | Ar24 | Ar24 |
| 1(90) ~ 99820(90) | Ar25 | Ar25 |
| 1(91) ~ 99820(91) | Ar26 | Ar26 |
| 1(92) ~ 99820(92) | Ar27 | Ar27 |
| 1(93) ~ 99820(93) | Ar28 | Ar28 |
| 1(94) ~ 99820(94) | Ar29 | Ar29 |
| 1(95) ~ 99820(95) | Ar30 | Ar30 |
| 1(96) ~ 99820(96) | Ar31 | Ar31 |
| 1(97) ~ 99820(97) | Ar32 | Ar32 |
| 1(98) ~ 99820(98) | Ar33 | Ar33 |
| 1(99) ~ 99820(99) | Ar34 | Ar34 |

(continued)

| No. | Ar1 | Ar2 |
|---|---|---|
| 1(100) ~ 99820(100) | Ar35 | Ar35 |
| 1(101) ~ 99820(101) | Ar36 | Ar36 |
| 1(102) ~ 99820(102) | Ar37 | Ar37 |
| 1(103) ~ 99820(103) | Ar38 | Ar38 |
| 1(104) ~ 99820(104) | Ar39 | Ar39 |
| 1(105) ~ 99820(105) | Ar40 | Ar40 |
| 1(106) ~ 99820(106) | Ar41 | Ar41 |
| 1(107) ~ 99820(107) | Ar42 | Ar42 |
| 1(108) ~ 99820(108) | Ar43 | Ar43 |
| 1(109) ~ 99820(109) | Ar44 | Ar44 |
| 1(110) ~ 99820(110) | Ar45 | Ar45 |
| 1(111) ~ 99820(111) | Ar46 | Ar46 |
| 1(112) ~ 99820(112) | Ar47 | Ar47 |
| 1(113) ~ 99820(113) | Ar48 | Ar48 |
| 1(114) ~ 99820(114) | Ar49 | Ar49 |
| 1(115) ~ 99820(115) | Ar50 | Ar50 |
| 1(116) ~ 99820(116) | Ar51 | Ar51 |
| 1(117) ~ 99820(117) | Ar52 | Ar52 |
| 1(118) ~ 99820(118) | Ar53 | Ar53 |
| 1(119) ~ 99820(119) | Ar54 | Ar54 |
| 1(120) ~ 99820(120) | Ar55 | Ar55 |
| 1(121) ~ 99820(121) | Ar66 | Ar56 |
| 1(122) ~ 99820(122) | Ar57 | Ar57 |
| 1(123) ~ 99820(123) | Ar58 | Ar58 |
| 1(124) ~ 99820(124) | Ar59 | Ar59 |
| 1(125) ~ 99820(125) | Ar60 | Ar60 |
| 1(126) ~ 99820(126) | Ar61 | Ar61 |
| 1(127) ~ 99820(127) | Ar62 | Ar62 |
| 1(128) ~ 99820(128) | Ar63 | Ar63 |
| 1(129) ~ 99820(129) | Ar64 | Ar64 |
| 1(130) ~ 99820(130) | Ar65 | Ar65 |
| 1(131) ~ 99820(131) | Ar66 | Ar66 |
| 1(132) ~ 99820(132) | Ar67 | Ar67 |

In the above, specific examples where $R^1$ in the general formula (1) is a hydrogen atom are exemplified as Compounds 1 to 99820, and 1(1) to 99820(132). Those obtained by replacing the hydrogen atom of $R^1$ in these compounds to a deuterium atom are further exemplified herein as Compounds 1d to 99820d, and 1d(1) to 99820d(132).

In one preferred aspect of the present invention, the compound represented by the general formula (1) is selected from the following group of compounds.

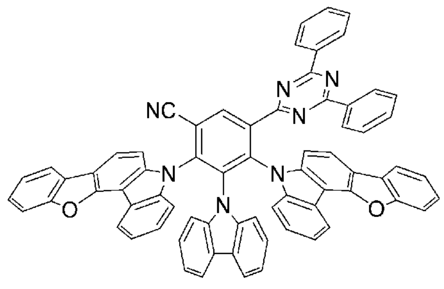

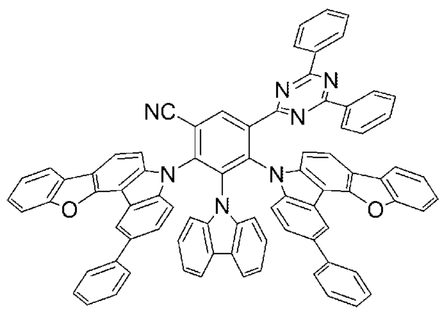

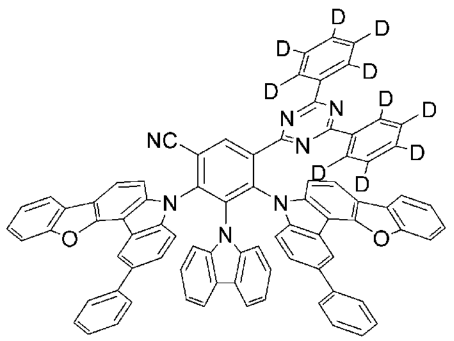

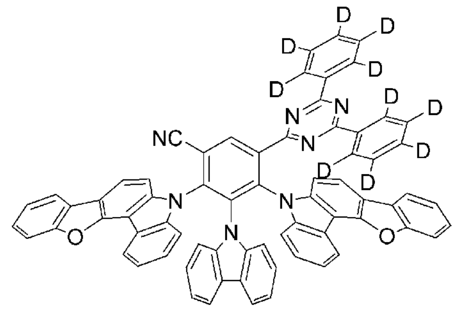

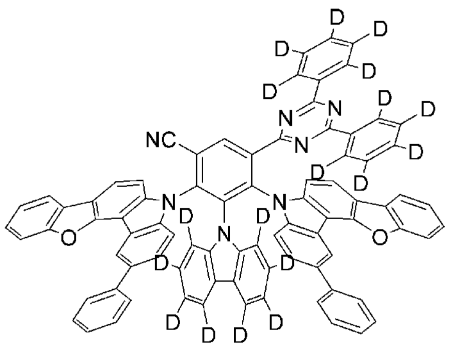

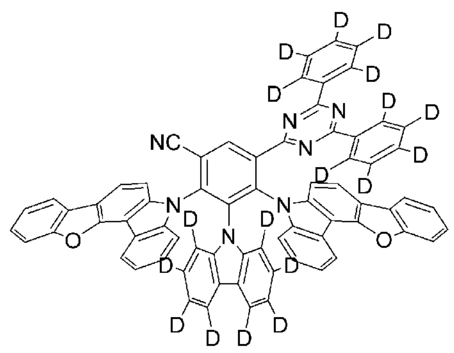

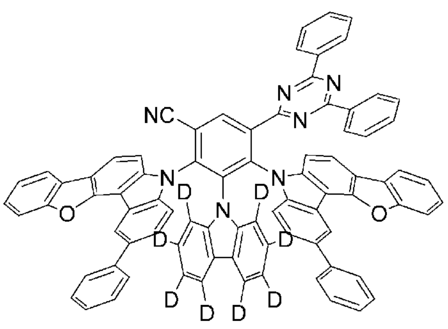

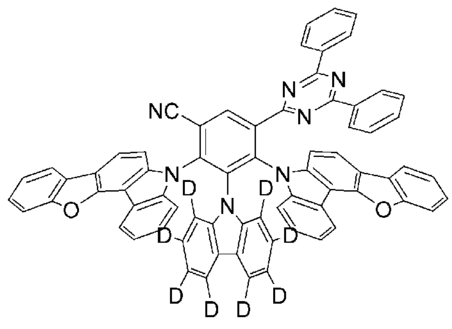

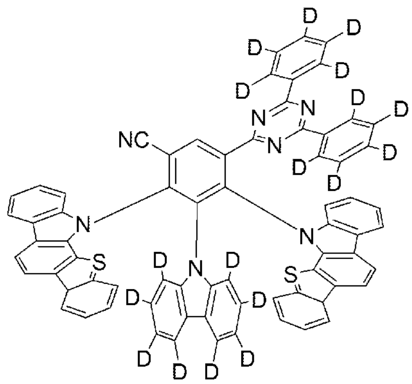

In one preferred aspect of the present invention, the compound represented by the general formula (1) is selected from the following group of compounds.

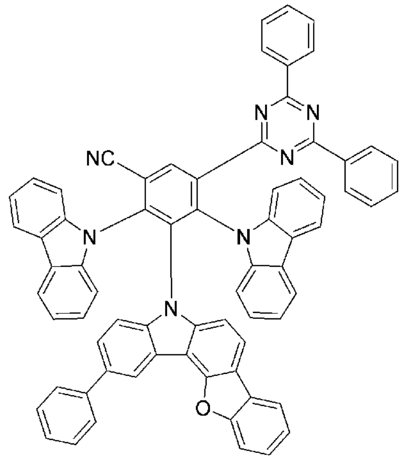

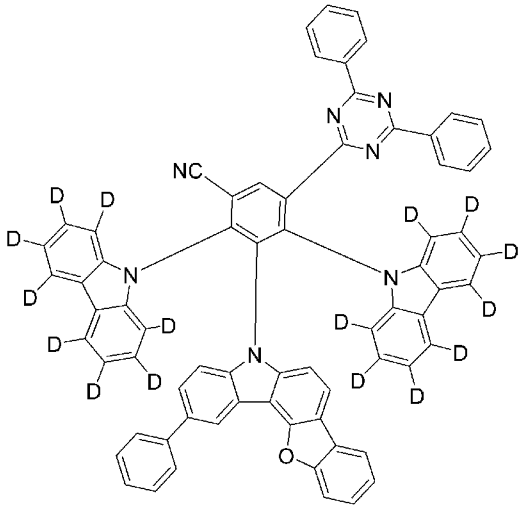

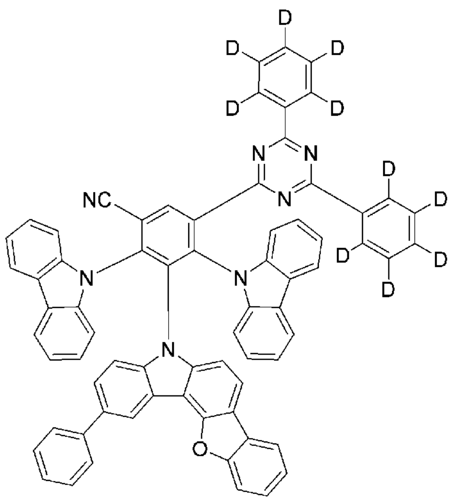

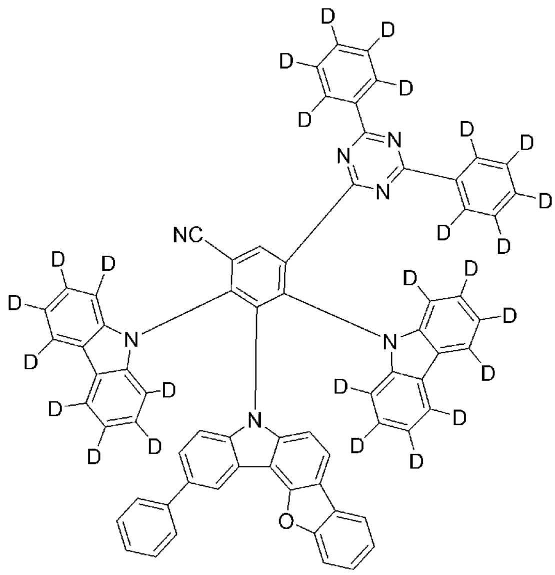

In one preferred aspect of the present invention, the compound represented by the general formula (1) is selected from the following group of compounds.

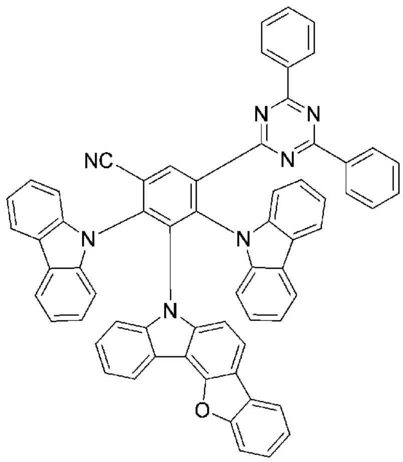

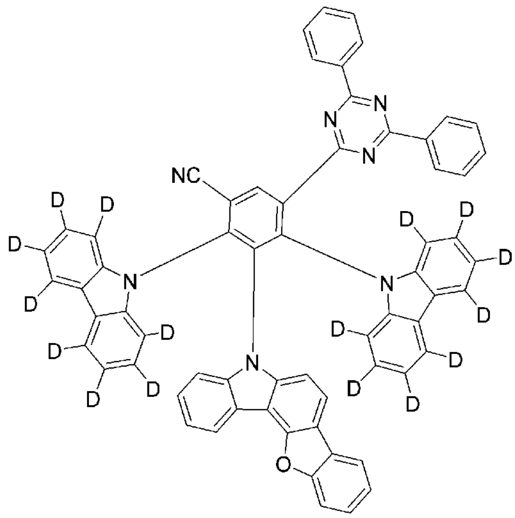

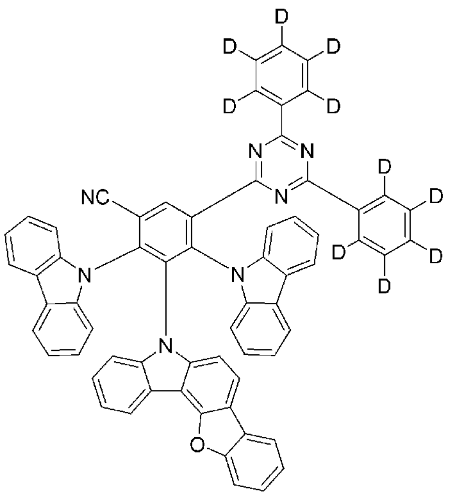

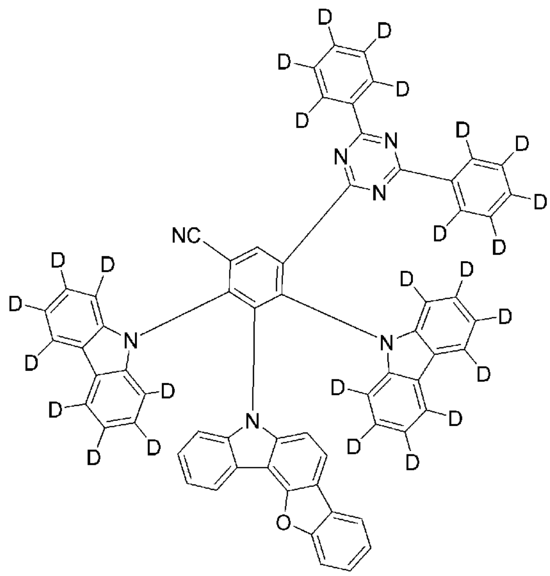

In one preferred aspect of the present invention, the compound represented by the general formula (1) is selected from the following group of compounds.

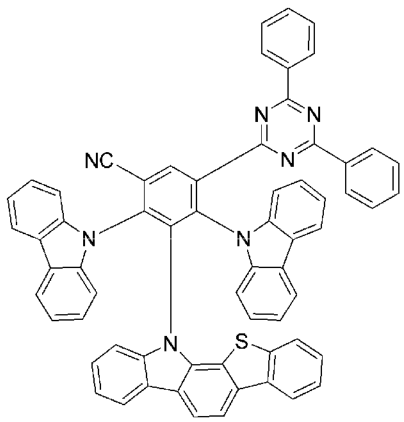

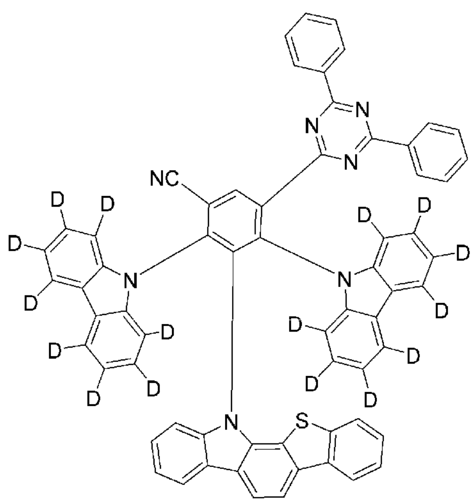

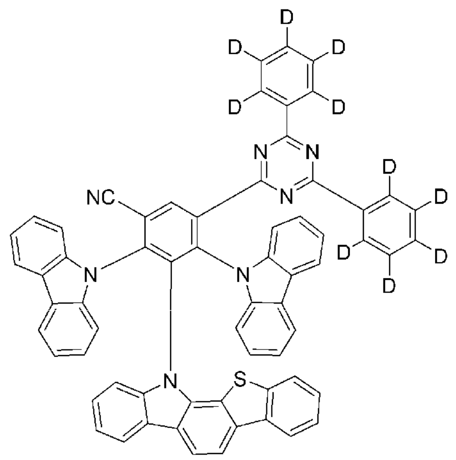 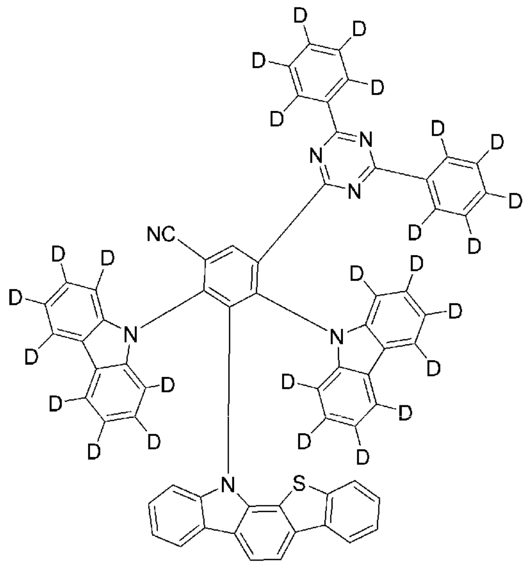

The molecular weight of the compound represented by the general formula (1) is preferably 1500 or less, more preferably 1200 or less, still more preferably 1000 or less, and even more preferably 900 or less, for example, in the case where an organic layer containing the compound represented by the general formula (1) is intended to be film-formed and used by a vapor deposition method. The lower limit of the molecular weight is the molecular weight of the minimum compound represented by the general formula (1).

The compound represented by the general formula (1) can be formed into a film by a coating method regardless of the molecular weight. When the coating method is used, even a compound having a relatively large molecular weight can be formed into a film. The compound represented by the general formula (1) has an advantage of being easily dissolved in an organic solvent. For this reason, the compound represented by the general formula (1) is easily applicable to a coating method and is easily purified to increase its purity.

It is also conceivable to use a compound containing a plurality of structures represented by the general formula (1) in a molecule as a light emitting material by applying the present invention.

For example, it is conceivable that a polymer obtained by allowing a polymerizable group to be present in the structure represented by the general formula (1) in advance and polymerizing the polymerizable group is used as the light emitting material. For example, it is conceivable that a polymer having a repeating unit is obtained by preparing a monomer containing a polymerizable functional group at any site of the general formula (1) and polymerizing the monomer alone or copolymerizing the monomer with another monomer, and the polymer is used as the light emitting material. Alternatively, it is also conceivable to obtain a dimer or a trimer by coupling compounds having a structure represented by the general formula (1) to each other and to use the dimer or the trimer as a light emitting material.

Examples of the polymer having a repeating unit containing a structure represented by the general formula (1) include polymers containing a structure represented by any one of the following two general formulae.

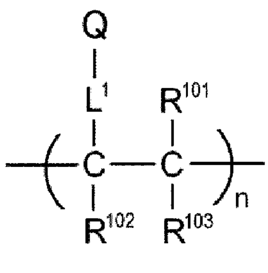 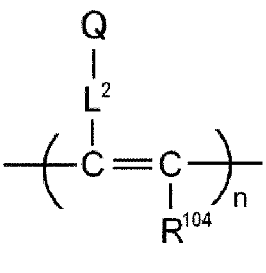

In the above general formulae, Q represents a group containing the structure represented by the general formula (1), and $L^1$ and $L^2$ represent a linking group. The linking group preferably has 0 to 20 carbon atoms, more preferably 1 to 15 carbon atoms, and still more preferably 2 to 10 carbon atoms. The linking group preferably has a structure represented by $-X^{11}-L^{11}-$. Here, $X^{11}$ represents an oxygen atom or a sulfur atom, and is preferably an oxygen atom. $L^{11}$ represents a linking group, and is preferably a substituted or unsubstituted alkylene group or a substituted or unsubstituted arylene group, and more preferably a substituted or unsubstituted alkylene group having 1 to 10 carbon atoms or a substituted or unsubstituted phenylene group.

In the above general formulae, $R^{101}$, $R^{102}$, $R^{103}$ and $R^{104}$ each independently represent a substituent. It is

preferably a substituted or unsubstituted alkyl group having 1 to 6 carbon atoms, a substituted or unsubstituted alkoxy group having 1 to 6 carbon atoms, or a halogen atom, more preferably an unsubstituted alkyl group having 1 to 3 carbon atoms, an unsubstituted alkoxy group having 1 to 3 carbon atoms, a fluorine atom, or a chlorine atom, and still more preferably an unsubstituted alkyl group having 1 to 3 carbon atoms or an unsubstituted alkoxy group having 1 to 3 carbon atoms.

The linking group represented by $L^1$ and $L^2$ can bond to any site of the general formula (1) constituting Q. Two or more linking groups can be linked to one Q to form a cross-linked structure or a network structure.

Specific structural examples of the repeating unit include structures represented by the following formulae.

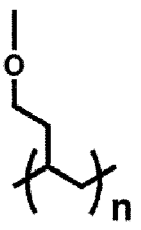
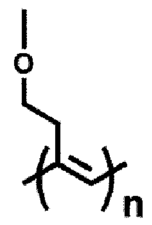
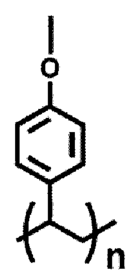
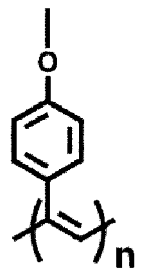

The polymer having a repeating unit including these formulae can be synthesized by introducing a hydroxy group into any site of the general formula (1), reacting the following compound using the hydroxy group as a linker to introduce a polymerizable group, and polymerizing the polymerizable group.

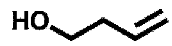
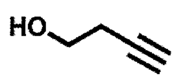
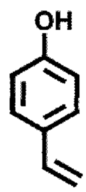
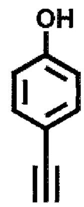

The polymer having a structure represented by the general formula (1) in the molecule can be a polymer having only a repeating unit that has the structure represented by the general formula (1), or can be a polymer containing a repeating unit that has any other structure. The repeating unit having the structure represented by the general formula (1) to be contained in the polymer can be a single kind or two or more kinds. The repeating unit not having the structure represented by the general formula (1) includes those derived from monomers used in general copolymerization. For example, it includes repeating units derived from monomers having an ethylenically unsaturated bond, such as ethylene or styrene.

In some embodiments, the compound represented by the general formula (1) is a light emitting material.

In some embodiments, the compound represented by the general formula (1) is a compound capable of emitting delayed fluorescence.

In some embodiments of the present disclosure, the compound represented by the general formula (1) is, when excited thermally or by an electronic means, able to emit light in a UV region, emit light of blue, green, yellow, orange, or red in a visible spectral region (e.g., about 420 nm to about 500 nm, about 500 nm to about 600 nm, or about 600 nm to about 700 nm) or emit light in a near IR region.

In preferred embodiments of the present disclosure, the compound represented by the general formula (1) is, when excited thermally or by an electronic means, able to emit light of a green region in a visible spectrum (e.g., about 490 nm to about 575 nm, about 510 nm).

In some embodiments of the present disclosure, an organic semiconductor device using the compound represented by the general formula (1) can be produced. The organic semiconductor device referred to herein can be an organic optical device in which light is interposed or an organic device in which light is not interposed. The organic optical device can be an organic light emitting device in which the device emits light, an organic light receiving device in which the device receives light, or a device in which energy transfer by light occurs in the device. In some embodiments of the present disclosure, an organic optical device such as an organic electroluminescent device or a solid-state imaging device (for example, a CMOS image sensor) can be produced by using the compound represented by the general formula (1). In some embodiments of the present disclosure, a CMOS (complementary metal-oxide semiconductor) or the like using the compound represented by the general formula (1) can be produced.

Electronic characteristics of small-molecule chemical substance libraries can be calculated by known *ab initio*

quantum chemistry calculation. For example, according to time-dependent density functional theory calculation using 6-31G* as a basis, and a functional group known as Becke's three parameters, Lee-Yang-Parr hybrid functionals, the Hartree-Fock equation (TD-DFTB3LYP/6-31G*) is analyzed and molecular fractions (parts) having HOMO not lower than a specific threshold value and LUMO not higher than a specific threshold value can be screened.

With that, for example, in the presence of a HOMO energy (for example, ionizing potential) of -6.5 eV or more, a donor part ("D") can be selected. On the other hand, for example, in the presence of a LUMO energy (for example, electron affinity) of -0.5 eV or less, an acceptor part ("A") can be selected. A bridge part ("B") is a strong conjugated system, for example, capable of strictly limiting the acceptor part and the donor part in a specific three-dimensional configuration, and therefore prevents the donor part and the acceptor part from overlapping in the $\pi$-conjugated system.

In some embodiments, a compound library is screened using at least one of the following characteristics.

1. Light emission around a specific wavelength
2. A triplet state over a calculated specific energy level
3. $\Delta E_{ST}$ value lower than a specific value
4. Quantum yield more than a specific value
5. HOMO level
6. LUMO level

In some embodiments, the difference ($\Delta E_{ST}$) between the lowest singlet excited state and the lowest triplet excited state at 77 K is less than about 0.5 eV, less than about 0.4 eV, less than about 0.3 eV, less than about 0.2 eV, or less than about 0.1 eV In some embodiments, $\Delta E_{ST}$ value is less than about 0.09 eV, less than about 0.08 eV, less than about 0.07 eV, less than about 0.06 eV, less than about 0.05 eV, less than about 0.04 eV, less than about 0.03 eV, less than about 0.02 eV, or less than about 0.01 eV

In some embodiments, the compound represented by the general formula (1) shows a quantum yield of more than 25%, for example, about 30%, about 35%, about 40%, about 45%, about 50%, about 55%, about 60%, about 65%, about 70%, about 75%, about 80%, about 85%, about 90%, about 95% or more.

[Method for Synthesizing Compound Represented by General Formula (1)]

The compound represented by the general formula (1) includes a novel compound.

The compound represented by the general formula (1) can be synthesized by combining known reactions. For example, a compound of the general formula (1) where one or two of $R^2$ to $R^4$ are halogen atoms is first synthesized, and then reacted with a substituted or unsubstituted carbazole different from $R^2$ to $R^4$ to give the compound of the general formula (1). For details of the reaction conditions, Synthesis Examples described later can be referred to.

[Structure Using Compound Represented by General Formula (1)]

In some embodiments, the compound represented by the general formula (1) is used along with one or more materials (e.g., small molecules, polymers, metals, metal complexes), by combining them, or by dispersing the compound, or by covalent-bonding with the compound, or by coating with the compound, or by carrying the compound, or by associating with the compound, and solid films or layers are formed. For example, by combining the compound represented by the general formula (1) with an electroactive material, a film can be formed. In some cases, the compound represented by the general formula (1) can be combined with a hole transporting polymer. In some cases, the compound represented by the general formula (1) can be combined with an electron transporting polymer. In some cases, the compound represented by the general formula (1) can be combined with a hole transporting polymer and an electron transporting polymer. In some cases, the compound represented by the general formula (1) can be combined with a copolymer having both a hole transporting moiety and an electron transporting moiety. In the embodiments mentioned above, the electrons and/or the holes formed in a solid film or layer can be interacted with the compound represented by the general formula (1).

[Film Formation]

In some embodiments, a film containing the compound represented by the general formula (1) can be formed in a wet process. In a wet process, a solution prepared by dissolving a composition containing the compound of the present invention is applied onto a surface, and then the solvent is removed to form a film. The wet process includes a spin coating method, a slit coating method, an inkjet method (a spraying method), a gravure printing method, an offset printing method and flexographic printing method, which, however are not limitative. In the wet process, an appropriate organic solvent capable of dissolving a composition containing the compound of the present invention is selected and

used. In some embodiments, a substituent (e.g., an alkyl group) capable of increasing the solubility in an organic solvent can be introduced into the compound contained in the composition.

In some embodiments, a film containing the compound of the present invention can be formed in a dry process. In some embodiments, a vacuum deposition method is employable as a dry process, which, however, is not limitative. In the case where a vacuum deposition method is employed, compounds to constitute a film can be vapor co-deposited from individual vapor deposition sources, or can be vapor co-deposited from a single vapor deposition source formed by mixing the compounds. In the case where a single vapor deposition source is used, a mixed powder prepared by mixing compound powders can be used, or a compression molded body prepared by compression-molding the mixed powder can be used, or a mixture prepared by heating and melting the compounds and cooling the resulting melt can be used. In some embodiments, by vapor co-deposition under the condition where the vapor deposition rate (weight reduction rate) of the plural compounds contained in a single vapor deposition source is the same or is nearly the same, a film having a compositional ratio corresponding to the compositional ratio of the plural compounds contained in the vapor deposition source can be formed. When plural compounds are mixed in the same compositional ratio as the compositional ratio of the film to be formed to prepare a vapor deposition source, a film having a desired compositional ratio can be formed in a simplified manner. In some embodiments, the temperature at which the compounds to be vapor co-deposited have the same weight reduction ratio is specifically defined, and the temperature can be employed as the temperature of vapor co-deposition.

[Use Examples of Compound Represented by General Formula (1)]

The compound represented by the general formula (1) is useful as a material for an organic light emitting device. In particular, the compound is preferably used for an organic light emitting diode or the like.

Organic Light Emitting Diode:

One embodiment of the present invention relates to use of the compound represented by the general formula (1) of the present invention as a light emitting material for organic light emitting devices. In some embodiments, the compound represented by the general formula (1) of the present invention can be effectively used as a light emitting material in a light emitting layer in an organic light emitting device. In some embodiments, the compound represented by the general formula (1) includes a delayed fluorescent material that emits delayed fluorescence. In some embodiments, the present invention provides a delayed fluorescent material having a structure represented by the general formula (1). In some embodiments, the present invention relates to use of the compound represented by the general formula (1) as a delayed fluorescent material. In some embodiments, the compound represented by the general formula (1) of the present invention can be used as a host material, and can be used along with one or more light emitting materials, and the light emitting material can be a fluorescent material, a phosphorescent material or a TADF. In some embodiments, the compound represented by the general formula (1) can be used as a hole transporting material. In some embodiments, the compound represented by the general formula (1) can be used as an electron transporting material. In some embodiments, the present invention relates to a method of generating delayed fluorescence from the compound represented by the general formula (1). In some embodiments, the organic light emitting device containing the compound as a light emitting material emits delayed fluorescence and shows a high light emission efficiency.

In some embodiments, the light emitting layer contains the compound represented by the general formula (1), and the compound represented by the general formula (1) is aligned in parallel to the substrate. In some embodiments, the substrate is a film-forming surface. In some embodiment, the alignment of the compound represented by the general formula (1) relative to the film-forming surface can have some influence on the propagation direction of light emitted by the aligned compounds, or can determine the direction. In some embodiments, by aligning the propagation direction of light emitted by the compound represented by the general formula (1), the light extraction efficiency from the light emitting layer can be improved.

One aspect of the present invention relates to an organic light emitting device. In some embodiments, the organic light emitting device includes a light emitting layer. In some embodiments, the light emitting layer contains, as a light emitting material, the compound represented by the general formula (1). In some embodiments, the organic light emitting device is an organic photoluminescent device (organic PL device). In some embodiments, the organic light emitting device is an organic electroluminescent device (organic EL device). In some embodiments, the compound represented by the general formula (1) assists light irradiation from the other light emitting materials contained in the light emitting layer (as a so-called assist dopant). In some embodiments, the compound represented by the general formula (1) contained in the light emitting layer is in a lowest excited singlet energy level, and is contained between the lowest excited single energy level of the host material contained in the light emitting layer and the lowest excited singlet energy level of the other light emitting materials contained in the light emitting layer.

In some embodiments, the organic photoluminescent device comprises at least one light emitting layer. In some

embodiments, the organic electroluminescent device includes at least an anode, a cathode, and an organic layer between the anode and the cathode. In some embodiments, the organic layer includes at least a light emitting layer. In some embodiments, the organic layer includes only a light emitting layer. In some embodiments, the organic layer includes one or more organic layers in addition to the light emitting layer. Examples of the organic layer include a hole transporting layer, a hole injection layer, an electron barrier layer, a hole barrier layer, an electron injection layer, an electron transporting layer and an exciton barrier layer. In some embodiments, the hole transporting layer can be a hole injection and transporting layer having a hole injection function, and the electron transporting layer can be an electron injection and transporting layer having an electron injection function.

Light Emitting Layer:

In some embodiments, the light emitting layer is a layer where holes and electrons injected from the anode and the cathode, respectively, are recombined to form excitons. In some embodiments, the layer emits light.

In some embodiments, only a light emitting material is used as the light emitting layer. In some embodiments, the light emitting layer contains a light emitting material and a host material. In some embodiments, the light emitting material is one or more compounds represented by the general formula (1). In some embodiments, for improving luminous radiation efficiency of an organic electroluminescent device and an organic photoluminescent device, the singlet exciton and the triplet exciton generated in a light emitting material are confined inside the light emitting material. In some embodiments, a host material is used in the light emitting layer in addition to a light emitting material. In some embodiments, the host material is an organic compound. In some embodiments, the organic compound has an excited singlet energy and an excited triplet energy, and at least one of them is higher than those in the light emitting material of the present invention. In some embodiments, the singlet exciton and the triplet exciton generated in the light emitting material of the present invention are confined in the molecules of the light emitting material of the present invention. In some embodiments, the singlet and triplet excitons are fully confined for improving luminous radiation efficiency. In some embodiments, although high luminous radiation efficiency is still attained, singlet excitons and triplet excitons are not fully confined, that is, a host material capable of attaining high luminous radiation efficiency can be used in the present invention with no specific limitation. In some embodiments, in the light emitting material in the light emitting layer of the device of the present invention, luminous radiation occurs. In some embodiments, radiated light includes both fluorescence and delayed fluorescence. In some embodiments, radiated light includes radiated light from a host material. In some embodiments, radiated light is composed of radiated light from a host material. In some embodiments, radiated light includes radiated light from the compound represented by the general formula (1) and radiated light from a host material. In some embodiment, a TADF molecule and a host material are used. In some embodiments, TADF is an assist dopant and has a lower excited singlet energy than the host material in the light emitting layer and a higher excited singlet energy than the light emitting material in the light emitting layer.

In the case where the compound represented by the general formula (1) is used as an assist dopant, various compounds can be employed as a light emitting material (preferably a fluorescent material). As such light emitting materials, employable are an anthracene derivative, a tetracene derivative, a naphthacene derivative, a pyrene derivative, a perylene derivative, a chrysene derivative, a rubrene derivative, a coumarin derivative, a pyran derivative, a stilbene derivative, a fluorene derivative, an anthryl derivative, a pyrromethene derivative, a terphenyl derivative, a terphenylene derivative, a fluoranthene derivative, an amine derivative, a quinacridone derivative, an oxadiazole derivative, a malononitrile derivative, a pyran derivative, a carbazole derivative, a julolidine derivative, a thiazole derivative, and a derivative having a metal (Al, Zn). These exemplified skeletons can have a substituent, or may not have a substituent. These exemplified skeletons can be combined.

Light emitting materials that can be used in combination with the assist dopant having a structure represented by the general formula (1) are shown below.

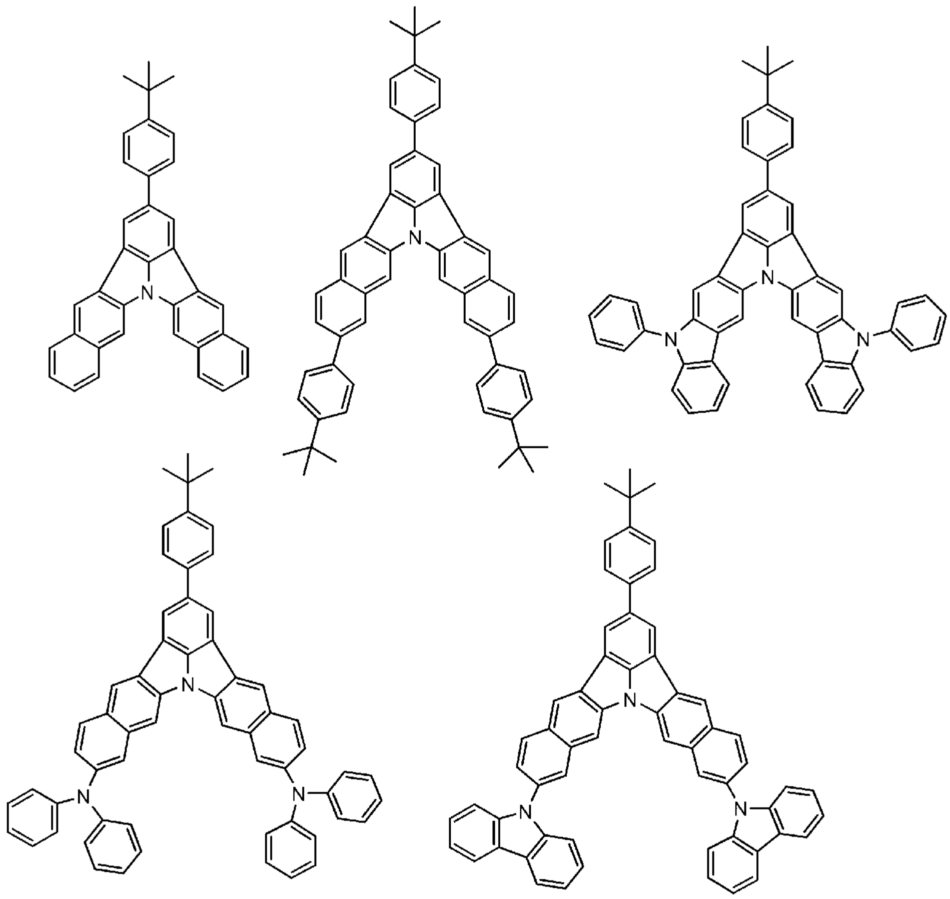

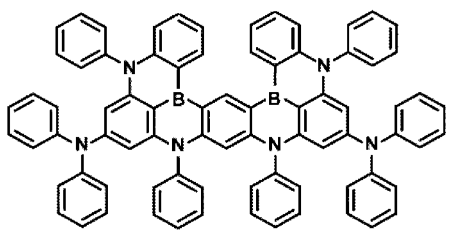

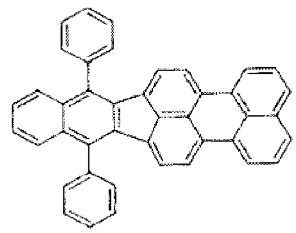

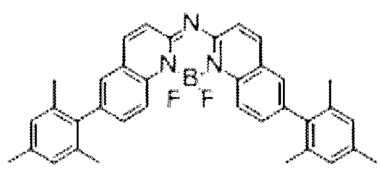

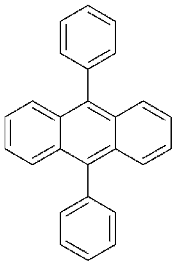
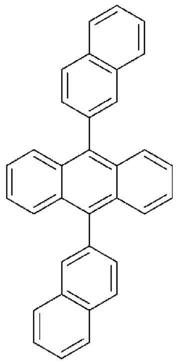
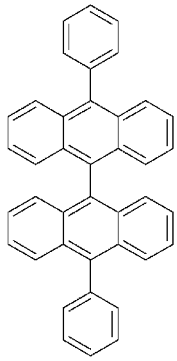
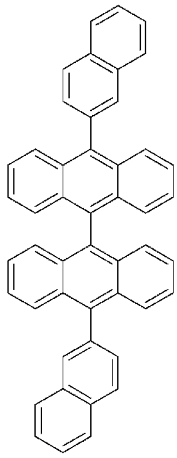

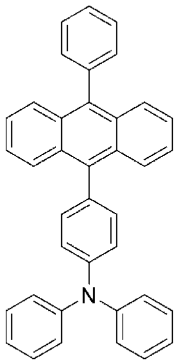
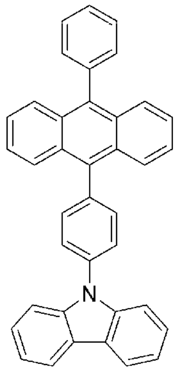
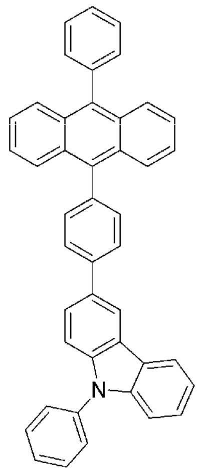
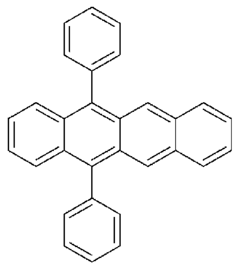

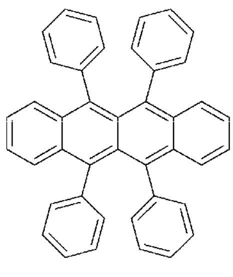
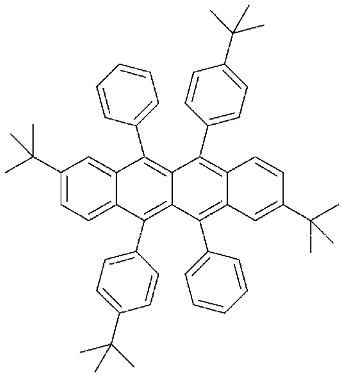
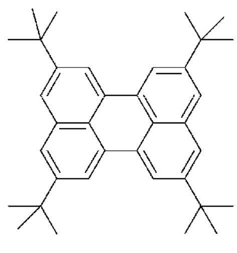

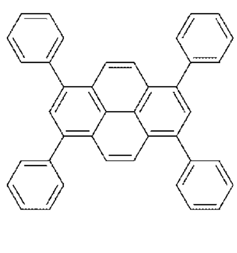
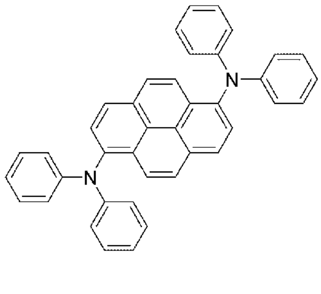
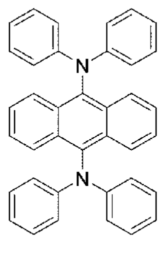

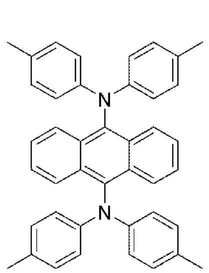

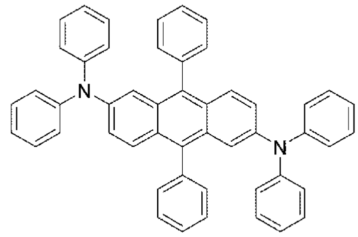

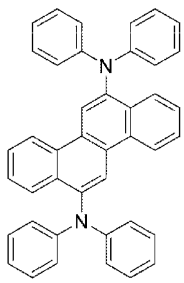

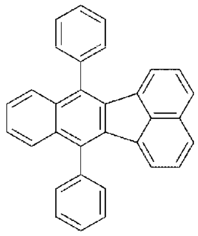

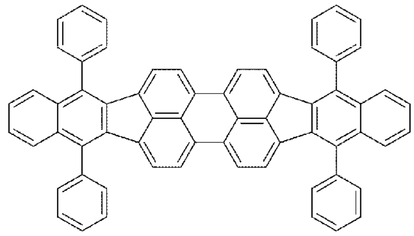

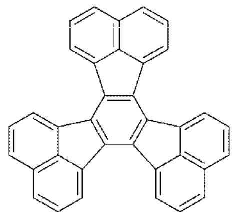

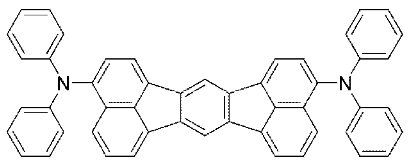

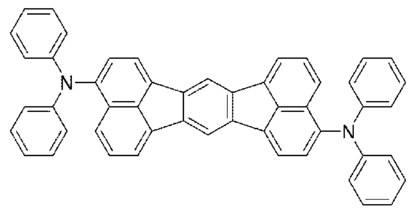

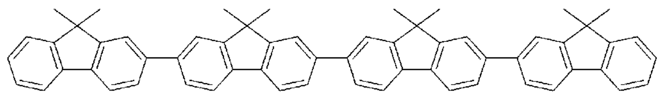

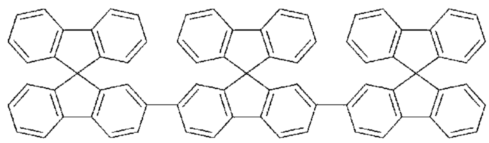

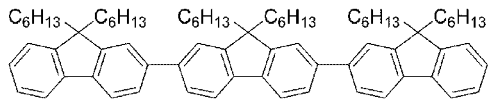

In addition, the compounds described in WO2015/022974, paragraphs 0220 to 0239 are also especially favorably employable as a light emitting material for use along with the assist dopant having a structure represented by the general formula (1).

Compounds represented by the following general formula (E1) are further preferred light emitting materials.

General Formula (E1)

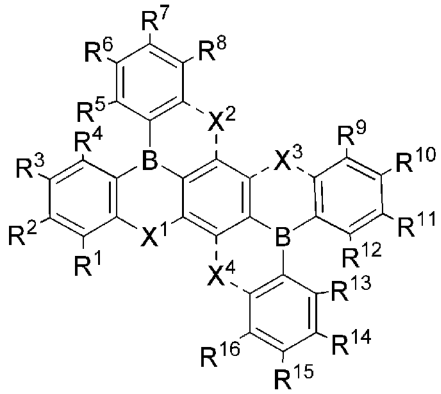

In the general formula (E1), $R^1$ and $R^3$ to $R^{16}$ each independently represent a hydrogen atom, a deuterium atom, or a substituent. $R^2$ represents an acceptor group, or $R^1$ and $R^2$ bond to each other to form an acceptor group, or $R^2$ and $R^3$ bond to each other to form an acceptor group. $R^3$ and $R^4$, $R^4$ and $R^5$, $R^5$ and $R^6$, $R^6$ and $R^7$, $R^7$ and $R^8$, $R^9$ and $R^{10}$, $R^{10}$ and $R^{11}$, $R^{11}$ and $R^{12}$, $R^{12}$ and $R^{13}$, $R^{13}$ and $R^{14}$, $R^{14}$ and $R^{15}$, and $R^{15}$ and $R^{16}$ each can bond to each other to form a cyclic structure. $X^1$ represents O or NR, and R represents a substituent. Of $X^2$ to $X^4$, at least one of $X^3$ and $X^4$ is O or NR, and the remainder may be O or R, or unlinked. When not linked, both ends each independently represent a hydrogen atom, a deuterium atom or a substituent. In the general formula (1), C-$R^1$, C-$R^3$, C-$R^4$, C-$R^5$, C-$R^6$, C-$R^7$, C-$R^8$, C-$R^9$, C-$R^{10}$, C-$R^{11}$, C-$R^{12}$, C-$R^{13}$, C-$R^{14}$, C-$R^{15}$, and C-$R^{16}$ can be substituted with N.

Compounds represented by the following general formula (E2) are further preferred light emitting materials.

General Formula (E2)

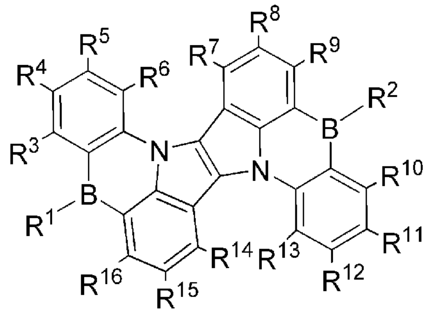

In the general formula (E2), $R^1$ and $R^2$ each independently represent a substituted or unsubstituted alkyl group, a substituted or unsubstituted aryl group, or a substituted or unsubstituted heteroaryl group, and $R^3$ to $R^{16}$ each independently represent a hydrogen atom, a deuterium atom or a substituent. $R^1$ and $R^3$, $R^3$ and $R^4$, $R^4$ and $R^5$, $R^5$ and $R^6$, $R^6$ and $R^7$, $R^7$ and $R^8$, $R^8$ and $R^9$, $R^9$ and $R^2$, $R^2$ and $R^{10}$, $R^{10}$ and $R^{11}$, $R^{11}$ and $R^{12}$, $R^{12}$ and $R^{13}$, $R^{13}$ and $R^{14}$, $R^{14}$ and $R^{15}$, $R^{15}$ and $R^{16}$, and $R^{16}$ and $R^1$ each can bond to each other to form a cyclic structure. In the general formula (1), C-$R^3$, C-$R^4$, C-$R^5$, C-$R^6$, C-$R^7$, C-$R^8$, C-$R^9$, C-$R^{10}$, C-$R^{11}$, C-$R^{12}$, C-$R^{13}$, C-$R^{14}$, C-$R^{15}$, and C-$R^{16}$ can be substituted with N.

Compounds represented by the following general formula (E3) are further preferred light emitting materials.

General Formula (E3)

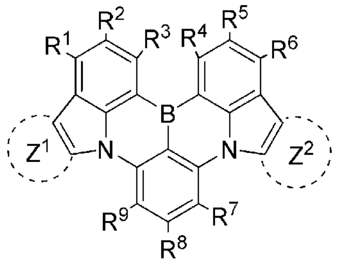

In the general formula (E3), $Z^1$ and $Z^2$ each independently represent a substituted or unsubstituted aromatic ring, or a substituted or unsubstituted heteroaromatic ring, $R^1$ to $R^9$ each independently represent a hydrogen atom, a deuterium atom or a substituent. $R^1$ and $R^2$, $R^2$ and $R^3$, $R^3$ and $R^4$, $R^4$ and $R^5$, $R^5$ and $R^6$, $R^7$ and $R^8$, and $R^8$ and $R^9$ each can bond to each other to form a cyclic structure. However, at least one of the ring formed by $Z^1$, $Z^2$, $R^1$ and $R^2$ bonding to each other, the ring formed by $R^2$ and $R^3$ bonding to each other, the ring formed by $R^4$ and $R^5$ bonding to each other, and the ring formed by $R^5$ and $R^6$ bonding to each other is a furan ring of a substituted or unsubstituted benzofuran, a thiophene ring of a substituted or unsubstituted benzothiophene, or a pyrrole ring of a substituted or unsubstituted indole, and at least one of $R^1$ to $R^9$ is a substituted or unsubstituted aryl group or an acceptor group, or at least one of $Z^1$ and $Z^2$ is a ring having an aryl group or an acceptor group as a substituent. Of the benzene ring skeleton-constituting carbon atoms to constitute the benzofuran ring, the benzothiophene ring, and the indole ring, a substitutable carbon atom can be substituted with a nitrogen atom. In the general formula (1), C-$R^1$, C-$R^2$, C-$R^3$, C-$R^4$, C-$R^5$, C-$R^6$, C-$R^7$, C-$R^8$, and C-$R^9$ can be substituted with N.

Compounds represented by the following general formula (E4) are further preferred light emitting materials.

General Formula (E4)

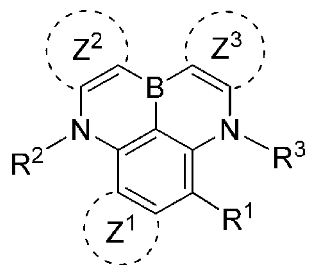

In the general formula (E4), $Z^1$ represents a furan ring fused with a substituted or unsubstituted benzene ring, a thiophene ring fused with a substituted or unsubstituted benzene ring, or an N-substituted pyrrole ring fused with a substituted or unsubstituted benzene ring, $Z^2$ and $Z^3$ each independently represent a substituted or unsubstituted aromatic ring, or a substituted or unsubstituted heteroaromatic ring, $R^1$ represents a hydrogen atom, a deuterium atom, or a substituent, and $R^2$ and $R^3$ each independently represent a substituted or unsubstituted aryl group, or a substituted or unsubstituted heteroaryl group. $Z^1$ and $R^1$, $R^2$ and $Z^2$, $Z^2$ and $Z^3$, and $Z^3$ and $R^3$ each can bond to each other to form a cyclic structure. However, at least one pair of $R^2$ and $Z^2$, $Z^2$ and $Z^3$, and $Z^3$ and $R^3$ bonds to each other to form a cyclic structure.

Compounds represented by the following general formula (E5) are further preferred light emitting materials.

General Formula (E5)

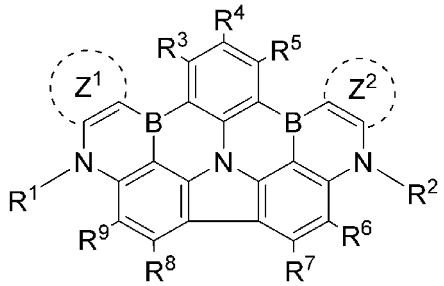

In the general formula (E5), $R^1$ and $R^2$ each independently represent a substituted or unsubstituted alkyl group, a substituted or unsubstituted aryl group, or a substituted or unsubstituted heteroaryl group, $Z^1$ and $Z^2$ each independently represent a substituted or unsubstituted aromatic ring, or a substituted or unsubstituted heteroaromatic ring, and $R^3$ to $R^9$ each independently represent a hydrogen atom, a deuterium atom or a substituent. However, at least one of $R^1$, $R^2$, $Z^1$ and $Z^2$ includes a substituted or unsubstituted benzofuran ring, a substituted or unsubstituted benzothiophene ring, or a substituted or unsubstituted indole ring. $R^1$ and $Z^1$, $Z^1$ and $R^3$, $R^3$ and $R^4$, $R^4$ and $R^5$, $R^5$ and $Z^2$, $Z^2$ and $R^2$, $R^2$ and $R^6$, $R^6$ and $R^7$, $R^7$ and $R^8$, $R^8$ and $R^9$, and $R^9$ and $R^1$ each can bond to each other to form a cyclic structure. Of the benzene ring skeleton-constituting carbon atoms to constitute the benzofuran ring, the benzothiophene ring, and the indole ring, a substitutable carbon atom can be substituted with a nitrogen atom. In the general formula (1), C-$R^3$, C-$R^4$, C-$R^5$, C-$R^6$, C-$R^7$, C-$R^8$, and C-$R^9$ can be substituted with N.

Compounds represented by the following general formula (E6) are further preferred light emitting materials.

General Formula (E6)

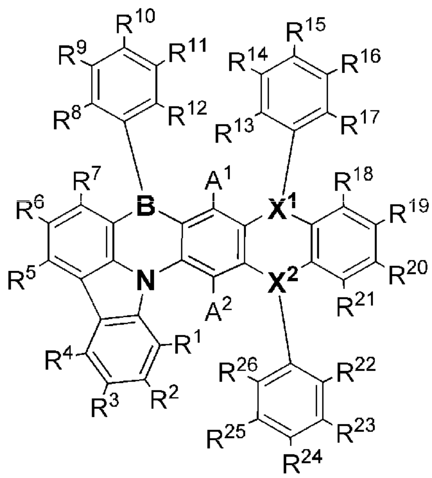

In the general formula (E6), one of $X^1$ and $X^2$ is a nitrogen atom, and the other is a boron atom. $R^1$ to $R^{26}$, $A^1$ and $A^2$ each independently represent a hydrogen atom, a deuterium atom, or a substituent. $R^1$ and $R^2$, $R^2$ and $R^3$, $R^3$ and $R^4$, $R^4$ and $R^5$, $R^5$ and $R^6$, $R^6$ and $R^7$, $R^7$ and $R^8$, $R^8$ and $R^9$, $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$ $R^{16}$ and $R^{17}$, $R^{17}$ and $R^{18}$, $R^{18}$ and $R^{19}$, $R^{19}$ and $R^{20}$, $R^{20}$ and $R^{21}$, $R^{21}$ and $R^{22}$ , $R^{22}$ and $R^{23}$, $R^{23}$ and $R^{24}$, $R^{24}$ and $R^{25}$, and $R^{25}$ and $R^{26}$ each can bond to each other to form a cyclic structure. However, when $X^1$ is a nitrogen atom, $R^{17}$ and $R^{18}$ bond to each other to be a single bond to form a pyrrole ring, and when $X^2$ is a nitrogen atom, $R^{21}$ and $R^{22}$ bond to each other to be a single bond to form a pyrrole ring. However, in the case where $X^1$ is a nitrogen atom, and where $R^7$ and $R^8$ and $R^{21}$ and $R^{22}$ each bond to each other via a nitrogen atom to form a 6-membered ring, and $R^{17}$ and $R^{18}$ bond to each other to form a single bond, at least one of $R^1$ to $R^6$ is a substituted or unsubstituted aryl group, or any of $R^1$ and $R^2$, $R^2$ and $R^3$, $R^3$ and $R^4$, $R^4$ and $R^5$, and $R^5$ and $R^6$ bond to each other to

form an aromatic ring or a heteroaromatic ring.

Compounds represented by the following general formula (E7) are further preferred light emitting materials.

General Formula (E7)

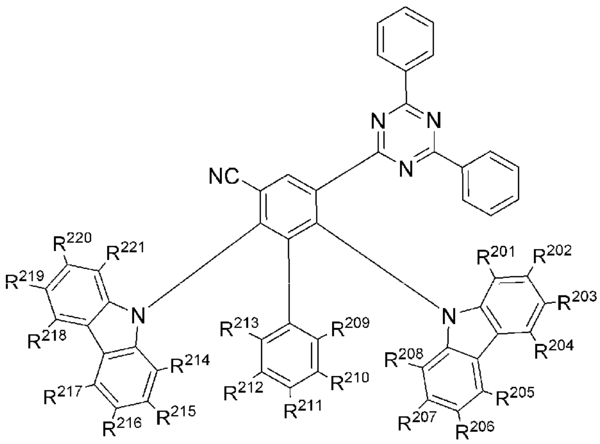

In the general formula (E7), $R^{201}$ to $R^{221}$ each independently represent a hydrogen atom, a deuterium atom or a substituent, preferably a hydrogen atom, a deuterium atom, an alkyl group, an aryl group, or a group formed by combining an alkyl group and an aryl group. At least one pair of $R^{201}$ and $R^{202}$, $R^{202}$ and $R^{203}$, $R^{203}$ and $R^{204}$, $R^{205}$ and $R^{206}$, $R^{206}$ and $R^{207}$ , $R^{207}$ and $R^{208}$, $R^{214}$ and $R^{215}$, $R^{215}$ and $R^{216}$, $R^{216}$ and $R^{217}$, $R^{218}$ and $R^{219}$, $R^{219}$ and $R^{220}$, and $R^{220}$ and $R^{221}$ each bond to each other to form a benzofuro structure or a benzothieno structure. Preferably, one or two pairs of $R^{201}$ and $R^{202}$, $R^{202}$ and $R^{203}$, $R^{203}$ and $R^{204}$, $R^{205}$ and $R^{206}$ , $R^{206}$ and $R^{207}$ and $R^{207}$ and $R^{208}$, and one or two pairs of $R^{214}$ and $R^{215}$, $R^{215}$ and $R^{216}$, $R^{216}$ and $R^{217}$, $R^{218}$ and $R^{219}$, $R^{219}$ and $R^{220}$ and $R^{220}$ and $R^{221}$ bond to each other to form a benzofuro structure or a benzothieno structure. Further preferably, $R^{203}$ and $R^{204}$ bond to each other to form a benzofuro structure or a benzothieno structure, even more preferably, $R^{203}$ and $R^{204}$, and $R^{216}$ and $R^{217}$ each bond to each other to form a benzofuro structure or a benzothieno structure. Especially preferably, $R^{203}$ and $R^{204}$, and $R^{216}$ and $R^{217}$ each bond to each other to form a benzofuro structure or a benzothieno structure, and $R^{206}$ and $R^{219}$ each represent a substituted or unsubstituted aryl group (preferably, a substituted or unsubstituted phenyl group, more preferably an unsubstituted phenyl group).

Further, compounds represented by the general formula (1) described in each specification of Japanese Patent Application Nos. 2021-103698, 2021-103699, 2021-103700, 2021-081332, 2021-103701, 2021-151805, and 2021-188860 can be used as a light emitting material. Descriptions of these general formulae (1) and specific compounds are hereby incorporated by reference as a part of this description.

In some embodiments where a host material is used, the amount of the compound of the present invention contained in a light emitting layer as a light emitting material is 0.1% by weight or more. In some embodiments where a host material is used, the amount of the compound of the present invention contained in a light emitting layer as a light emitting material is 1% by weight or more. In some embodiments where a host material is used, the amount of the compound of the present invention contained in a light emitting layer as a light emitting material is 50% by weight or less. In some embodiments where a host material is used, the amount of the compound of the present invention contained in a light emitting layer as a light emitting material is 20% by weight or less. In some embodiments where a host material is used, the amount of the compound of the present invention contained in a light emitting layer as a light emitting material is 10% by weight or less.

In some embodiments, the host material in a light emitting layer is an organic compound having a hole transporting capability and an electron transporting capability. In some embodiments, the host material in a light emitting layer is an organic compound that prevents increase in the wavelength of emitted light. In some embodiments, the host material in a light emitting layer is an organic compound having a high glass transition temperature.

In some embodiments, the host material is selected from the group consisting of the followings:

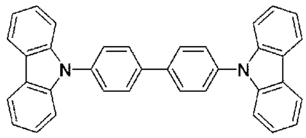
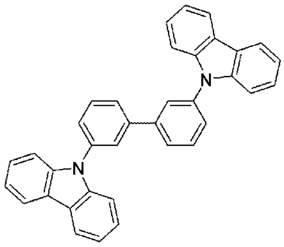
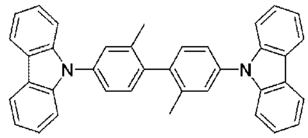

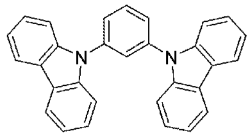
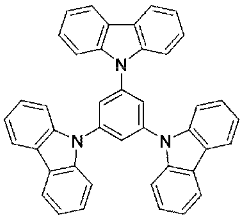
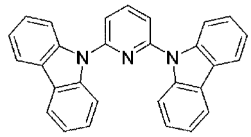

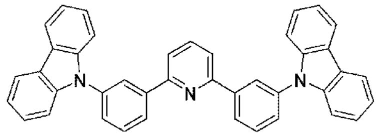
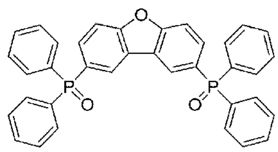

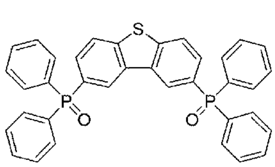
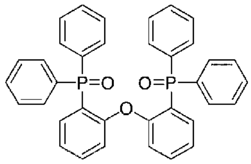
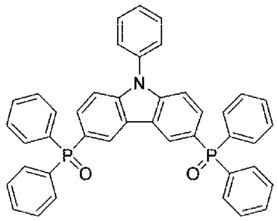

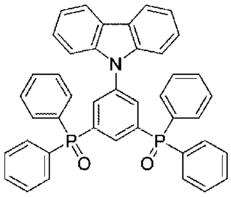
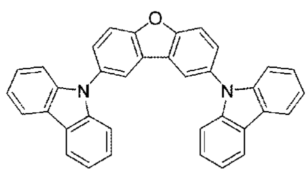
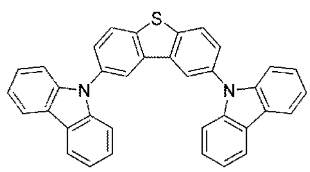

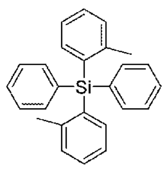
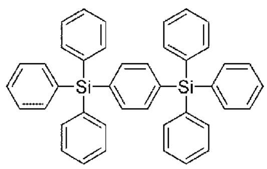
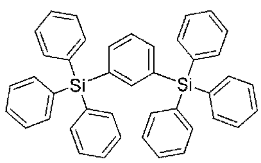
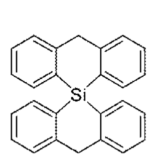

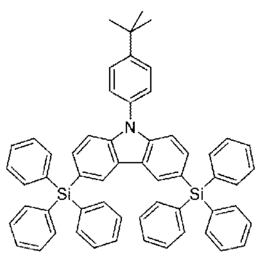
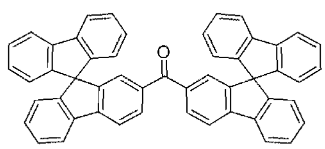
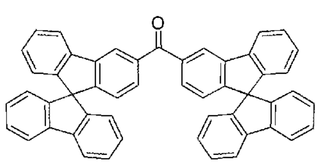

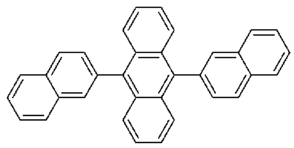
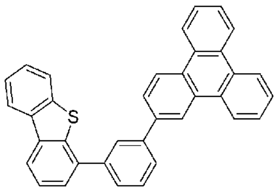
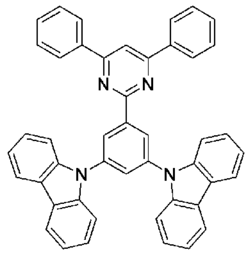

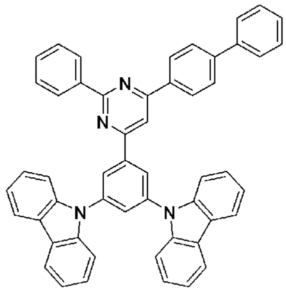
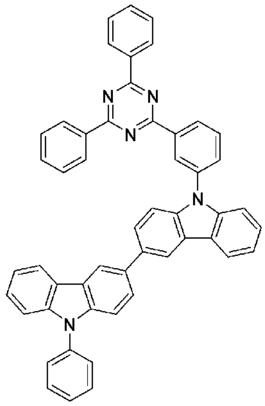
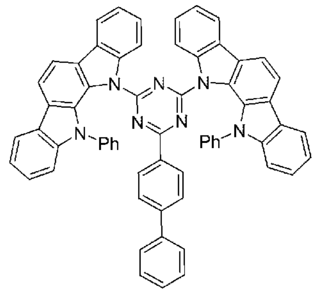

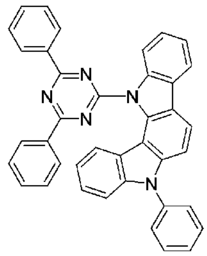
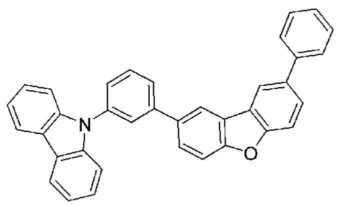
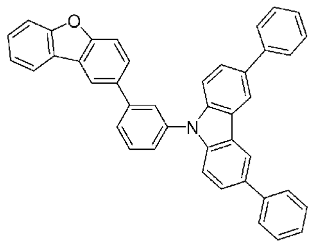

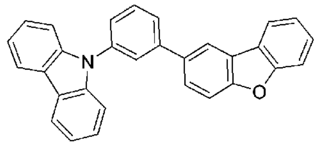
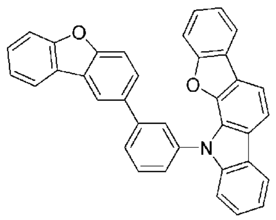
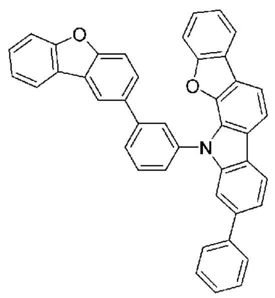

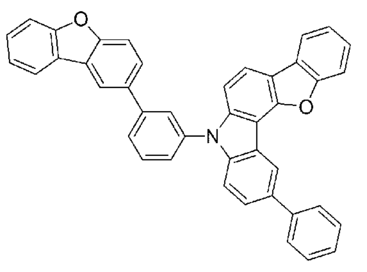
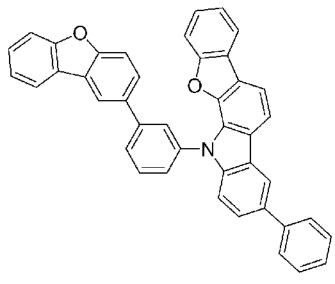

In some embodiments, the light emitting layer contains two or more kinds of TADF molecules differing in the structure. For example, the light emitting layer can contain three kinds of materials of a host material, a first TADF molecule and a second TADF molecule whose excited singlet energy level is higher in that order. In that case, both the

first TADF molecule and the second TADF molecule are preferably such that the difference $\Delta E_{ST}$ between the lowest excited singlet energy level and the lowest excited triplet energy level at 77 K is 0.3 eV or less, more preferably 0.25 eV or less, even more preferably 0.2 eV or less, further more preferably 0.15 eV or less, further more preferably 0.1 eV or less, further more preferably 0.07 eV or less, further more preferably 0.05 eV or less, further more preferably 0.03 eV or less, and particularly preferably 0.01 eV or less. The content of the first TADF molecule in the light emitting layer is preferably larger than the content of the second TADF molecule therein. The content of the host material in the light emitting layer is preferably larger than the content of the second TADF molecule therein. The content of the first TADF molecule in the light emitting layer can be larger than or can be smaller than or can be the same as the content of the host material therein. In some embodiments, the composition in the light emitting layer can be 10 to 70% by weight of a host material, 10 to 80% by weight of a first TADF molecule, and 0.1 to 30% by weighty of a second TADF molecule. In some embodiments, the composition in the light emitting layer can be 20 to 45% by weight of a host material, 50 to 75% by weight of a first TADF molecule, and 5 to 20% by weighty of a second TADF molecule. In some embodiments, the emission quantum yield φPL1(A) by photo-excitation of a vapor co-deposited film of a first TADF molecule and a host material (the content of the first TADF molecule in the vapor co-deposited film = A% by weight) and the emission quantum yield cpPL2(A) by photo-excitation of a vapor co-deposited film of a second TADF molecule and a host material (the content of the second TADF molecule in the vapor co-deposited film = A% by weight) satisfy a relational formula φPL1(A) > φPL2(A). In some embodiments, the emission quantum yield φPL2(B) by photo-excitation of a vapor co-deposited film of a second TADF molecule and a host material (the content of the second TADF molecule in the vapor co-deposited film = B% by weight) and the emission quantum yield cpPL2(100) by photo-excitation of a single film of a second TADF molecule satisfy a relational formula φPL2(B) > φPL2(100). In some embodiments, the light emitting layer can contain three kinds of TADF molecules differing in the structure. The compound of the present invention can be any of the plural TADF compounds contained in the light emitting layer.

In some embodiments, the light emitting layer can be composed of materials selected from the group consisting of a host material, an assist dopant and a light emitting material. In some embodiments, the light emitting layer does not contain a metal element. In some embodiments, the light emitting layer can be formed of a material composed of atoms alone selected from the group consisting of a carbon atom, a hydrogen atom, a deuterium atom, a nitrogen atom, an oxygen atom and a sulfur atom. Or the light emitting layer can be formed of a material composed of atoms alone selected from the group consisting of a carbon atom, a hydrogen atom, a deuterium atom, a nitrogen atom and an oxygen atom. Or the light emitting layer can be formed of a material composed of atoms alone selected from the group consisting of a carbon atom, a hydrogen atom, a nitrogen atom and an oxygen atom.

In the case where the light emitting layer contains any other TADF material than the compound of the present invention, the TADF material can be a known delayed fluorescent material. As preferred delayed fluorescent materials, there can be mentioned compounds included in the general formulae described in WO2013/154064, paragraphs 0008 to 0048 and 0095 to 0133; WO2013/011954, paragraphs 0007 to 0047 and 0073 to 0085; WO2013/011955, paragraphs 0007 to 0033 and 0059 to 0066; WO2013/081088, paragraphs 0008 to 0071 and 0118 to 0133; JP 2013-256490 A, paragraphs 0009 to 0046 and 0093 to 0134; JP 2013-116975 A, paragraphs 0008 to 0020 and 0038 to 0040; WO2013/133359, paragraphs 0007 to 0032 and 0079 to 0084; WO2013/161437, paragraphs 0008 to 0054 and 0101 to 0121; JP 2014-9352 A, paragraphs 0007 to 0041 and 0060 to 0069; JP 2014-9224 A, paragraphs 0008 to 0048 and 0067 to 0076; JP 2017-119663 A, paragraphs 0013 to 0025; JP 2017-119664 A, paragraphs 0013 to 0026; JP 2017-222623 A, paragraphs 0012 to 0025; JP 2017-226838 A, paragraphs 0010 to 0050; JP 2018-100411 A, paragraphs 0012 to 0043; WO2018/047853, paragraphs 0016 to 0044; and especially, exemplary compounds therein capable of emitting delayed fluorescence. In addition, also preferably employable here are light emitting materials capable of emitting delayed fluorescence, as described in JP 2013-253121 A, WO2013/133359, WO2014/034535, WO2014/115743, WO2014/122895, WO2014/126200, WO2014/136758, WO2014/133121, WO2014/136860, WO2014/196585, WO2014/189122, WO2014/168101, WO2015/008580, WO2014/203840, WO2015/002213, WO2015/016200, WO2015/019725, WO2015/072470, WO2015/108049, WO2015/080182, WO2015/072537, WO2015/080183, JP 2015-129240 A, WO2015/129714, WO2015/129715, WO2015/133501, WO2015/136880, WO2015/137244, WO2015/137202, WO2015/137136, WO2015/146541, and WO2015/159541. These patent publications described in this paragraph are hereby incorporated as a part of this description by reference.

In the following, the constituent members and the other layers than the light emitting layer of the organic electroluminescent device are described.

Substrate:

In some embodiments, the organic electroluminescent device of the invention is supported by a substrate, wherein the substrate is not particularly limited and can be any of those that have been commonly used in an organic electroluminescent device, for example those formed of glass, transparent plastics, quartz and silicon.

Anode:

In some embodiments, the anode of the organic electroluminescent device is made of a metal, an alloy, an electroconductive compound, or a combination thereof. In some embodiments, the metal, alloy, or electroconductive compound has a large work function (4 eV or more). In some embodiments, the metal is Au. In some embodiments, the electroconductive transparent material is selected from CuI, indium tin oxide (ITO), $SnO_2$, and ZnO. In some embodiments, an amorphous material capable of forming a transparent electroconductive film, such as IDIXO ($In_2O_3$-ZnO), is be used. In some embodiments, the anode is a thin film. In some embodiments, the thin film is made by vapor deposition or sputtering. In some embodiments, the film is patterned by a photolithography method. In some embodiments, where the pattern may not require high accuracy (for example, approximately 100 μm or more), the pattern can be formed with a mask having a desired shape on vapor deposition or sputtering of the electrode material. In some embodiments, when a material can be applied as a coating, such as an organic electroconductive compound, a wet film forming method, such as a printing method or a coating method is used. In some embodiments, when the emitted light goes through the anode, the anode has a transmittance of more than 10%, and the anode has a sheet resistance of several hundred Ohm per unit area or less. In some embodiments, the thickness of the anode is from 10 to 1,000 nm. In some embodiments, the thickness of the anode is from 10 to 200 nm. In some embodiments, the thickness of the anode varies depending on the material used.

Cathode:

In some embodiments, the cathode is made of an electrode material such as a metal having a small work function (4 eV or less) (referred to as an electron injection metal), an alloy, an electroconductive compound, or a combination thereof. In some embodiments, the electrode material is selected from sodium, a sodium-potassium alloy, magnesium, lithium, a magnesium-copper mixture, a magnesium-silver mixture, a magnesium-aluminum mixture, a magnesium-indium mixture, an aluminum-aluminum oxide ($Al_2O_3$) mixture, indium, a lithium-aluminum mixture, and a rare earth element. In some embodiments, a mixture of an electron injection metal and a second metal that is a stable metal having a larger work function than the electron injection metal is used. In some embodiments, the mixture is selected from a magnesium-silver mixture, a magnesium-aluminum mixture, a magnesium-indium mixture, an aluminum-aluminum oxide ($Al_2O_3$) mixture, a lithium-aluminum mixture, and aluminum. In some embodiments, the mixture increases the electron injection property and the durability against oxidation. In some embodiments, the cathode is produced by forming the electrode material into a thin film by vapor deposition or sputtering. In some embodiments, the cathode has a sheet resistance of several hundred Ohm per unit area or less. In some embodiments, the thickness of the cathode ranges from 10 nm to 5 μm. In some embodiments, the thickness of the cathode ranges from 50 to 200 nm. In some embodiments, for transmitting the emitted light, any one of the anode and the cathode of the organic electroluminescent device is transparent or translucent. In some embodiments, the transparent or translucent electroluminescent devices enhances the light emission luminance.

In some embodiments, the cathode is formed with an electroconductive transparent material, as described for the anode, to form a transparent or translucent cathode. In some embodiments, a device comprises an anode and a cathode, both being transparent or translucent.

Injection Layer:

An injection layer is a layer between the electrode and the organic layer. In some embodiments, the injection layer decreases the drive voltage and enhances the light emission luminance. In some embodiments, the injection layer includes a hole injection layer and an electron injection layer. The injection layer can be positioned between the anode and the light emitting layer or the hole transporting layer, and between the cathode and the light emitting layer or the electron transporting layer. In some embodiments, an injection layer is present. In some embodiments, no injection layer is present.

Preferred compound examples for use as a hole injection material are shown below.

$MoO_3$,

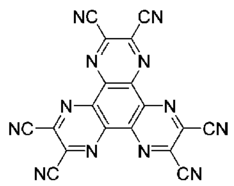 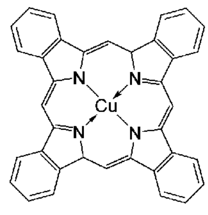 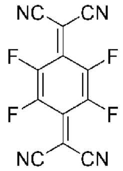 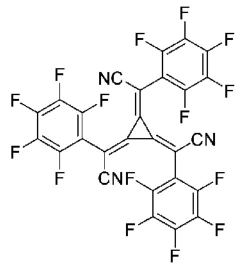

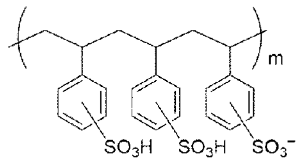 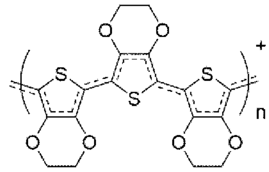

Next, preferred compound examples for use as an electron injection material are shown below.

LiF, CsF, 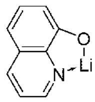

Barrier Layer:

A barrier layer is a layer capable of inhibiting charges (electrons or holes) and/or excitons present in the light emitting layer from being diffused outside the light emitting layer. In some embodiments, the electron barrier layer is between the light emitting layer and the hole transporting layer, and inhibits electrons from passing through the light emitting layer toward the hole transporting layer. In some embodiments, the hole barrier layer is between the light emitting layer and the electron transporting layer, and inhibits holes from passing through the light emitting layer toward the electron transporting layer. In some embodiments, the barrier layer inhibits excitons from being diffused outside the light emitting layer. In some embodiments, the electron barrier layer and the hole barrier layer are exciton barrier layers. As used herein, the term "electron barrier layer" or "exciton barrier layer" includes a layer that has both the function of an electron barrier layer and the function of an exciton barrier layer.

Hole Barrier Layer:

A hole barrier layer acts as an electron transporting layer. In some embodiments, the hole barrier layer inhibits holes from reaching the electron transporting layer while transporting electrons. In some embodiments, the hole barrier layer enhances the recombination probability of electrons and holes in the light emitting layer. The material used for the hole barrier layer can be the same materials as the ones described for the electron transporting layer.

Preferred compound examples for use for the hole barrier layer are shown below.

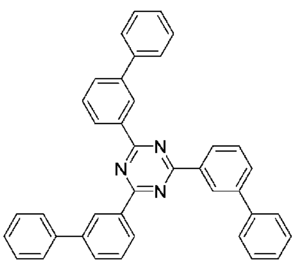 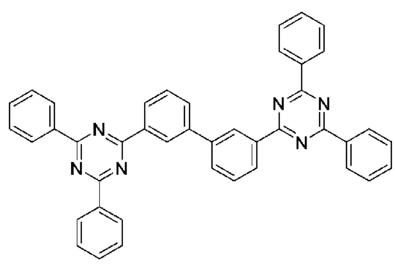 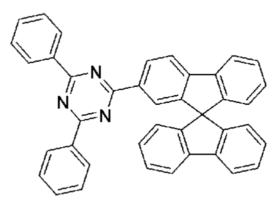

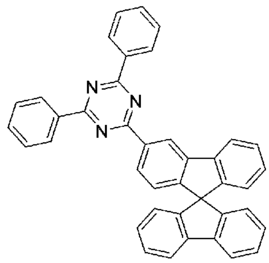
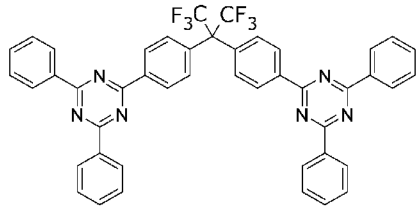
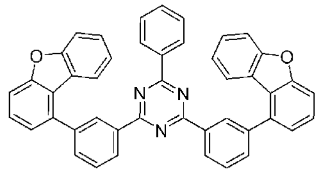

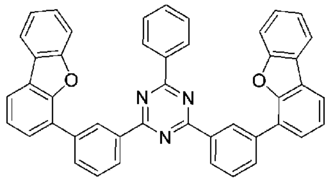
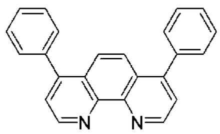
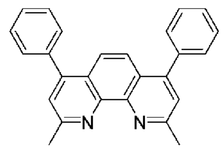

Electron Barrier Layer:

An electron barrier layer transports holes. In some embodiments, the electron barrier layer inhibits electrons from reaching the hole transporting layer while transporting holes. In some embodiments, the electron barrier layer enhances the recombination probability of electrons and holes in the light emitting layer. The material used for the electron barrier layer can be the same material as the ones described above for the hole transporting layer.

Preferred compound examples for use as the electron barrier material are shown below.

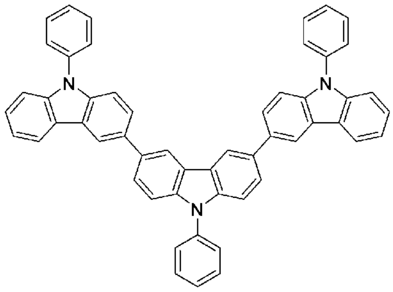
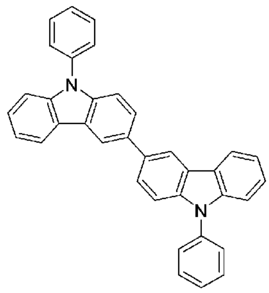
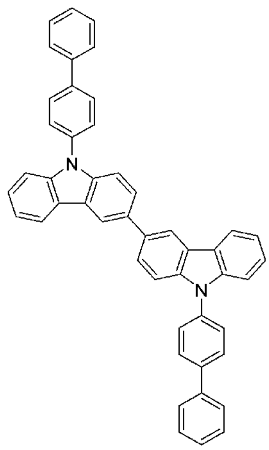

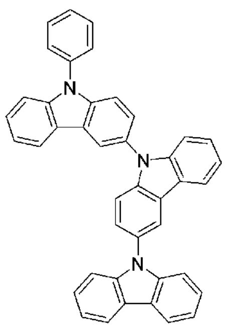
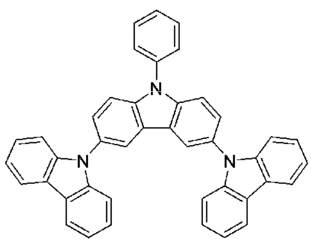
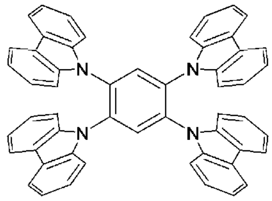

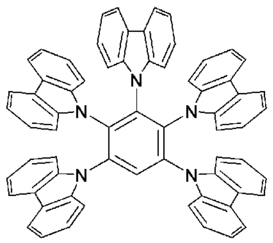
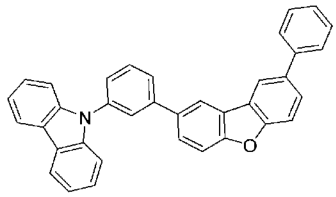
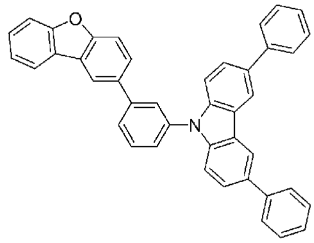

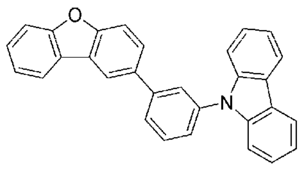
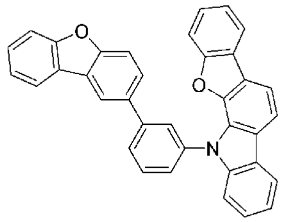
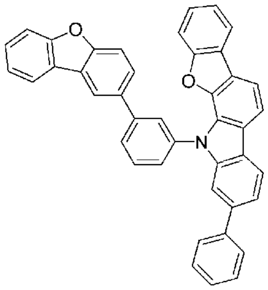

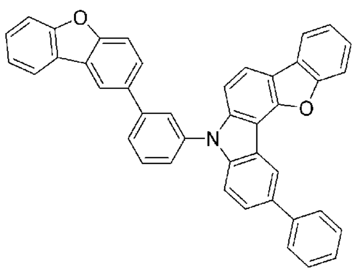
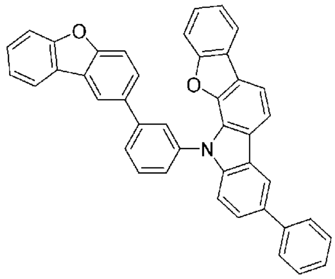

Exciton Barrier Layer:

An exciton barrier layer inhibits excitons generated through recombination of holes and electrons in the light emitting layer from being diffused to the charge transporting layer. In some embodiments, the exciton barrier layer enables effective confinement of excitons in the light emitting layer. In some embodiments, the light emission efficiency of the device is enhanced. In some embodiments, the exciton barrier layer is adjacent to the light emitting layer on any of the side of the anode and the side of the cathode, and on both the sides. In some embodiments, where the exciton barrier layer is on the side of the anode, the layer can be between the hole transporting layer and the light emitting layer and adjacent to the light emitting layer. In some embodiments, where the exciton barrier layer is on the side of the cathode, the layer can be between the light emitting layer and the cathode and adjacent to the light emitting layer. In some embodiments, a hole injection layer, an electron barrier layer, or a similar layer is between the anode and the exciton barrier layer that is adjacent to the light emitting layer on the side of the anode. In some embodiments, a hole injection layer, an electron barrier layer, a hole barrier layer, or a similar layer is between the cathode and the exciton barrier layer that is adjacent to the light emitting layer on the side of the cathode. In some embodiments, the exciton barrier layer comprises excited singlet energy and excited triplet energy, at least one of which is higher than the excited singlet energy and the excited triplet energy of the light emitting material, respectively.

Hole Transporting Layer:

The hole transporting layer comprises a hole transporting material. In some embodiments, the hole transporting layer is a single layer. In some embodiments, the hole transporting layer comprises a plurality of layers.

In some embodiments, the hole transporting material has one of injection or transporting property of holes and barrier property of electrons. In some embodiments, the hole transporting material is an organic material. In some embodiments, the hole transporting material is an inorganic material. Examples of known hole transporting materials that can be used in the present invention include but are not limited to a triazole derivative, an oxadiazole derivative, an imidazole derivative, a carbazole derivative, an indolocarbazole derivative, a polyarylalkane derivative, a pyrazoline derivative, a pyrazolone derivative, a phenylenediamine derivative, an allylamine derivative, an amino-substituted chalcone derivative, an oxazole derivative, a styrylanthracene derivative, a fluorenone derivative, a hydrazone derivative, a

stilbene derivative, a silazane derivative, an aniline copolymer and an electroconductive polymer oligomer (particularly, a thiophene oligomer), or a combination thereof. In some embodiments, the hole transporting material is selected from a porphyrin compound, an aromatic tertiary amine compound, and a styrylamine compound. In some embodiments, the hole transporting material is an aromatic tertiary amine compound. Preferred compound examples for use as the hole transporting material are shown below.

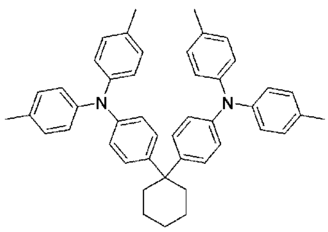
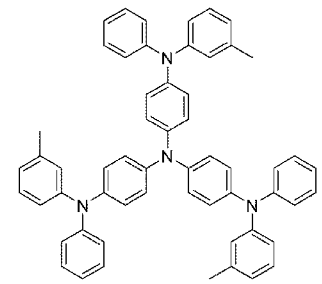
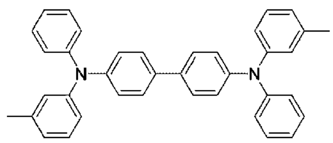
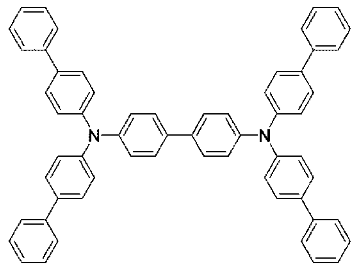
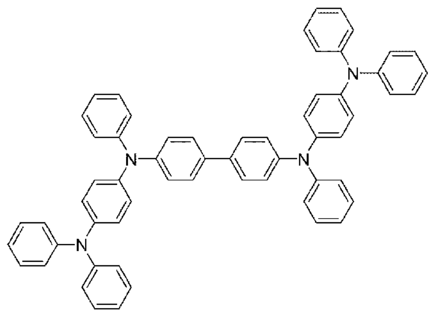
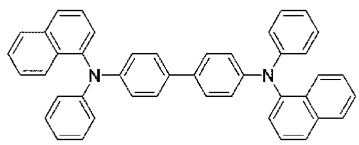
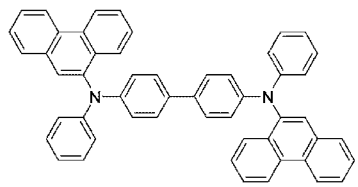
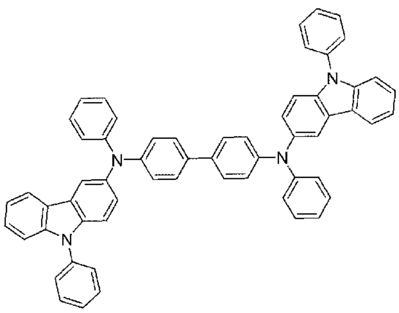
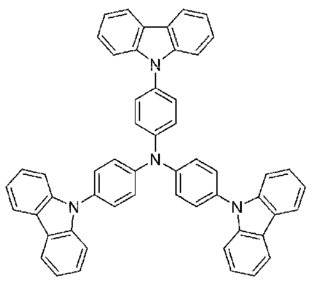
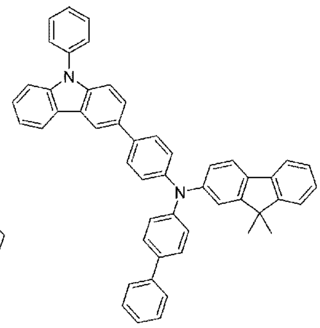
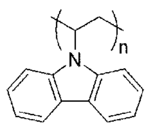
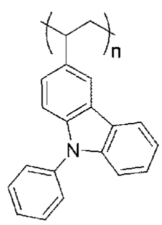
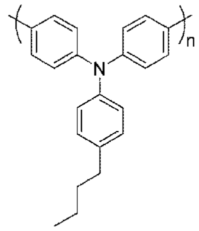

Electron Transporting Layer:

The electron transporting layer comprises an electron transporting material. In some embodiments, the electron

transporting layer is a single layer. In some embodiments, the electron transporting layer comprises a plurality of layers.

In some embodiments, the electron transporting material needs only to have a function of transporting electrons, which are injected from the cathode, to the light emitting layer. In some embodiments, the electron transporting material also function as a hole barrier material. Examples of the electron transporting layer that can be used in the present invention include but are not limited to a nitro-substituted fluorene derivative, a diphenylquinone derivative, a thiopyran dioxide derivative, carbodiimide, a fluorenylidene methane derivative, anthraquinodimethane, an anthrone derivatives, an oxadiazole derivative, an azole derivative, an azine derivative, or a combination thereof, or a polymer thereof. In some embodiments, the electron transporting material is a thiadiazole derivative, or a quinoxaline derivative. In some embodiments, the electron transporting material is a polymer material. Preferred compound examples for use as the electron transporting material are shown below.

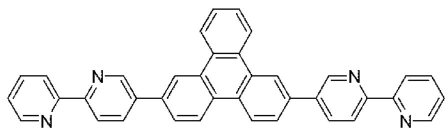
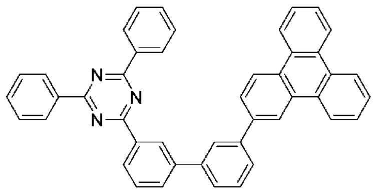

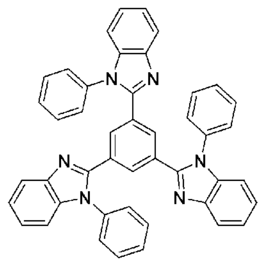
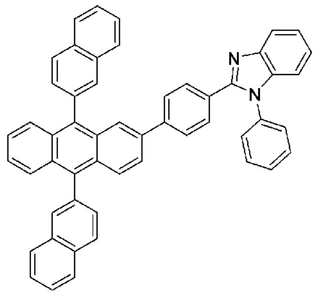
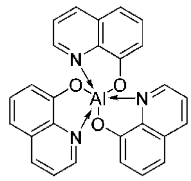

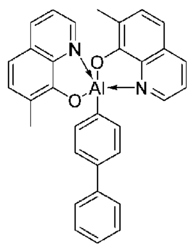
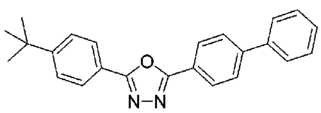
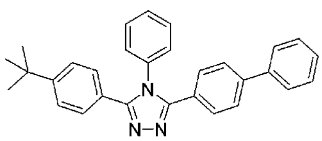

Hereinunder, compound examples preferred as a material that can be added to the organic layers are shown. For example, it is conceivable to add these as a stabilization material.

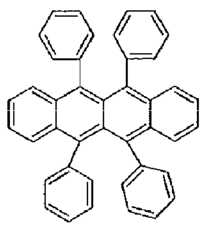
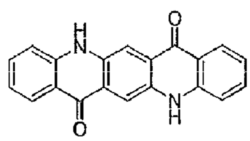
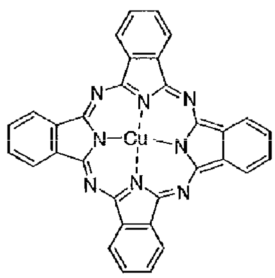

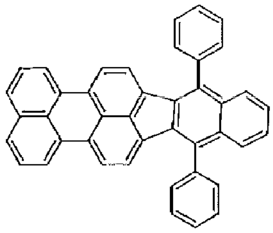 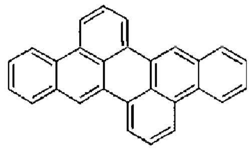

Preferred materials for use in the organic electroluminescent device are specifically shown. However, the materials usable in the invention should not be limitatively interpreted by the following exemplary compounds. Compounds that are exemplified as materials having a specific function can also be used as materials having any other function.

Devices:

In some embodiments, an light emitting layer is incorporated into a device. For example, the device includes, but is not limited to an OLED bulb, an OLED lamp, a television screen, a computer monitor, a mobile phone, and a tablet.

In some embodiments, an electronic device includes an OLED comprising an anode, a cathode, and at least one organic layer comprising a light emitting layer between the anode and the cathode.

In some embodiments, compositions described herein can be incorporated into various light-sensitive or light-activated devices, such as OLEDs or opto-electronic devices. In some embodiments, the composition can be useful in facilitating charge transfer or energy transfer within a device and/or as a hole-transport material. The device can be, for example, an organic light emitting diode (OLED), an organic integrated circuit (O-IC), an organic field-effect transistor (O-FET), an organic thin-film transistor (O-TFT), an organic light emitting transistor (O-LET), an organic solar cell (O-SC), an organic optical detector, an organic photoreceptor, an organic field-quench device (O-FQD), a light emitting electrochemical cell (LEC) or an organic laser diode (O-laser).

Bulbs or Lamps:

In some embodiments, an electronic device includes an OLED comprising an anode, a cathode, and at least one organic layer comprising a light emitting layer between the anode and the cathode.

In some embodiments, a device comprises OLEDs that differ in color. In some embodiments, a device comprises an array comprising a combination of OLEDs. In some embodiments, the combination of OLEDs is a combination of three colors (e.g., RGB). In some embodiments, the combination of OLEDs is a combination of colors that are not red, green, or blue (for example, orange and yellow green). In some embodiments, the combination of OLEDs is a combination of two, four, or more colors.

In some embodiments, a device is an OLED light comprising:

a circuit board having a first surface with a mounting surface and an opposing second surface, and defining at least one opening;
at least one OLED on the mounting surface, the at least one OLED configured to emanate light, the OLED comprising: an anode, a cathode, and at least one organic layer comprising a light emitting layer between the anode and the cathode;
a housing for the circuit board; and
at least one connector arranged at an end of the housing, the housing and the connector defining a package adapted for installation in a light fixture.

In some embodiments, the OLED light comprises a plurality of OLEDs mounted on a circuit board such that light emanates in a plurality of directions. In some embodiments, a portion of the light emanated in a first direction is deflected to emanate in a second direction. In some embodiments, a reflector is used to deflect the light emanated in a first direction.

Displays or Screens:

In some embodiments, the light emitting layer of the invention can be used in a screen or a display. In some embodiments, the compounds of the invention are deposited onto a substrate using a process including, but not limited to, vacuum evaporation, deposition, vapor deposition, or chemical vapor deposition (CVD). In some embodiments, the substrate is a photoplate structure useful in a two-sided etching that provides a unique aspect ratio pixel. The screen

(which can also be referred to as a mask) is used in a process in the manufacturing of OLED displays. The corresponding artwork pattern design facilitates a very steep and narrow tie-bar between the pixels in the vertical direction and a large, sweeping bevel opening in the horizontal direction. This allows the fine patterning of pixels needed for high resolution displays while optimizing the chemical deposition onto a TFT backplane.

The internal patterning of the pixel allows the construction of a three-dimensional pixel opening with varying aspect ratios in the horizontal and vertical directions. Additionally, the use of imaged "stripes" or halftone circles within the pixel area inhibits etching in specific areas until these specific patterns are undercut and fall off the substrate. At that point the entire pixel area is subjected to a similar etching rate but the depths are varying depending on the halftone pattern. Varying the size and spacing of the halftone pattern allow etching to be inhibited at different rates within the pixel and allow a localized deeper etch needed to create steep vertical bevels.

A preferred material for the deposition mask is invar. Invar is a metal alloy that is cold rolled into a long thin sheet in a steel mill. Invar cannot be electrodeposited onto a rotating mandrel as the nickel mask. A preferred and more cost feasible method for forming the opening areas in the mask used for deposition is through a wet chemical etching.

In some embodiments, a screen or display pattern is a pixel matrix on a substrate. In some embodiments, a screen or display pattern is fabricated using lithography (e.g., photolithography and e-beam lithography). In some embodiments, a screen or display pattern is fabricated using a wet chemical etching. In further embodiments, a screen or display pattern is fabricated using plasma etching.

Methods of Manufacturing Devices:

An OLED display is generally manufactured by forming a large mother panel and then cutting the mother panel in units of cell panels. In general, each of the cell panels on the mother panel is formed by forming a thin film transistor (TFT) including an active layer and a source/drain electrode on a base substrate, applying a planarization film to the TFT, and sequentially forming a pixel electrode, a light emitting layer, a counter electrode, and an encapsulation layer, and then is cut from the mother panel.

An OLED display is generally manufactured by forming a large mother panel and then cutting the mother panel in units of cell panels. In general, each of the cell panels on the mother panel is formed by forming a thin film transistor (TFT) including an active layer and a source/drain electrode on a base substrate, applying a planarization film to the TFT, and sequentially forming a pixel electrode, a light emitting layer, a counter electrode, and an encapsulation layer, and then is cut from the mother panel.

In another aspect, provided herein is a method of manufacturing an organic light emitting diode (OLED) display, the method comprising:

forming a barrier layer on a base substrate of a mother panel;
forming a plurality of display units in units of cell panels on the barrier layer;
forming an encapsulation layer on each of the display units of the cell panels;
applying an organic film to an interface portion between the cell panels.

In some embodiments, the barrier layer is an inorganic film formed of, for example, SiNx, and an edge portion of the barrier layer is covered with an organic film formed of polyimide or acryl. In some embodiments, the organic film helps the mother panel to be softly cut in units of the cell panel.

In some embodiments, the thin film transistor (TFT) layer includes a light emitting layer, a gate electrode, and a source/drain electrode. Each of the plurality of display units may include a thin film transistor (TFT) layer, a planarization film formed on the TFT layer, and a light emitting unit formed on the planarization film, wherein the organic film applied to the interface portion is formed of a same material as a material of the planarization film and is formed at a same time as the planarization film is formed. In some embodiments, a light emitting unit is connected to the TFT layer with a passivation layer and a planarization film therebetween and an encapsulation layer that covers and protects the light emitting unit. In some embodiments of the method of manufacturing, the organic film contacts neither the display units nor the encapsulation layer.

Each of the organic film and the planarization film can include any one of polyimide and acryl. In some embodiments, the barrier layer can be an inorganic film. In some embodiments, the base substrate can be formed of polyimide. The method can further include, before the forming of the barrier layer on one surface of the base substrate formed of polyimide, attaching a carrier substrate formed of a glass material to another surface of the base substrate, and before the cutting along the interface portion, separating the carrier substrate from the base substrate. In some embodiments, the OLED display is a flexible display.

In some embodiments, the passivation layer is an organic film disposed on the TFT layer to cover the TFT layer. In some embodiments, the planarization film is an organic film formed on the passivation layer. In some embodiments, the planarization film is formed of polyimide or acryl, like the organic film formed on the edge portion of the barrier

layer. In some embodiments, the planarization film and the organic film are simultaneously formed when the OLED display is manufactured. In some embodiments, the organic film can be formed on the edge portion of the barrier layer such that a portion of the organic film directly contacts the base substrate and a remaining portion of the organic film contacts the barrier layer while surrounding the edge portion of the barrier layer.

In some embodiments, the light emitting layer includes a pixel electrode, a counter electrode, and an organic light emitting layer disposed between the pixel electrode and the counter electrode. In some embodiments, the pixel electrode is connected to the source/drain electrode of the TFT layer.

In some embodiments, when a voltage is applied to the pixel electrode through the TFT layer, an appropriate voltage is formed between the pixel electrode and the counter electrode, and thus the organic light emitting layer emits light, thereby forming an image. Hereinafter, an image forming unit including the TFT layer and the light emitting unit is referred to as a display unit.

In some embodiments, the encapsulation layer that covers the display unit and prevents penetration of external moisture can be formed to have a thin film encapsulation structure in which an organic film and an inorganic film are alternately stacked. In some embodiments, the encapsulation layer has a thin film encapsulation structure in which a plurality of thin films are stacked. In some embodiments, the organic film applied to the interface portion is spaced apart from each of the plurality of display units. In some embodiments, the organic film is formed such that a portion of the organic film directly contacts the base substrate and a remaining portion of the organic film contacts the barrier layer while surrounding an edge portion of the barrier layer.

In one embodiment, the OLED display is flexible and uses the soft base substrate formed of polyimide. In some embodiments, the base substrate is formed on a carrier substrate formed of a glass material, and then the carrier substrate is separated.

In some embodiments, the barrier layer is formed on a surface of the base substrate opposite to the carrier substrate. In one embodiment, the barrier layer is patterned according to a size of each of the cell panels. For example, while the base substrate is formed over the entire surface of a mother panel, the barrier layer is formed according to a size of each of the cell panels, and thus a groove is formed at an interface portion between the barrier layers of the cell panels. Each of the cell panels can be cut along the groove.

In some embodiments, the method of manufacture further comprises cutting along the interface portion, wherein a groove is formed in the barrier layer, wherein at least a portion of the organic film is formed in the groove, and wherein the groove does not penetrate into the base substrate. In some embodiments, the TFT layer of each of the cell panels is formed, and the passivation layer which is an inorganic film and the planarization film which is an organic film are disposed on the TFT layer to cover the TFT layer. At the same time as the planarization film formed of, for example, polyimide or acryl is formed, the groove at the interface portion is covered with the organic film formed of, for example, polyimide or acryl. This is to prevent cracks from occurring by allowing the organic film to absorb an impact generated when each of the cell panels is cut along the groove at the interface portion. That is, if the entire barrier layer is entirely exposed without the organic film, an impact generated when each of the cell panels is cut along the groove at the interface portion is transferred to the barrier layer, thereby increasing the risk of cracks. However, in one embodiment, since the groove at the interface portion between the barrier layers is covered with the organic film and the organic film absorbs an impact that would otherwise be transferred to the barrier layer, each of the cell panels can be softly cut and cracks can be prevented from occurring in the barrier layer. In one embodiment, the organic film covering the groove at the interface portion and the planarization film are spaced apart from each other. For example, if the organic film and the planarization film are connected to each other as one layer, since external moisture may penetrate into the display unit through the planarization film and a portion where the organic film remains, the organic film and the planarization film are spaced apart from each other such that the organic film is spaced apart from the display unit.

In some embodiments, the display unit is formed by forming the light emitting unit, and the encapsulation layer is disposed on the display unit to cover the display unit. As such, once the mother panel is completely manufactured, the carrier substrate that supports the base substrate is separated from the base substrate. In some embodiments, when a laser beam is emitted toward the carrier substrate, the carrier substrate is separated from the base substrate due to a difference in a thermal expansion coefficient between the carrier substrate and the base substrate.

In some embodiments, the mother panel is cut in units of the cell panels. In some embodiments, the mother panel is cut along an interface portion between the cell panels by using a cutter. In some embodiments, since the groove at the interface portion along which the mother panel is cut is covered with the organic film, the organic film absorbs an impact during the cutting. In some embodiments, cracks can be prevented from occurring in the barrier layer during the cutting.

In some embodiments, the methods reduce a defect rate of a product and stabilize its quality.

Another aspect is an OLED display including: a barrier layer that is formed on a base substrate; a display unit that is formed on the barrier layer; an encapsulation layer that is formed on the display unit; and an organic film that is applied to an edge portion of the barrier layer.

Examples

The features of the present invention will be described more specifically with reference to Synthesis Examples and Examples given below. The materials, processes, procedures and the like shown below can be appropriately modified unless they deviate from the substance of the invention. Accordingly, the scope of the invention is not construed as being limited to the specific examples shown below. Hereunder, the light emission characteristics were evaluated using a source meter (available from Keithley Instruments, Inc.: 2400 series), a semiconductor parameter analyzer (available from Agilent Technologies, Inc., E5273A), an optical power meter device (available from Newport Corporation, 1930C), an optical spectroscope (available from Ocean Optics Corporation, USB2000), a spectroradiometer (available from Topcon Corporation, SR-3), and a streak camera (available from Hamamatsu Photonics K.K., Model C4334). The energies of HOMO and LUMO were measured by atmospheric photoelectron spectroscopy (such as AC-3 manufactured by Riken Keiki Co., Ltd.).

In the following Synthesis Examples, compounds included in the general formula (1) were synthesized.

(Synthesis Example 1) Synthesis of Compound 1

Compound b

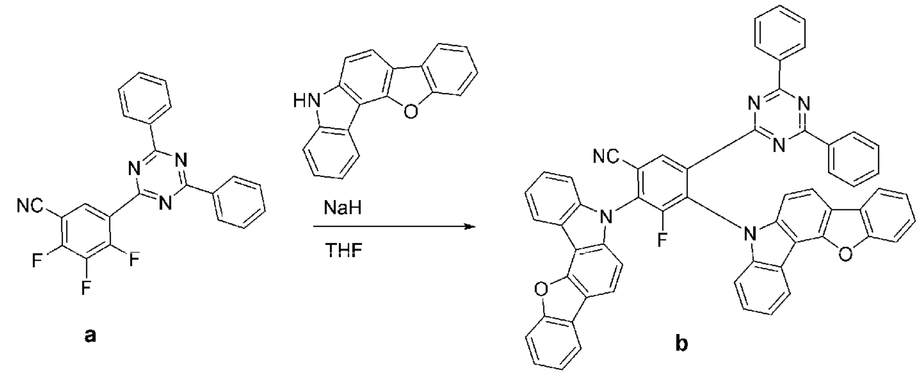


a

b

A tetrahydrofuran solution (25 mL) of 5H-benzofuro[3,2-c]carbazole (2.0 g, 7.7 mmol) and sodium hydride (0.3 g, 7.9 mmol) was added to a tetrahydrofuran solution (100 mL) of Compound a (1.5 g, 3.9 mmol) at -10°C under a nitrogen stream, and stirred for 2 hours. The mixture was restored to room temperature, then the reaction was stopped with an aqueous ammonium chloride solution, and ethyl acetate was added thereto. The organic layer was separated by liquid-liquid separation, and dried with magnesium sulfate. After solvent concentration, the residue was purified by silica gel column chromatography (toluene/hexane = 2/8) and reprecipitation (toluene/methanol) to give Compound b (2.8 g, 3.2 mmol, yield 84%).

$^1$H NMR (400 MHz, $CDCl_3$) δ 8.97 (d, J = 1.6 Hz, 1H), 8.63 (d, J = 7.6 Hz, 1H), 8.42 (d, J = 7.6 Hz, 1H), 8.12-8.10 (m, 1H), 8.03-7.94 (m, 7H), 7.77 (d, J = 8.0, 1H), 7.68 (d,J = 8.0 Hz, 1H), 7.68-7.60 (m, 1H), 7.57-7.31 (m, 13H), 7.26-7.22 (m, 4H).

ASAP Mass Spectrometry: theoretical value 862.25, observed value 863.35

Compound c

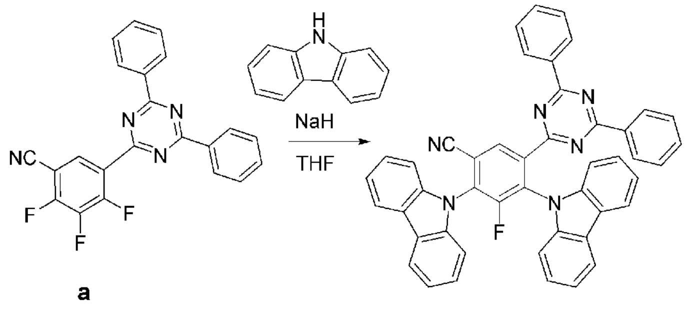


a

c

A tetrahydrofuran solution (25 mL) of carbazole (1.3 g, 7.7 mmol) and sodium hydride (0.3 g, 7.9 mmol) was added to a tetrahydrofuran solution (100 mL) of Compound a (1.5 g, 3.9 mmol) at -10°C under a nitrogen stream, and stirred for 2 hours. The mixture was restored to room temperature, then the reaction was stopped with an aqueous ammonium chloride solution, and ethyl acetate was added thereto. The organic layer was separated by liquid-liquid separation, and dried with magnesium sulfate. After solvent concentration, the residue was purified by silica gel column chromatography (toluene/hexane = 2/8) and reprecipitation (toluene/methanol) to give Compound c (2.3 g, 3.4 mmol, yield 88%).

$^1$H NMR (400 MHz, $CDCl_3$) $\delta$ 8.89 (d, J = 2.0 Hz, 1H), 8.63 (d, J = 7.6 Hz, 2H), 8.42 (d, J = 6.8 Hz, 4H), 7.78 (d, J = 7.6 Hz, 2H), 7.56-7.50 (m, 4H), 7.42-7.35 (m, 11H), 7.28-7.24 (m, 3H).

ASAP Mass Spectrometry: theoretical value 682.23, observed value 683.36

Compound 1

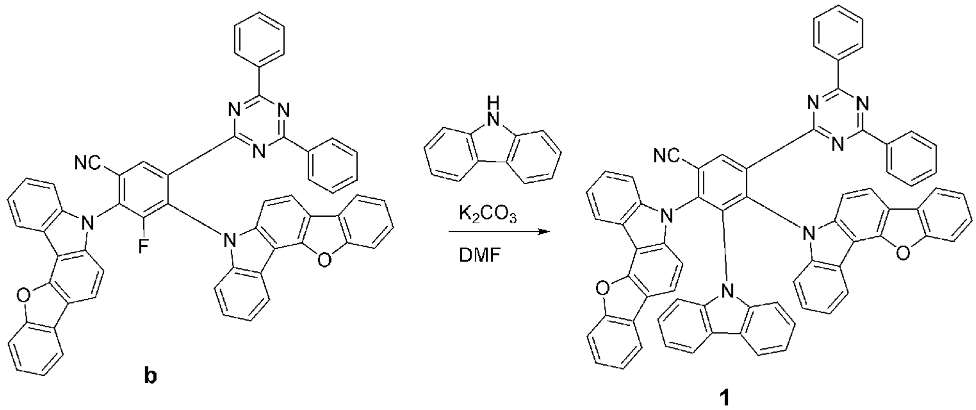


b

1

Under a nitrogen stream, an N,N-dimethylformamide DMF solution (32 mL) solution of Compound b (1.4 g, 1.6 mmol), 9H-carbazole (0.3 g, 1.9 mmol) and potassium carbonate (0.3 g, 2.4 mmol) was stirred under heat at 80°C for 12 hours. Thereafter this was restored to room temperature, then water and methanol were added thereto to precipitate a solid, and the solid was recovered by filtration. The recovered reaction mixture was purified by silica gel column chromatography (toluene/hexane = 2/1) and reprecipitation (toluene/methanol) to give Compound 1 (1.2 g, 1.18 mmol, yield 74%).

$^1$H NMR (400 MHz, $CDCl_3$) $\delta$ 9.13 (s, 1H), 8.20-8.17 (m, 1H), 8.00-7.88 (m, 6H), 7.81 (d, J = 7.2, 1H), 7.69-7.63 (m, 4H), 7.44-7.16 (m, 17H), 7.11-7.00 (m, 5H), 6.79-6.53 (m, 4H).

ASAP Mass Spectrometry: theoretical value 1009.32, observed value 1010.59

(Synthesis Example 2) Synthesis of Compound 24974

Compound 24974

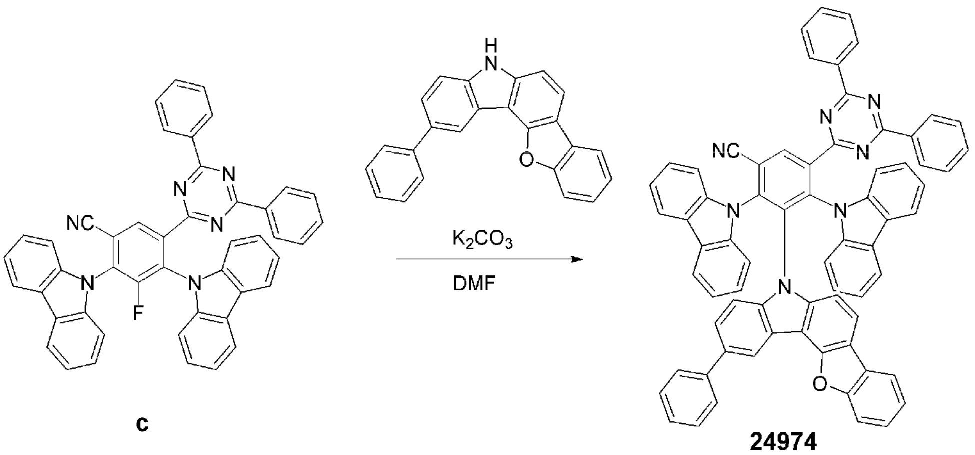


c

24974

Under a nitrogen stream, an N,N-dimethylformamide solution (30 mL) of Compound c (1.0 g, 1.5 mmol), 2-phenyl-5H-benzofuro[3,2-c]carbazole (0.6 g, 1.9 mmol) and potassium carbonate (0.3 g, 2.2 mmol) was stirred under heat at 80°C for 12 hours. Thereafter this was restored to room temperature, then water and methanol were added thereto to precipitate a solid, and the solid was recovered by filtration. The recovered reaction mixture was purified by silica gel column chromatography (toluene/hexane = 2/1) and reprecipitation (toluene/methanol) to give Compound 24974 (1.2 g, 1.2 mmol, yield 83%).

$^{1}H$ NMR (400 MHz, $CDCl_3$) δ 9.11 (s, 1H), 8.00-7.97 (m, 5H), 7.76 (d, J = 7.6 Hz, 1H), 7.70-7.65 (m, 2H), 7.54-7.47 (m, 6H), 7.41 (t, J = 7.2, 2H), 7.36-7.21 (m, 13H), 7.15-6.91 (m, 11H).
ASAP Mass Spectrometry: theoretical value 995.34, observed value 996.40

(Synthesis Example 3) Synthesis of Compound 24974(91)

Compound 24974(91)

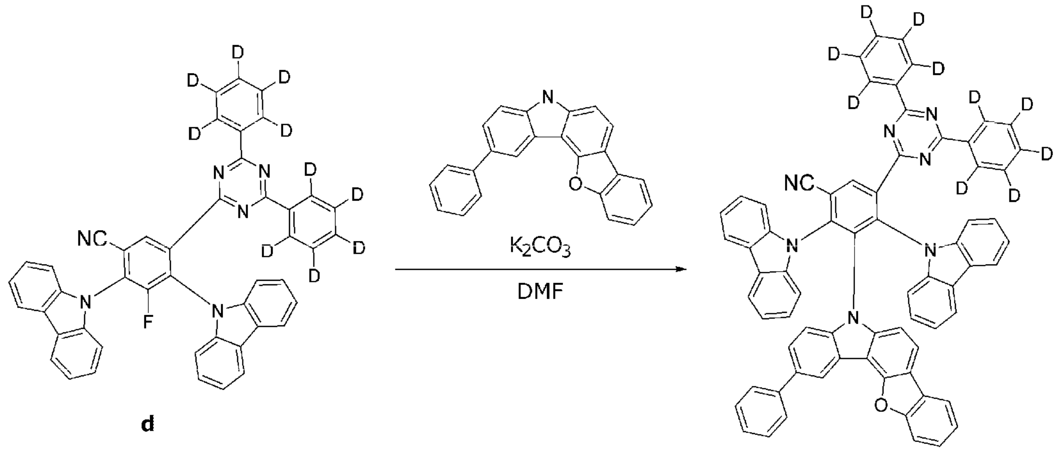


d

24974(91)

Under a nitrogen stream, an N,N-dimethylformamide solution (30 mL) of Compound d (2.0 g, 2.9 mmol), 2-phenyl-5H-benzofuro[3,2-c]carbazole (1.3 g, 3.8 mmol) and potassium carbonate (0.6 g, 4.3 mmol) was stirred under heat at 110°C for 12 hours. Thereafter this was restored to room temperature, then water and methanol were added thereto to precipitate a solid, and the solid was recovered by filtration. The recovered reaction mixture was purified by silica gel column chromatography (toluene/hexane/chloroform =7/2.5/0.5). A solid of a purified product was suspended in toluene and stirred by heating under reflux for 1 hour, then restored to room temperature, methanol was added, and the solid was separated by filtration to give Compound 24974(91) (2.7 g, 2.2 mmol, yield 92%).

$^{1}H$ NMR (400 MHz, $CDCl_3$) δ 9.11 (s, 1H), 8.01 (s, 1H), 7.76 (d, J = 7.6 Hz, 1H), 7.69-7.65 (m, 2H), 7.54-7.50 (m, 5H), 7.41 (t, J = 7.2, 2H), 7.36-7.21 (m, 8H), 7.15-6.91 (m, 11H).

(Synthesis Example 4) Synthesis of Compound 33530(91)

Compound f

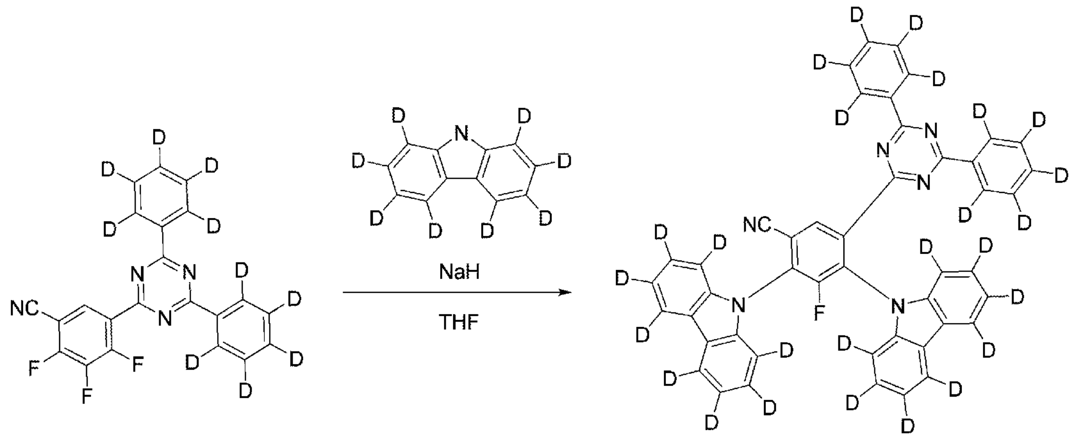


e f

Under a nitrogen stream, a tetrahydrofuran solution (24 mL) of deuterated 9H-carbazole (1.3 g, 7.7 mmol) and sodium hydride (0.3 g, 7.7 mmol) was added to a tetrahydrofuran solution (100 mL) of Compound e (1.5 g, 3.8 mmol) at -15°C, and stirred for 3 hours. The mixture was restored to room temperature, further stirred for 2 hours, and then the reaction was stopped with an aqueous solution of saturated ammonium chloride (10 mL). After the organic solvent was concentrated, the residue was filtered, and washed with ion-exchanged water and methanol, and the solid was separated by filtration. The resultant solid was purified by column chromatography (toluene/hexane/chloroform = 8/1.5/0.5)) and reprecipitation (chloroform/methanol = 50 mL/100 mL) to give Compound f (2.5 g, 3.6 mmol, yield 96%).

$^1H$ NMR (400 MHz, $CDCl_3$) $\delta$ 8.89 (m, 1H).

Compound 33530(91)

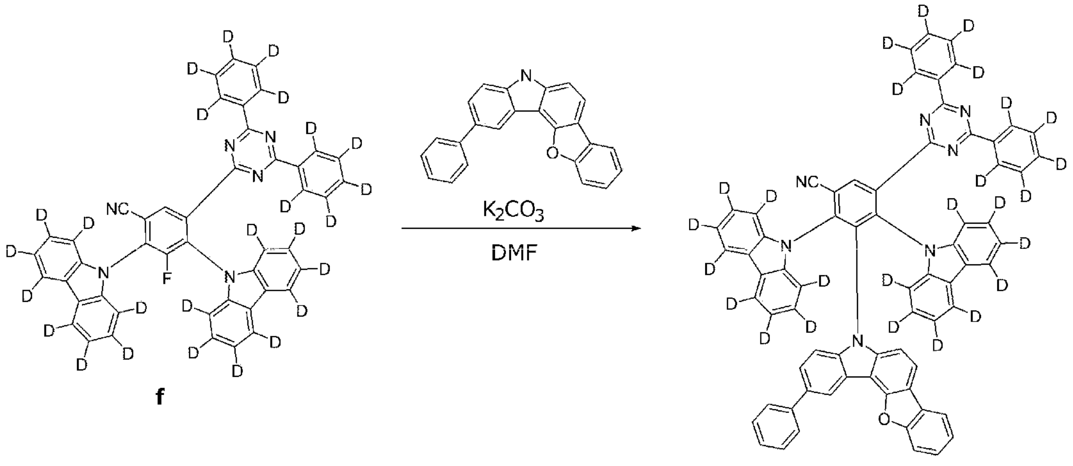


f

33530(91)

Under a nitrogen stream, an N,N-dimethylformamide solution (30 mL) of Compound f (1.8 g, 2.6 mmol), 2-phenyl-5H-benzofuro[3,2-c]carbazole (1.1 g, 3.3 mmol) and potassium carbonate (0.5 g, 3.8 mmol) was stirred under heat at 110°C for 12 hours. Thereafter this was restored to room temperature, then water and methanol were added thereto to precipitate a solid, and the solid was recovered by filtration. The recovered reaction mixture was purified by silica gel column chromatography (toluene/hexane/chloroform =7/2.5/0.5). A solid of the purified product was suspended in toluene (50 mL) and stirred by heating under reflux for 1 hour, then restored to room temperature, and methanol (100 mL) was added, and the solid was separated by filtration to give Compound 33530(91) (2.3 g, 2.2 mmol, yield 87%).

$^1H$ NMR (400 MHz, $CDCl_3$) $\delta$ 9.10 (s, 1H), 8.00 (s, 1H), 7.75 (d, J = 7.6 Hz, 1H), 7.53-7.46 (m, 3H), 7.40 (t, J = 7.6 Hz 2H), 7.35-7.20 (m, 4H), 7.04-6.91 (m, 3H).

(Synthesis Compound 5) Synthesis of Compound 33523(91)

Compound 33523(91)

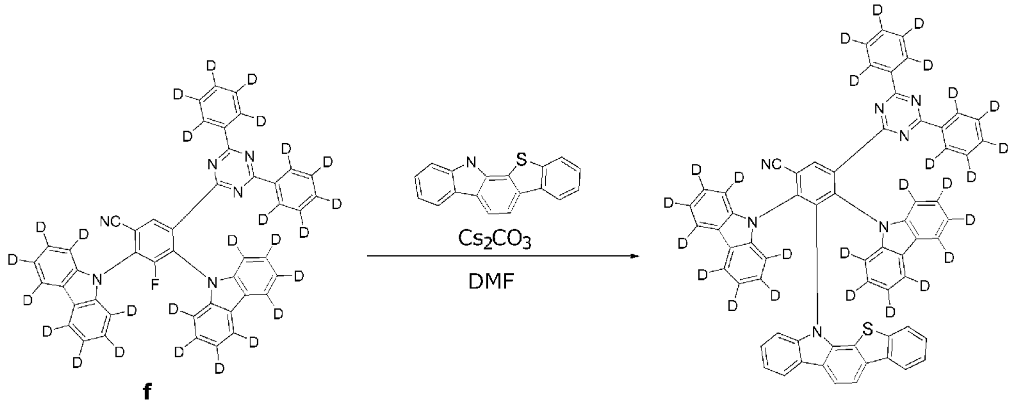


f

33523(91)

Under a nitrogen stream, an N,N-dimethylformamide solution (30 mL) of Compound f (0.6 g, 0.9 mmol), 12H-[1]benzothieno[2,3-a]carbazole (0.3 g, 1.1 mmol) and cesium carbonate (0.3 g, 1.0 mmol) was stirred under heat at 120°C for 12 hours. Thereafter this was restored to room temperature, then water and methanol were added thereto to precipitate a solid, and the solid was recovered by filtration. The recovered reaction mixture was purified by silica gel column chromatography (toluene/hexane/chloroform = 7/2.5/0.5), the resultant solid was reprecipitated from toluene/methanol, and the solid was separated by filtration to give Compound 33523(91) at yield of 58% (0.6 g, 0.6 mmol). ASAP Mass Spectrometry: theoretical value 961.45, observed value 962.77

(Example 1) Preparation and Evaluation of Thin Film

Compound 1 was vapor-deposited on a quartz substrate by a vacuum deposition method under conditions of a vacuum degree of less than $1 \times 10^{-3}$ Pa to form a neat thin film of Compound 1 having a thickness of 100 nm.

Separately, Compound 1 and mCBP were vapor-deposited from different vapor deposition sources on a quartz substrate by a vacuum deposition method under conditions of a vacuum degree of less than $1 \times 10^{-3}$ Pa to form a doped thin film having a content of Compound 1 of 20% by weight and a thickness of 100 nm.

In the same manner but using Compound 24974, Compound 24974(91), Compound 33530(91) and Comparative Compound 1 in place of Compound 1, neat thin films and doped thin films were formed.

The maximum emission wavelength (λmax) was measured when the formed doped thin films were irradiated with excitation light of 300 nm. Also, using the formed neat thin films, the HOMO energy ($E_{HOMO}$) and the LUMO energy ($E_{LUMO}$) were measured. The results are shown in Table 4. The photoluminescence quantum yield was 79% for the doped film of Compound 24974 (91) and 84% for the doped film of

Compound 33530 (91).

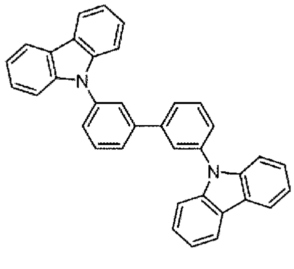

mCBP

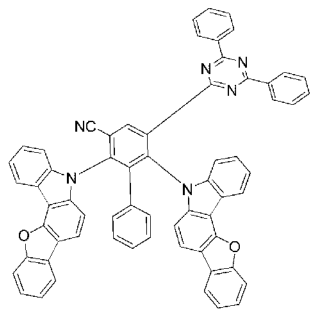

Comparative Compound 1

(Example 2) Production and Evaluation of Organic Electroluminescent Device

On a glass substrate on which an anode made of indium-tin oxide (ITO) having a film thickness of 50 nm was formed, each thin film was laminated by a vacuum deposition method at a vacuum degree of $5.0 \times 10^{-5}$ Pa. First, HAT-

CN was formed to a thickness of 10 nm on the ITO, NPD was formed to a thickness of 35 nm on the HAT-CN, and further PTCz was formed to a thickness of 10 nm on the NPD. Next, H1 and Compound 1 were vapor co-deposited from different vapor deposition sources to form a layer with a thickness of 40 nm as a light emitting layer. The content of Compound 1 in the light emitting layer was 30% by mass. Next, after ET1 was formed to a thickness of 10 nm, Liq and SF3-TRZ were vapor co-deposited from different vapor deposition sources to form a layer with a thickness of 20 nm. The contents of Liq and SF3-TRZ in this layer were 30% by mass and 70% by mass, respectively. Furthermore, Liq was formed to a thickness of 2 nm, and aluminum (Al) was vapor-deposited to a thickness of 100 nm to form a cathode, thereby obtaining an organic electroluminescent device.

Organic electroluminescent devices were produced in the same manner except that Compound 24974, Compound 24974(91), Compound 33530(91), and Comparative Compound 1 were used instead of Compound 1.

Of each organic electroluminescent device, the external quantum efficiency (EQE) at 6.3 mA, the drive voltage ($V_{init}$), and the time taken until the emission intensity at 12.6 mA/cm$^2$ reached 95% at the start of the test (LT95) were measured, and the results are shown in Table 4. LT95 is expressed as a relative value when the device using Comparative Compound 1 is defined as 1.

Table 4

| | Compound 1 | Compound 24974 | Compound 24974 (91) | Compound 33530 (91) | Comparative Compound 1 |
|---|---|---|---|---|---|
| λmax (nm) | 537 | 526 | 528 | 523 | 519 |
| $E_{HOMO}$ (eV) | 6.00 | 5.93 | 5.93 | 5.96 | 5.93 |
| $E_{LUMO}$ (eV) | 3.52 | 3.40 | 3.38 | 3.38 | 3.35 |
| EQE (%) | 12 | 14 | 15.2 | 13.3 | 12 |
| $V_{init}$ (V) | 3.5 | 3.2 | 3.3 | 3.3 | 3.9 |
| LT95 | 2.0 | 4.0 | 4.0 | 4.0 | 1 |

It was confirmed that the use of the compound represented by the general formula (1) can improve the light emission efficiency of the device and reduce the drive voltage of the device. Also it was confirmed that the use of the compound represented by the general formula (1) can prolong the device lifetime and improve the durability.

(Example 9) Production and Evaluation of Organic Electroluminescent Device Using Compound 1 as Assist Dopant

An organic electroluminescent device was produced in the same manner as in Example 2 only except that a light emitting layer having a thickness of 40 nm was formed by depositing H1, Compound 1 and EM1 as a light emitting material in order of 69.5 % by weight, 30.0 % by weight and 0.5 % by weight from different evaporation sources in place of the light emitting layer in Example 2.

The above confirms that when the compound represented by the general formula (1) is used as an assist dopant, an organic electroluminescent device having a high light emission efficiency and a good durability can also be provided.

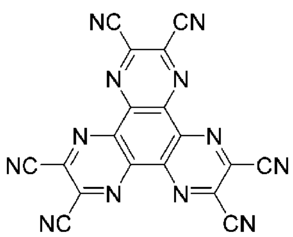

HAT−CN

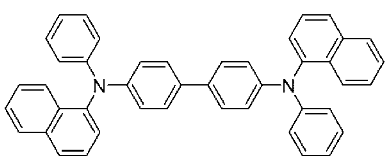

NPD

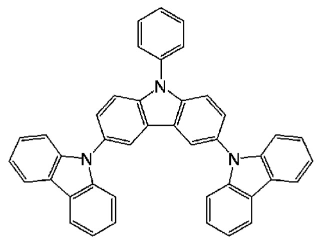

PTCz

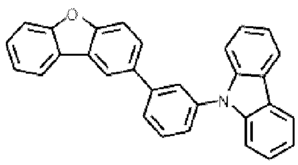

H1

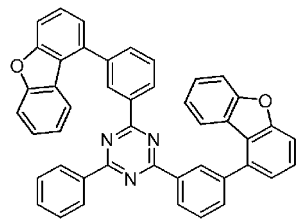

ET1

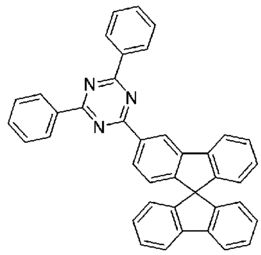

SF3-TRZ

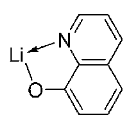

Liq

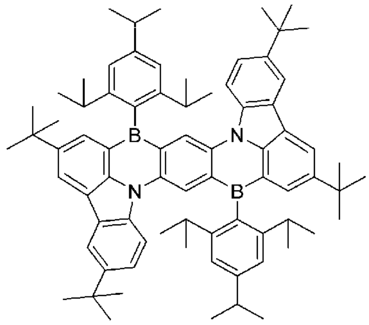

EM1

Industrial Applicability

By using a compound represented by the general formula (1), there can be provided an organic light emitting device having good light emission characteristics. Accordingly, the industrial applicability of the present invention is great.

## Claims

1. A compound represented by the following general formula (1):

General Formula (1)

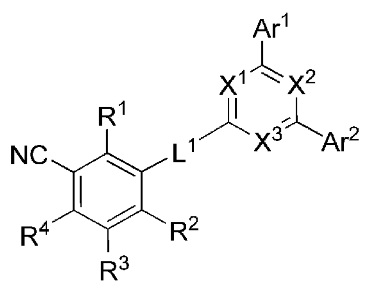

wherein $R^1$ represents a hydrogen atom, a deuterium atom, or a substituted or unsubstituted alkyl group;
$R^2$ to $R^4$ each independently represent a donor group that is not a substituted or unsubstituted aryl group, and $R^2$ to $R^4$ are not all the same;
$X^1$ to $X^3$ each independently represent N or C(R), and at least one of $X^1$ to $X^3$ is N;
R represents a hydrogen atom, a deuterium atom or a substituent;
$Ar^1$ and $Ar^2$ each independently represent a substituted or unsubstituted aryl group, or a substituted or unsubstituted heteroaryl group; and
$L^1$ represents a single bond or a divalent linking group.

2. The compound according to claim 1, wherein $R^2$ to $R^4$ each independently represent a substituted or unsubstituted

diarylamino group, provided that the two aryl groups constituting the diarylamino group can bond to each other.

3. The compound according to claim 2, wherein $R^2$ to $R^4$ each independently represent a substituted or unsubstituted carbazol-9-yl group.

4. The compound according to claim 1, wherein at least one of $R^2$ to $R^4$ is a substituted or unsubstituted ring-fused carbazol-9-yl group.

5. The compound according to claim 4, wherein at least one of $R^2$ to $R^4$ is a ring-fused carbazol-9-yl group substituted with one atom or group selected from the group consisting of a deuterium atom, an alkyl group and an aryl group or with a group formed by combining two or more thereof.

6. The compound according to claim 5, wherein the ring-fused carbazol-9-yl group is substituted with an aryl group optionally substituted with one atom or group selected from the group consisting of a deuterium atom, an alkyl group and an aryl group or with a group formed by combining two or more thereof.

7. The compound according to claim 4, wherein the ring-fused carbazol-9-yl group is a benzofuro-ring-fused carbazol-9-yl group or a benzothieno-ring-fused carbazol-9-yl group.

8. The compound according to claim 1, wherein at least one of $R^2$ to $R^4$ is a carbazol-9-yl group optionally substituted with a deuterium atom.

9. The compound according to claim 1, wherein $R^2$ and $R^3$ are the same.

10. The compound according to claim 1, wherein $R^2$ and $R^4$ are the same.

11. The compound according to claim 1, wherein $X^1$ to $X^3$ are N.

12. The compound according to claim 1, wherein $Ar^1$ and $Ar^2$ each are an aryl group optionally substituted with a deuterium atom.

13. The compound according to claim 1, wherein $L^1$ is a single bond.

14. The compound according to claim 1, wherein $R^1$ is a hydrogen atom.

15. The compound according to claim 1, wherein the compound has at least one deuterium atom.

16. A light emitting material comprising the compound according to any one of claims 1 to 15.

17. A delayed fluorescent material comprising the compound according to any one of claims 1 to 15.

18. A film comprising the compound according to any one of claims 1 to 15.

19. An organic semiconductor device comprising the compound according to any one of claims 1 to 15.

20. An organic light emitting device comprising the compound according to any one of claims 1 to 15.

21. The organic light emitting device according to claim 20, wherein the device has a layer containing the compound, and the layer also contains a host material.

22. The organic light emitting device according to claim 21, wherein the layer containing the compound further contains a delayed fluorescent material in addition to the compound and the host material, and the delayed fluorescent material has a lowest excited singlet energy lower than that of the host material and higher than that of the compound.

23. The organic light emitting device according to claim 21, wherein the device has a layer containing the compound, and the layer also contains a light emitting material having a structure different from that of the compound.

24. The organic light emitting device according to claim 21, wherein the amount of light emitted from the compound is

the largest among materials contained in the device.

**25.** The organic light emitting device according to claim 23, wherein the amount of light emitted from the light emitting material is larger than the amount of light emitted from the compound.

**26.** The organic light emitting device according to claim 20, which is an organic electroluminescent device.

**27.** The organic light emitting device according to claim 20, which emits delayed fluorescence.

**INTERNATIONAL SEARCH REPORT**

International application No. **PCT/JP2022/042231**

**A. CLASSIFICATION OF SUBJECT MATTER**

***C07D 491/048***(2006.01)i; ***C09K 11/06***(2006.01)i; ***C07D 519/00***(2006.01)i; ***H10K 50/00***(2023.01)i
FI: C07D491/048; H05B33/14 B; C09K11/06 690; C09K11/06 655; C07D519/00 CSP; C07D519/00 301

According to International Patent Classification (IPC) or to both national classification and IPC

**B. FIELDS SEARCHED**

Minimum documentation searched (classification system followed by classification symbols)

C07D491/048; C09K11/06; C07D519/00; H01L51/50

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | JP 2021-519765 A (KYULUX, INC.) 12 August 2021 (2021-08-12) claims, examples | 1-27 |
| A | WO 2021/157642 A1 (KYULUX INC.) 12 August 2021 (2021-08-12) paragraph [0043], compound TADF3 | 1-27 |
| A | WO 2020/085842 A1 (LG CHEM, LTD.) 30 April 2020 (2020-04-30) examples, claims | 1-27 |
| A | WO 2019/190223 A1 (LG CHEM, LTD.) 03 October 2019 (2019-10-03) compounds 6, 7, 10, claims | 1-27 |
| A | WO 2021/046523 A1 (KYULUX, INC.) 11 March 2021 (2021-03-11) pp. 25-28 | 1-27 |
| A | HANSCH, Corwin et al., A survey of Hammett substituent constants and resonance and field parameters, Chem. Rev., 1991, 91(2), 165-195, DOI: 10.1021/cr00002a004 tables | 1-27 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 November 2022** | **13 December 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/042231**

| Patent document cited in search report | Publication date (day/month/year) | Patent family member(s) | Publication date (day/month/year) |
|---|---|---|---|
| JP 2021-519765 A | 12 August 2021 | US 2021/0135119 A1<br>examples, claims<br>WO 2019/191665 A1<br>EP 3775100 A1<br>KR 10-2020-0139715 A<br>CN 112272695 A | |
| WO 2021/157642 A1 | 12 August 2021 | JP 2022-8106 A<br>WO 2022/168956 A1<br>WO 2021/235549 A1<br>WO 2021/157593 A1 | |
| WO 2020/085842 A1 | 30 April 2020 | US 2022/0271233 A1<br>examples, claims<br>CN 112533900 A<br>KR 10-2020-0047418 A | |
| WO 2019/190223 A1 | 03 October 2019 | CN 111247140 A<br>KR 10-2019-0113661 A | |
| WO 2021/046523 A1 | 11 March 2021 | EP 4025569 A1<br>CN 114341129 A<br>KR 10-2022-0059485 A<br>TW 202116757 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2021045623 A1
- WO 2015022974 A
- JP 2021103698 A
- JP 2021103699 A
- JP 2021103700 A
- JP 2021081332 A
- JP 2021103701 A
- JP 2021151805 A
- JP 2021188860 A
- WO 2013154064 A
- WO 2013011954 A
- WO 2013011955 A
- WO 2013081088 A
- JP 2013256490 A
- JP 2013116975 A
- WO 2013133359 A
- WO 2013161437 A
- JP 2014009352 A
- JP 2014009224 A
- JP 2017119663 A
- JP 2017119664 A
- JP 2017222623 A
- JP 2017226838 A
- JP 2018100411 A
- WO 2018047853 A
- JP 2013253121 A
- WO 2014034535 A
- WO 2014115743 A
- WO 2014122895 A
- WO 2014126200 A
- WO 2014136758 A
- WO 2014133121 A
- WO 2014136860 A
- WO 2014196585 A
- WO 2014189122 A
- WO 2014168101 A
- WO 2015008580 A
- WO 2014203840 A
- WO 2015002213 A
- WO 2015016200 A
- WO 2015019725 A
- WO 2015072470 A
- WO 2015108049 A
- WO 2015080182 A
- WO 2015072537 A
- WO 2015080183 A
- JP 2015129240 A
- WO 2015129714 A
- WO 2015129715 A
- WO 2015133501 A
- WO 2015136880 A
- WO 2015137244 A
- WO 2015137202 A
- WO 2015137136 A
- WO 2015146541 A
- WO 2015159541 A

### Non-patent literature cited in the description

- **HANSCH, C.** *Chem. Rev.,* 1991, vol. 91, 165-195